# EUROPEAN PATENT SPECIFICATION

(11) **EP 3 250 658 B1**
(45) Date of publication and mention of the grant of the patent: **19.06.2019**
(21) Application number: 16700236.9
(22) Date of filing: 07.01.2016
(51) Int. Cl.: C09K 11/06, H01L 51/50, C07D 405/14, C07F 9/50, C07F 9/53, C07D 403/04, C07D 403/14, C07D 409/12, C07D 409/14, C07D 491/048, C07D 495/04, C07F 5/02, C07D 497/04, C07F 7/08, C09K 11/02, H01L 51/00, C07D 487/04

(54) **MATERIALS FOR ELECTRONIC DEVICES**
MATERIALIEN FÜR ELEKTRONISCHE VORRICHTUNGEN
MATIÈRES POUR DISPOSITIFS ÉLECTRONIQUES

(30) Priority: 30.01.2015 EP 15000279
(43) Date of publication of application: 06.12.2017
(73) Proprietor: Merck Patent GmbH, 64293 Darmstadt (DE)
(72) Inventor: ANÉMIAN, Rémi, Manouk, Seoul 657-169 (KR); LEE, Dong-Hee, Gunpo-shi Gyeonggi-do 435-019 (KR); SEO, Ha-Na, Pyeongtaek 467-810 (KR)
(86) International application number: PCT/EP2016/000011
(87) International publication number: WO 2016/119992

(56) References cited:
- EP-A1- 2 599 773
- WO-A1-2011/055912
- WO-A1-2013/069939
- DE-A1-102010 014 933
- KR-A- 20140 034 710

## Description

The present application relates to materials for use in electronic devices, in particular in organic electroluminescent devices, and to electronic devices comprising these materials.

Electronic devices in the sense of this application are taken to mean, in particular, so-called organic electronic devices which comprise organic semiconductor materials as functional materials. Again in particular, these are taken to mean organic electroluminescent devices (OLEDs) and other electronic devices which are indicated below in the detailed description of the invention.

In general, the term OLED is taken to mean an electronic device which comprises at least one organic material and emits light on application of an electrical voltage. The precise structure of OLEDs is described, inter alia, in US 4539507, US 5151629, EP 0676461 and WO 98/27136.

There is major interest in improving the performance data of electronic devices, in particular OLEDs, in particular lifetime and efficiency and operating voltage. An important role is played here by organic emitter layers, in particular the matrix materials present therein, and organic layers having an electron-transporting function.

In order to achieve this technical object, there is a continuous search for novel materials which are suitable for use as matrix materials in emitting layers, in particular phosphorescent emitting layers. Furthermore, materials having electron-transporting and/or hole-blocking properties are sought for use in corresponding functional layers.

Phosphorescent emitting layers in the sense of the present application are organic layers which comprise at least one phosphorescent emitter compound.

Emitter compounds of an emitting layer are typically compounds which emit light on operation of the electronic device.

The term phosphorescent emitters in accordance with the present application encompasses compounds in which the light emission takes place through a spin-forbidden transition, for example a transition from an excited triplet state or a state having a relatively high spin quantum number, such as a quintet state.

A matrix material in a system comprising two or more materials is taken to mean the component whose proportion in the mixture is the greater. Correspondingly, a dopant in a system comprising two or more materials is taken to mean the component whose proportion in the mixture is the smaller. Instead of the term matrix material, the term host material is also used in many cases.

If an emitter compound is used in combination with one or more further compounds in an emitting layer, its proportion in the mixture is typically the relatively smaller. In this case, it may also be referred to as dopant compound. The one or more further compounds are typically present in the mixture in relatively larger proportion and can therefore be referred to in accordance with the above definition as matrix materials.

The use of compounds containing one or more indenocarbazole groups in electronic devices is known from the prior art, for example from WO 2010/136109 and WO 2011/000455.

The use of compounds containing one or more electron-deficient heteroaromatic six-membered rings in electronic devices is furthermore known from the prior art, for example from WO 2010/015306, WO 2007/063754 and WO 2008/056746.

The prior art again furthermore discloses the use of compounds which contain at least one electron-deficient heteroaromatic six-membered rings, at least one arylamino group and at least one indenocarbazole group in electronic devices. Such compounds and their use in electronic devices are disclosed, for example, in WO 2010/136109, WO 2014/090368, WO 2013/069939, KR 2014/0034710 and EP 2599773.

However, organic electroluminescent devices are still in need of improvement. More particularly, the efficiency in the case of fluorescent and also phosphorescent OLEDs should be improved. There is also a need of improvement in terms of OLEDs operating lifetime, in particular in the case of blue emission. Furthermore, it is highly desirable to reduce the operating voltage, both in the case of fluorescent and phosphorescent OLEDs, in order to improve the power efficiency. This is of major importance, in particular for mobile applications.

Considering matrix materials for phosphorescent emitters, there is a need for improvement so that these materials result in good efficiency, a long lifetime and a low operating voltage of the organic electroluminescent device, in which they are used. The properties of the matrix materials, in particular, are frequently limiting for the lifetime and efficiency of the electroluminescent device.

Surprisingly, it has now been found that excellent performance data can be achieved with a certain indenocarbazole compound which is connected to an electron-deficient six-membered heteroaromatic ring via its N atom and which comprises a particular arylamino group. In particular, an excellent lifetime and power efficiency are achieved on use in organic electroluminescent devices.

The present application thus relates to a compound of a formula (1-1) 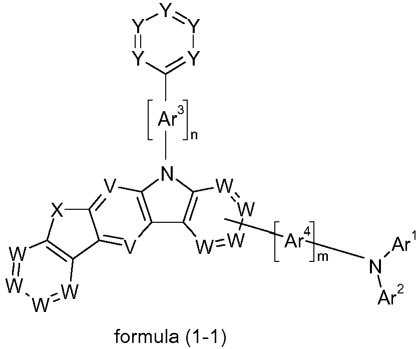 where:
Y is, identically or differently, equal to N or CR¹, with the proviso that the group of the formula (Y), 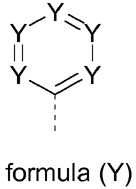 as constituent of the formula (1 -1) conforms to one of the following formulae (Y-1) to (Y-6), where the dashed line denotes the bond to the remainder of the compound,

| | |
|---|---|
| 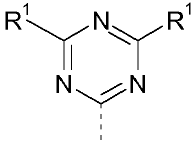 | 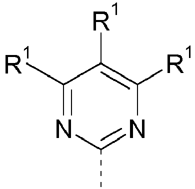 |
| formula (Y-1) | formula (Y-2) |
| 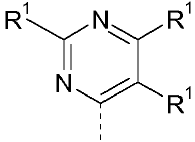 | 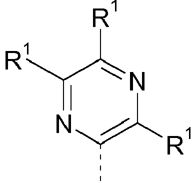 |
| formula (Y-3) | formula (Y-4) |
| 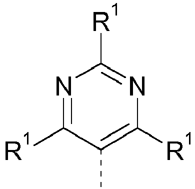 | 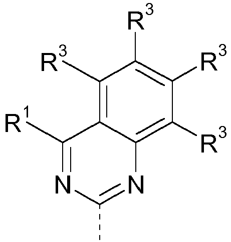 |
| formula (Y-5) | formula (Y-6) |

W is, identically or differently, equal to CR¹ or N;
V is, identically or differently, equal to CR¹ or N;
X is a divalent bridge selected from N(R²), B(R²), O, C(R²)₂, Si(R²)₂, C=NR², C=C(R²)₂, S, S=O, SO₂, P(R²) and P(=O)R²;
Ar¹, Ar² are, identically or differently, an aromatic or heteroaromatic ring system, which may be substituted by one or more radicals R¹; wherein at least one of the group Ar¹ or Ar² stands for an aromatic ring system having 12 to 30 aromatic C atoms, an heteroaryl group having 13 to 30 aromatic ring atoms or an aryl group having 10 to 20 aromatic C atoms, each of which may be substituted by one or more radicals R¹, Ar¹ and Ar² may also be connected to one another by a group E;
Ar³, Ar⁴ are on each occurrence, identically or differently, an aromatic ring system having 6 to 40 aromatic C atoms or an heteroaromatic ring system having 5 to 40 aromatic ring atoms, which may be substituted by one or more radicals R¹; with the proviso that Ar⁴ is not an anthracenylene group;
E is a single bond, N(R³), O, S, C(R³)₂, C(R³)₂-C(R³)₂, Si(R³)₂ or B(R³)₂;
R¹ is on each occurrence, identically or differently, H, D, F, Br, CI, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two adjacent radicals R¹ located on Ar¹ or two adjacent radicals R¹ located on Ar² may be linked to one another and may form a ring;
R² is on each occurrence, identically or differently, H, D, F, Br, Cl, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³; where two radicals R² may be linked to one another and may form a ring;
R³ is on each occurrence, identically or differently, H, D, F, Br, Cl, I, C(=O)R⁴, CN, Si(R⁴)₃, N(R⁴)₂, P(=O)(R⁴)₂, S(=O)R⁴, S(=O)₂R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is equal to 0 or 1;
m is equal to 0 or 1.

For the purposes of the present application, the following definitions of chemical groups apply:
An aryl group in the sense of this invention contains 6 to 60 aromatic ring atoms; a heteroaryl group in the sense of this invention contains 5 to 60 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aromatic ring system in the sense of this invention contains 6 to 60 C atoms in the ring system. A heteroaromatic ring system in the sense of this invention contains 5 to 60 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an sp³-hybridised C, Si, N or O atom, an sp²-hybridised C or N atom or an sp-hybridised C atom. Thus, for example, systems such as 9,9'-spirobifluorene, 9,9'-diarylfluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5 - 60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, ter-phenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or CH₂ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyl-oxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoro-ethylthio, ethenylthio, propenylthio, butenylthio, pentenylthio, cyclopenten-ylthio, hexenylthio, cyclohexenylthio, heptenylthio, cycloheptenylthio, octenylthio, cyclooctenylthio, ethynylthio, propynylthio, butynylthio, pentyn-ylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following scheme:

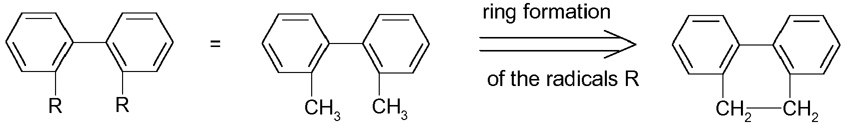

Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

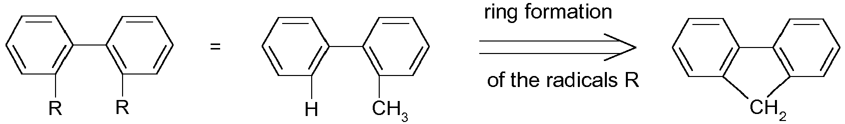

In accordance with a preferred embodiment, the index n is equal to 0.

In accordance with a further preferred embodiment, the index m is equal to 0 or 1.

In a particularly preferred embodiment, n is equal to 0 and m is equal to 1.

In accordance with a further preferred embodiment, a maximum of three groups W in an aromatic ring are equal to N, particularly preferably a maximum of two groups W in an aromatic ring are equal to N, and very particularly preferably a maximum of one group W in an aromatic ring is equal to N.

It is furthermore preferred for not more than two adjacent groups W in a six-membered ring to be equal to N.

It is especially preferred for W to be equal to CR¹.

In accordance with a further preferred embodiment, a maximum of one group V is equal to N.

It is especially preferred for V to be equal to CR¹.

In accordance with a preferred embodiment, X is selected from N(R²), O, C(R²)₂, S, more preferably X is C(R²)₂.

For the group Y, it is preferred for precisely two or precisely three groups Y in the ring to be equal to N, and for the remaining groups Y to be equal to CR¹. It is particularly preferred for precisely three groups Y in the ring to be equal to N, and for the remaining groups Y to be equal to CR¹.

In accordance with a further preferred embodiment, radicals R¹ in groups Y which represent CR¹ form a ring with one another. These are preferably radicals R¹ in adjacent groups Y which represent CR¹. In this case, the radicals R¹ in adjacent groups Y which represent CR¹ particularly preferably form a condensed-on benzene ring. In this case, it is very particularly preferred for precisely two groups Y to be equal to N.

For the group Ar⁴, it is preferred for it to represent a group of the following formula (Ar⁴-I) 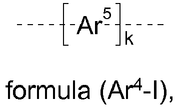 where the dashed lines represent the bonds to the indenocarbazole group and the six-membered ring containing the groups Y,
- Ar⁵: is on each occurrence, identically or differently, an aryl or heteroaryl group having 6 to 18 aromatic ring atoms, which may be substituted by one or more radicals R¹, where R¹ is defined as above; and
- k: is 1, 2, 3 or 4, where the index k is selected so that the number of aromatic ring atoms in the entire group Ar¹ does not exceed the number 40.

Ar⁵ is preferably on each occurrence, identically or differently, an aryl or heteroaryl group having 6 to 14 aromatic ring atoms, particularly preferably an aryl or heteroaryl group having 6 to 10 aromatic ring atoms, and very particularly preferably an aryl or heteroaryl group having 6 aromatic ring atoms, where the said groups may be substituted by one or more radicals R¹.

In accordance with a preferred embodiment, radicals R¹ here form rings between the aryl or heteroaryl groups Ar⁵ to which they are bonded. Particularly preferably, two groups Ar⁵ which represent phenyl are connected to form a fluorenyl group.

The index k is preferably 1, 2 or 3, more preferably 1 or 2 and particularly preferably 1.

Preferred embodiments of the group Ar⁴ conform to the formulae (Ar⁴-I-1) to (Ar⁴- I-26) indicated below:

| | |
|---|---|
| 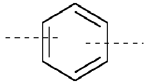 | 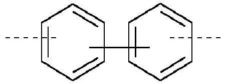 |
| formula (Ar⁴-I-1) | formula (Ar⁴-I-2) |
| 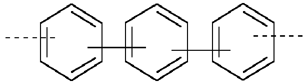 | 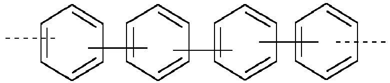 |
| formula (Ar⁴- I-3) | formula (Ar⁴- I-4) |
| 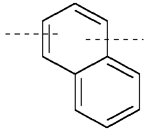 | 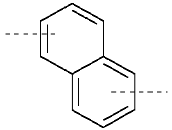 |
| formula (Ar⁴- I-5) | formula (Ar⁴- I-6) |
| 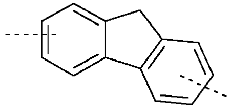 | 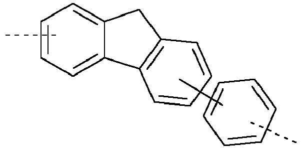 |
| formula (Ar⁴- I-7) | formula (Ar⁴- I-8) |
| 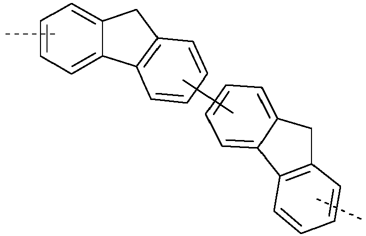 | 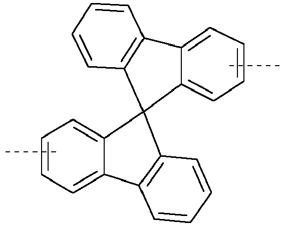 |
| formula (Ar⁴- I-9) | formula (Ar⁴-I-10) |
| 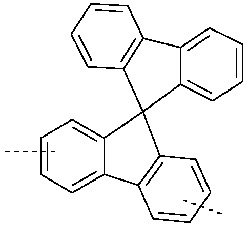 | 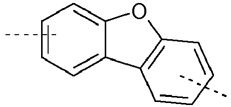 |
| formula (Ar⁴-I-11) | formula (Ar⁴- I-12) |
| 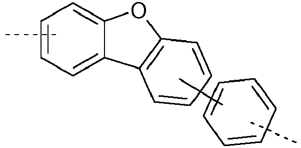 | 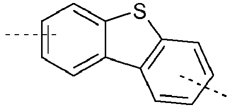 |
| formula (Ar⁴- I-13) | formula (Ar⁴- I-14) |
| 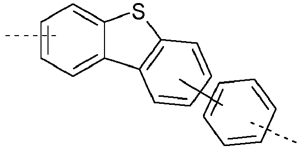 | 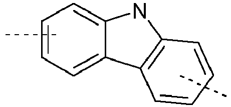 |
| formula (Ar⁴- I-15) | formula (Ar⁴- I-16) |
| 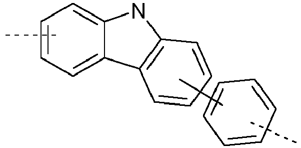 | 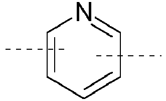 |
| formula (Ar⁴- I-17) | formula (Ar⁴- I-18) |
| 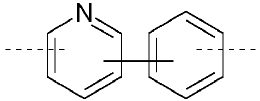 | 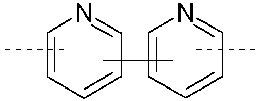 |
| formula (Ar⁴- I-19) | formula (Ar⁴- I-20) |
| 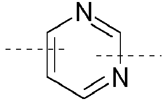 | 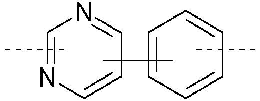 |
| formula (Ar⁴- I-21) | formula (Ar⁴- I-22) |
| 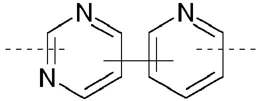 | 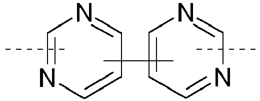 |
| formula (Ar⁴- I-23) | formula (Ar⁴- I-24) |
| 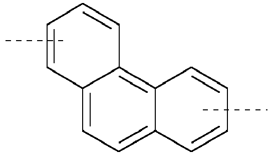 | 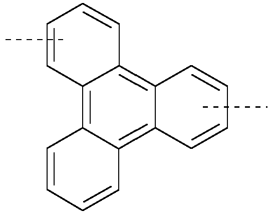 |
| formula (Ar⁴- I-25) | formula (Ar⁴- I-26) |

where the dashed lines represent the bonds to the indenocarbazole group and the diarylamino group -NAr¹Ar²,
and where the groups may be substituted by radicals R¹ at all free positions.

For the group Ar³, it is preferred for it to be selected from an aromatic or heteroaromatic ring system having 6 to 30 aromatic ring atoms, particularly preferably 6 to 24 aromatic ring atoms, which may be substituted by one or more radicals R¹.

Of the group (Y-1) to (Y-6), formula (Y-1) is particularly preferred.

The radical R¹ is preferably on each occurrence, identically or differently, H, D, F, CN, Si(R³)₃, N(R³)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R³ and where one or more CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, or an aromatic or heteroaromatic ring system having 5 to 20 aromatic ring atoms, which may in each case be substituted by one or more radicals R³, where two adjacent radicals R¹ located on Ar¹ or two adjacent radicals R¹ located on Ar² may be linked to one another and may form a ring.

The radical R² is preferably on each occurrence, identically or differently, H, D, F, CN, Si(R³)₃, N(R³)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R³ and where one or more CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, or an aromatic or heteroaromatic ring system having 5 to 20 aromatic ring atoms, which may in each case be substituted by one or more radicals R³; where two radicals R² may be linked to one another and may form a ring.

The radical R³ is preferably on each occurrence, identically or differently, H, D, F, CN, Si(R⁴)₃, N(R⁴)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R⁴ and where one or more CH₂ groups in the above-mentioned groups may be replaced by -C=C-, -R⁴C=CR⁴-, Si(R⁴)₂, C=O, C=NR⁴, -NR⁴-, -O-, -S-, -C(=O)O- or -C(=O)NR⁴-, or an aromatic or heteroaromatic ring system having 5 to 20 aromatic ring atoms, which may in each case be substituted by one or more radicals R⁴, where two or more radicals R³ may be linked to one another and may form a ring.

Regarding the bonding of the group Ar⁴ to the carbazole in formula (1-1), it is preferred for this to be in the positions denoted by "a" and "b", particularly preferably in the position denoted by "a", in formula (1-1-A) below. A corresponding situation applies, if the index m is equal to 0, to the diarylamino group (-NAr¹Ar²) bonded instead of Ar⁴.

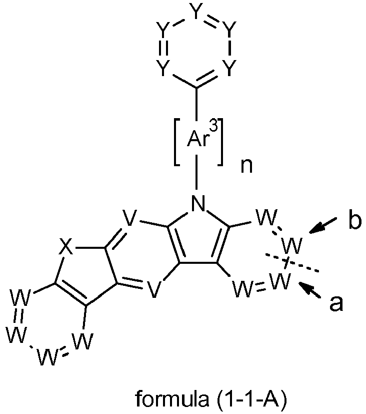

Preferred embodiments of compounds of the formula (1-1) conform to the following formula (1-1-1): 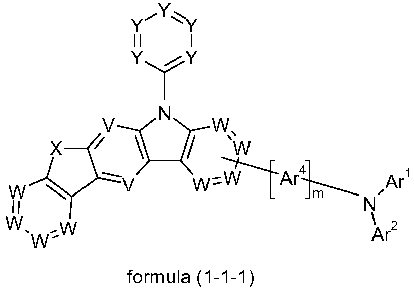 where the groups V, W, X, Ar⁴, Ar¹, Ar², Y and the six-membered ring containing the groups Y are as defined above.

Furthermore, the six-membered ring containing the groups Y in the above-mentioned formulae preferably conforms to the formula (Y-1).

More particularly preferred embodiments of compounds of the formula (1-1) conform to the following formula (1-1-2): 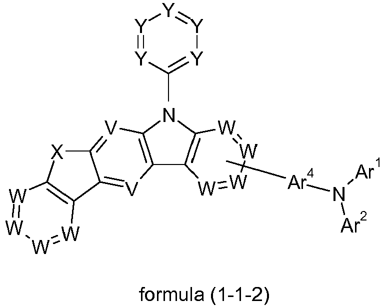 where the groups V, W, X, Ar⁴, Ar¹, Ar² and Y and the six-membered ring containing the groups Y are as defined above.

Furthermore, the six-membered ring containing the groups Y in the above-mentioned formulae preferably conforms to the formula (Y-1).

Very particularly preferred embodiments of compounds of the formula (1-1) conform to the following formula (1-1-3): 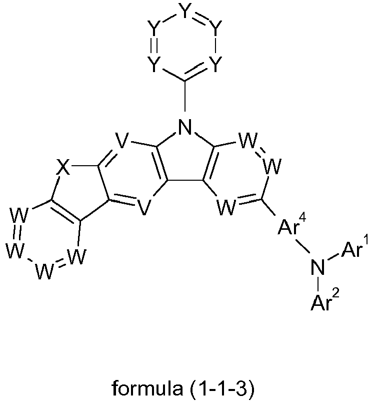 where the groups V, W, X, Y, Ar⁴, Ar¹, Ar², Y and the six-membered ring containing the groups Y are as defined above.

Furthermore, the six-membered ring containing the groups Y in the above-mentioned formulae preferably conforms to the formula (Y-1).

Further very particularly preferred embodiments of compounds of the formula (1-1) conform to the following formula (1-1-4): 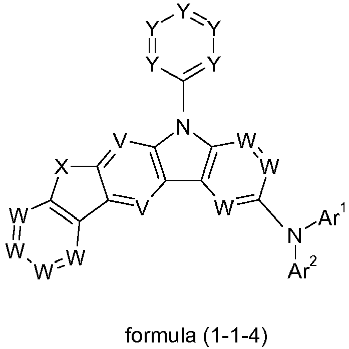 where the groups V, W, X, Ar¹, Ar², Y and the six-membered ring contain the groups Y are as defined above.

Furthermore, the six-membered ring containing the groups Y in the above-mentioned formulae preferably conforms to the formula (Y-1).

In accordance with a preferred embodiment, both groups Ar¹ and Ar² stands for an aromatic ring system having 12 to 30 aromatic C atoms, an heteroaryl group having 13 to 30 aromatic ring atoms or an aryl group having 10 to 20 aromatic C atoms, each of which may be substituted by one or more radicals R¹.

In accordance with a further preferred embodiment, at least one of the groups Ar¹ or Ar², is selected from the group consisting of biphenyl, terphenyl, quaterphenyl, fluorenyl, spirobifluorenyl, carbazolyl, dibenzofuranyl, dibenzothiophenyl, naphtyl, anthracyl, phenantryl, chrysenyl, triphenylenyl, pyrenyl, perylenyl, each of which may be substituted by one or more radicals R³ as defined above.

More preferably, both groups Ar¹ and Ar² are selected from the group consisting of biphenyl, terphenyl, quaterphenyl, fluorenyl, spirobifluorenyl, carbazolyl, dibenzofuranyl, dibenzothiophenyl, naphtyl, anthracyl, phenantryl, chrysenyl, triphenylenyl, pyrenyl, perylenyl, each of which may be substituted by one or more radicals R³ as defined above.

It is particularly preferred that at least one of the groups Ar¹ or Ar², is selected from the groups of the following formulae (11) to (77), 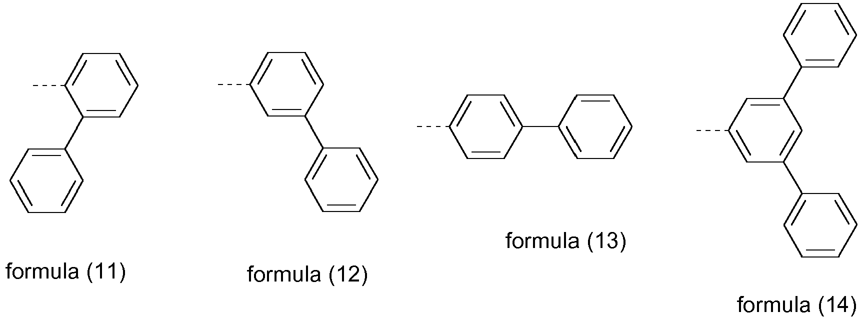 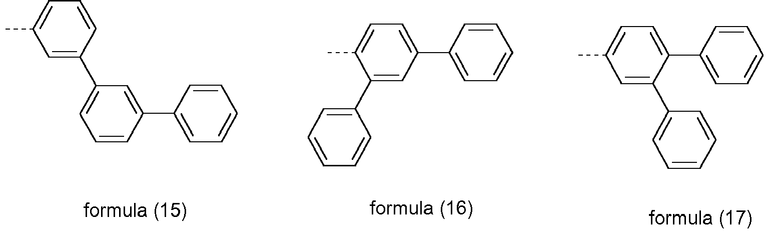 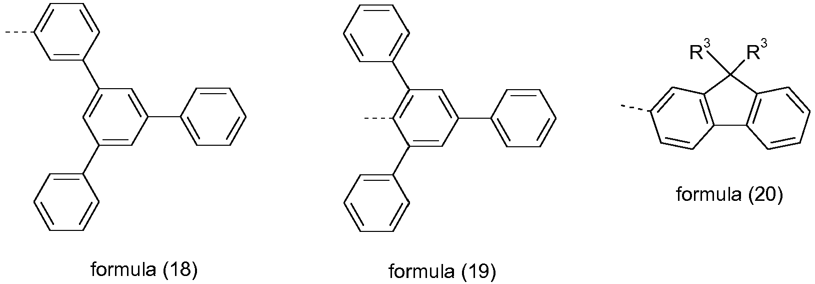 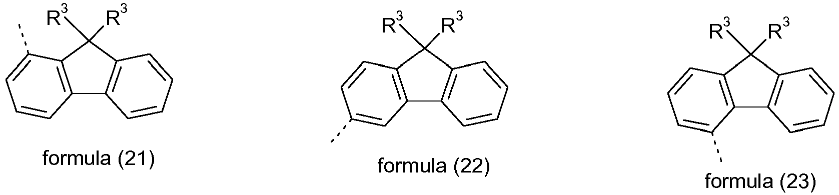 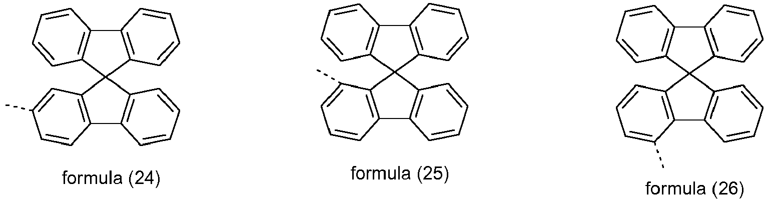 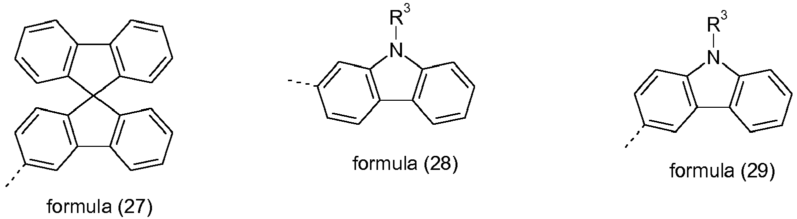 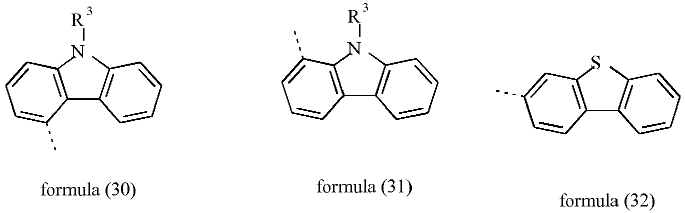 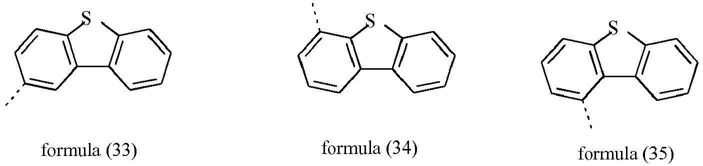 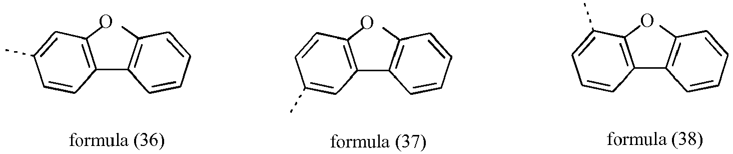 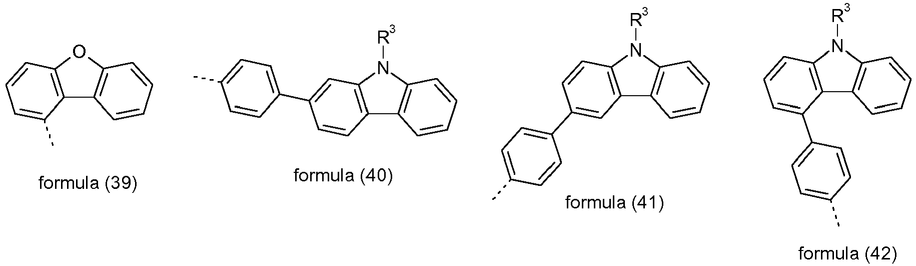 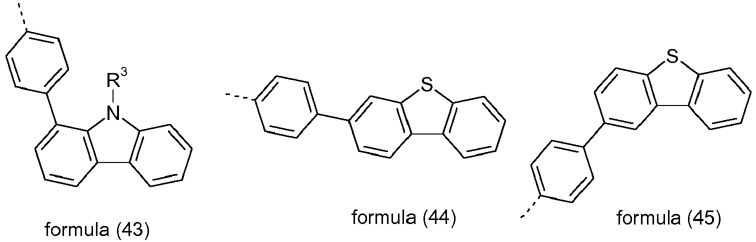 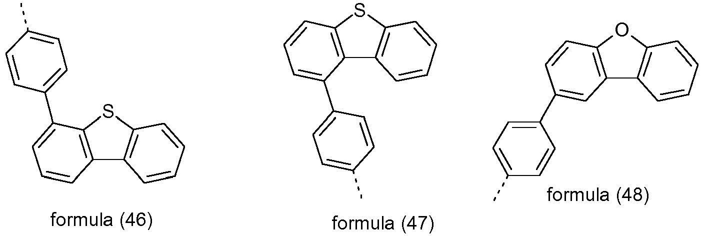 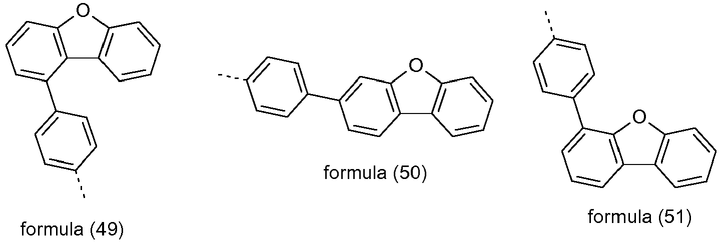 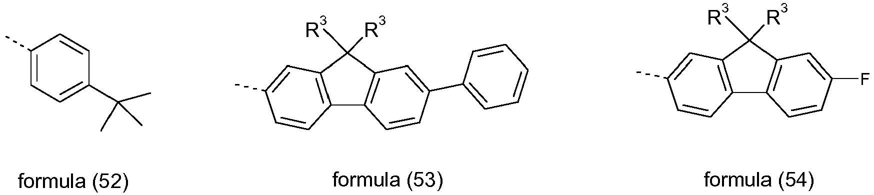 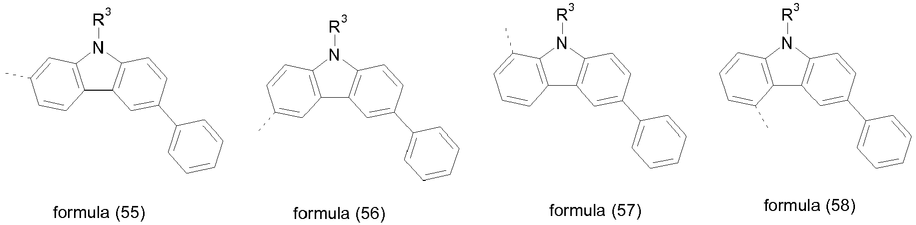 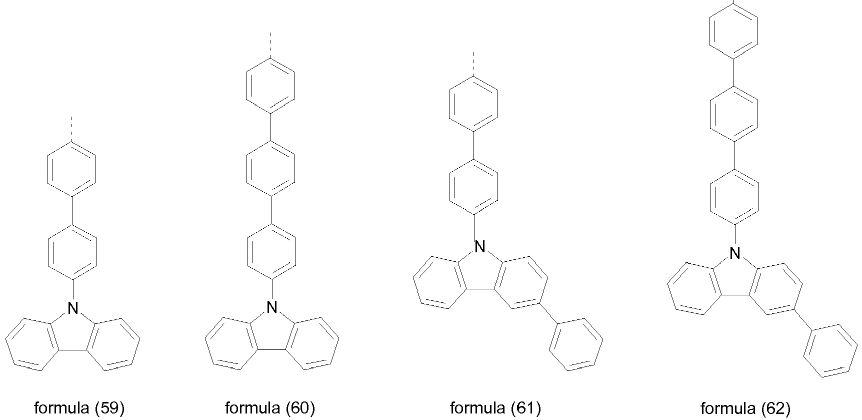 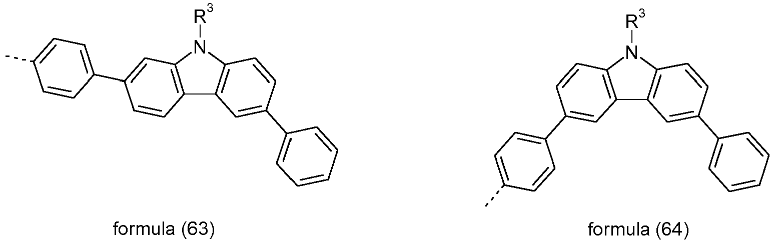 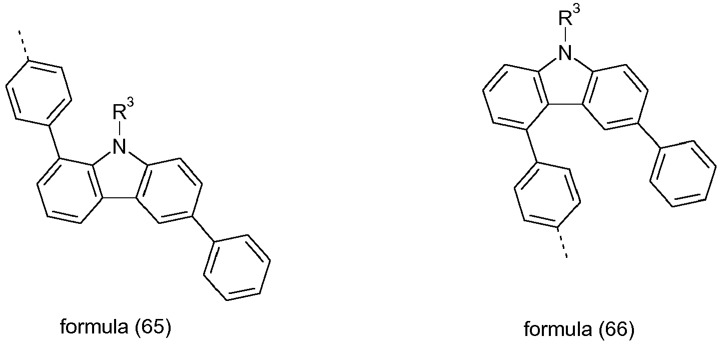 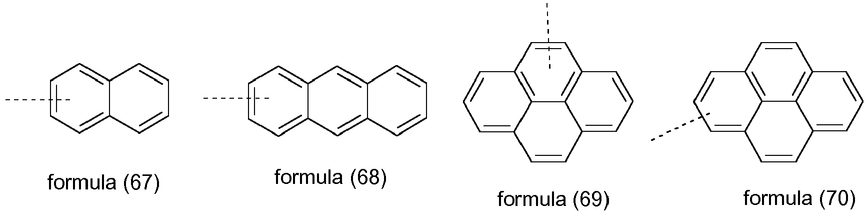 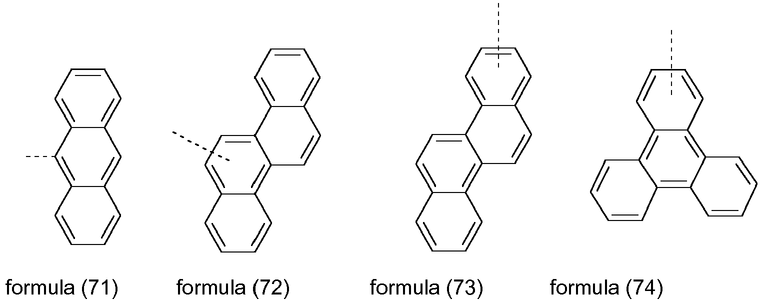 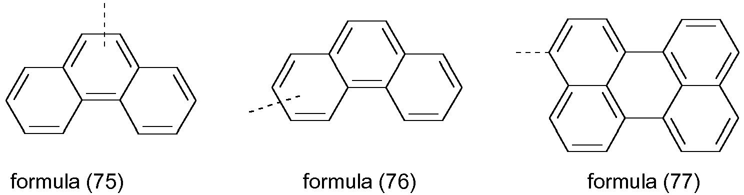 where the dashed lines represent the bonds to the N atom and the group Ar¹ or Ar², and the groups may be substituted by one or more radicals R³ as defined in claim 1.

It is very particularly preferred that both groups Ar¹ and Ar² are selected from the groups of formulae (11) to (77).

If the groups Ar¹ and Ar² in the compounds of the formulae (1), (1 -1), (1-1-1), (1-1-2), (1-1-3) and (1-1-4) or the preferred embodiment are linked to one another by a group E, the group -NAr¹Ar² preferably has the structure of one of the following formulae (78) to (85), more preferably one of the formulae (78) to (81):

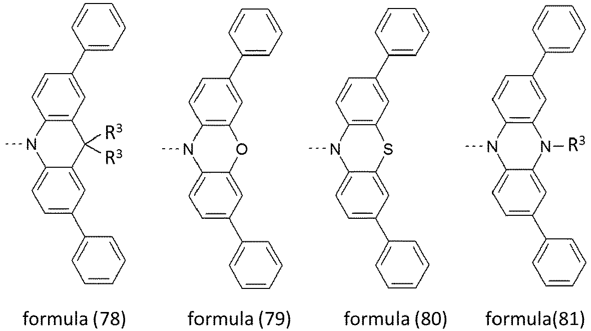

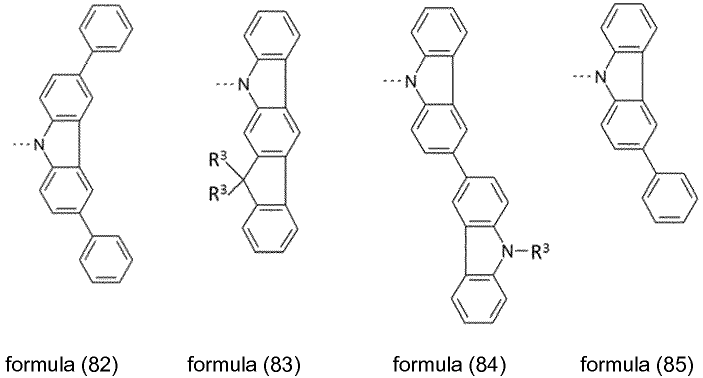

Particular preference is given to compounds of the formulae (1), (1-1), (1-1-1), (1-1-2), (1-1-3) and (1-1-4), in which the preferred embodiments mentioned above occur simultaneously. Particular preference is therefore given to compounds for which:
- Y: is a group of one of the formulae (Y-1) to (Y-6);
- W: is CR¹;
- X: is C(R²)₂;
- Ar¹, Ar²: are, identically or differently, a group of one of the formulae (10) to (77); or -NAr¹Ar² stands for a group of one of the formulae (78) to (81);
- R¹: is selected, identically or differently on each occurrence, from the group consisting of H, D, F, CN, Si(R³)₃, N(R³)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R³ and where one or more CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, or an aromatic or heteroaromatic ring system having 5 to 20 aromatic ring atoms, which may in each case be substituted by one or more radicals R³, where two adjacent radicals R¹ located on Ar¹ or two adjacent radicals R¹ located on Ar² may be linked to one another and may form a ring.
- R²: is selected, identically or differently on each occurrence from the group consisting of H, D, F, CN, Si(R³)₃, N(R³)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R³ and where one or more CH₂ groups in the above-mentioned groups may be replaced by -C=C-, -R³C=CR³-, Si(R³)₂, C=O, C=NR³, -NR³-, -O-, -S-, -C(=O)O- or -C(=O)NR³-, or an aromatic or heteroaromatic ring system having 5 to 20 aromatic ring atoms, which may in each case be substituted by one or more radicals R³; where two radicals R² may be linked to one another and may form a ring.
- R³: is selected, identically or differently on each occurrence from the group consisting of H, D, F, CN, Si(R⁴)₃, N(R⁴)₂, a straight-chain alkyl or alkoxy group having 1 to 10 C atoms or a branched or cyclic alkyl or alkoxy group having 3 to 10 C atoms, where the above-mentioned groups may each be substituted by one or more radicals R⁴ and where one or more CH₂ groups in the above-mentioned groups may be replaced by -C=C-, -R⁴C=CR⁴-, Si(R⁴)₂, C=O, C=NR⁴, -NR⁴-, -O-, -S-, -C(=O)O- or -C(=O)NR⁴-, or an aromatic or heteroaromatic ring system having 5 to 20 aromatic ring atoms, which may in each case be substituted by one or more radicals R⁴, where two or more radicals R³ may be linked to one another and may form a ring.
- m: is 0 or 1;
- n: is 0.

The following table shows examples of compounds of the formula (1):

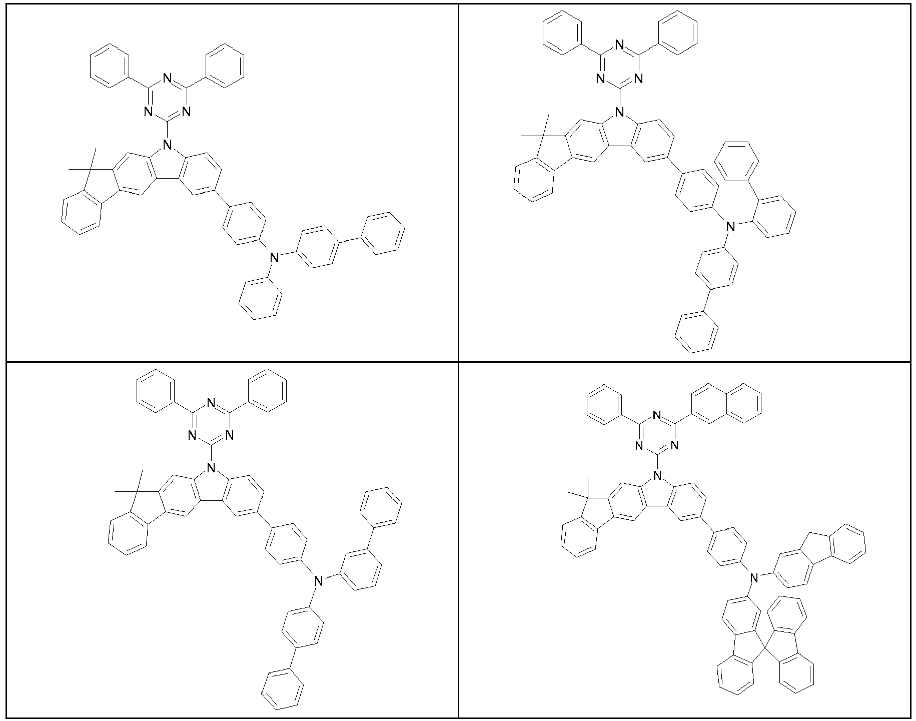

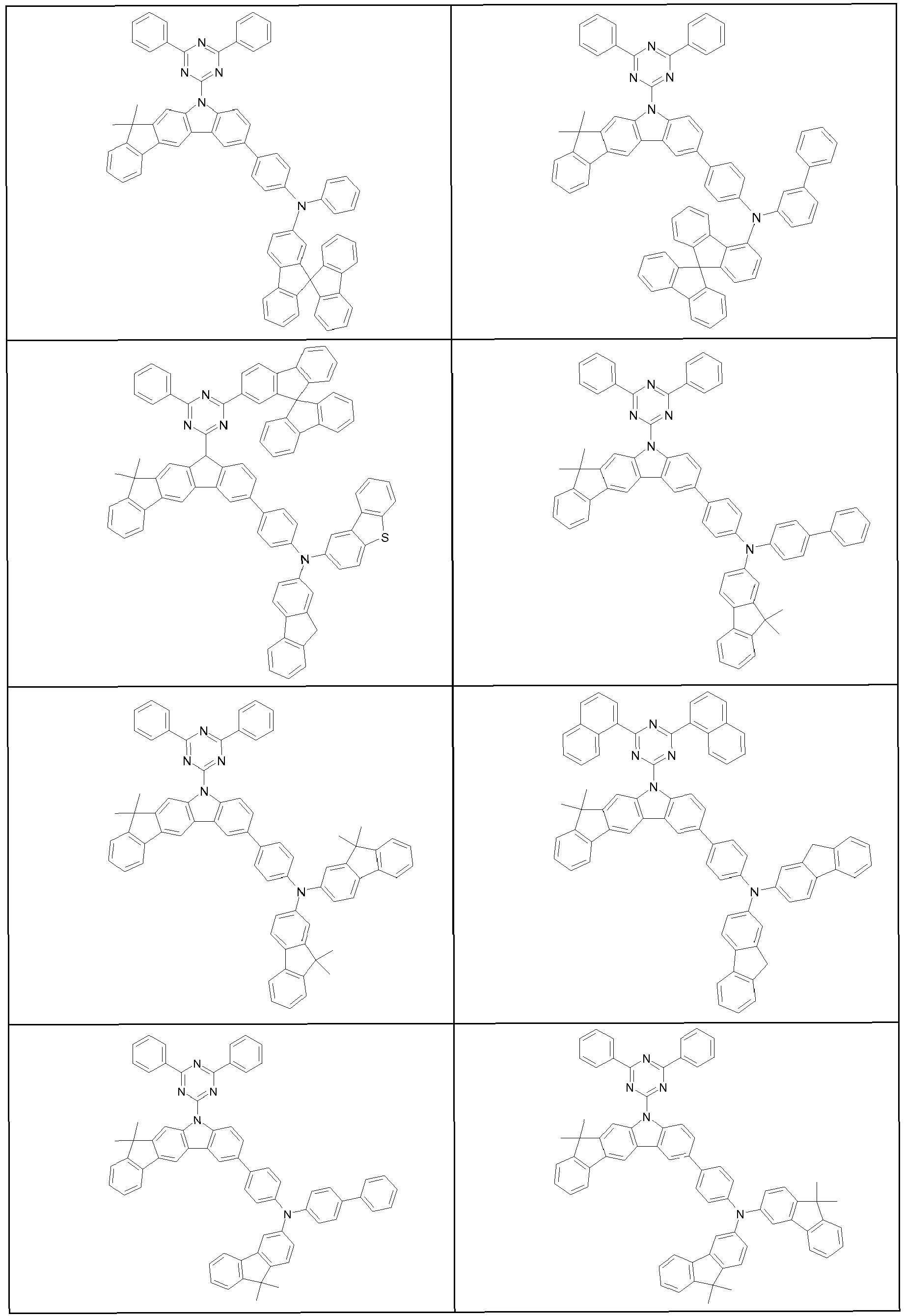

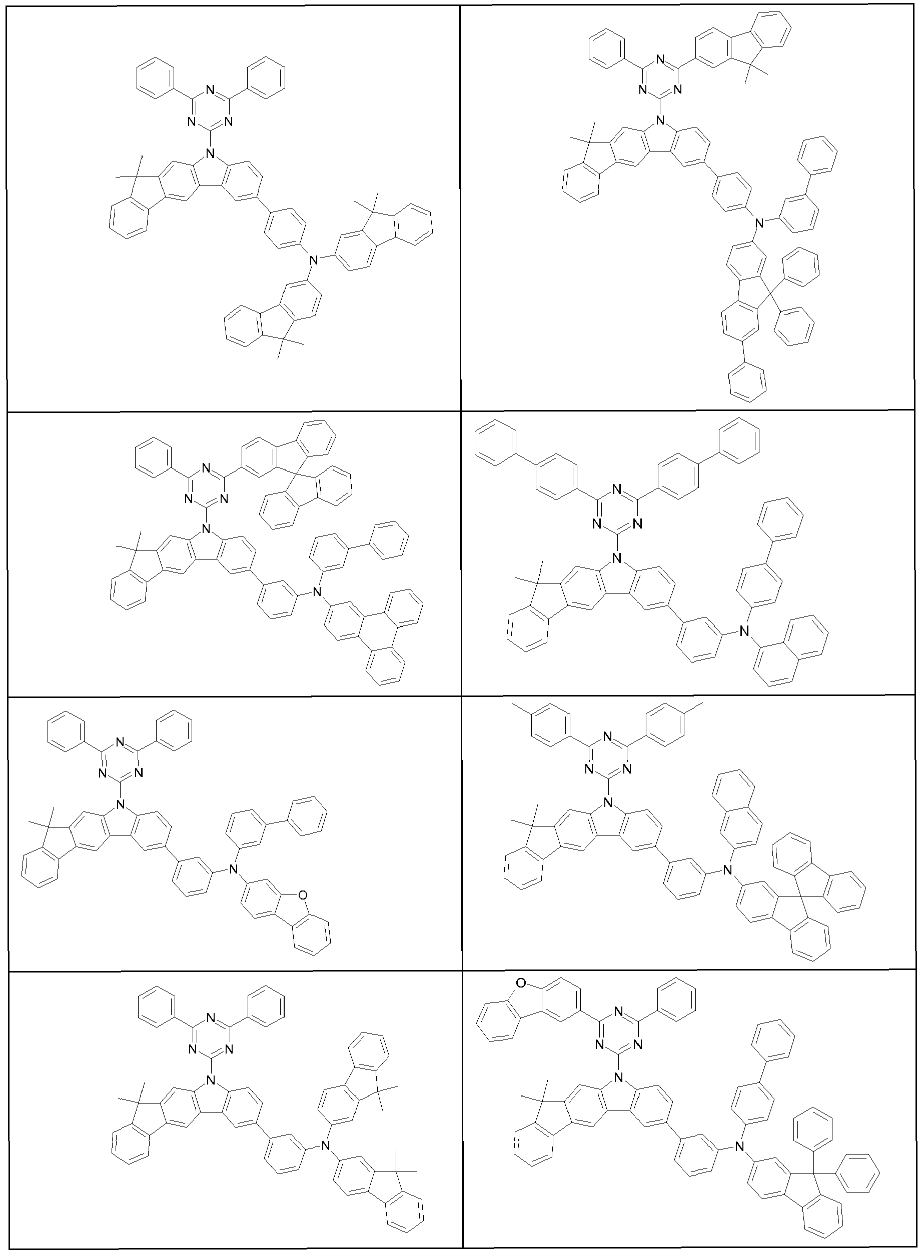

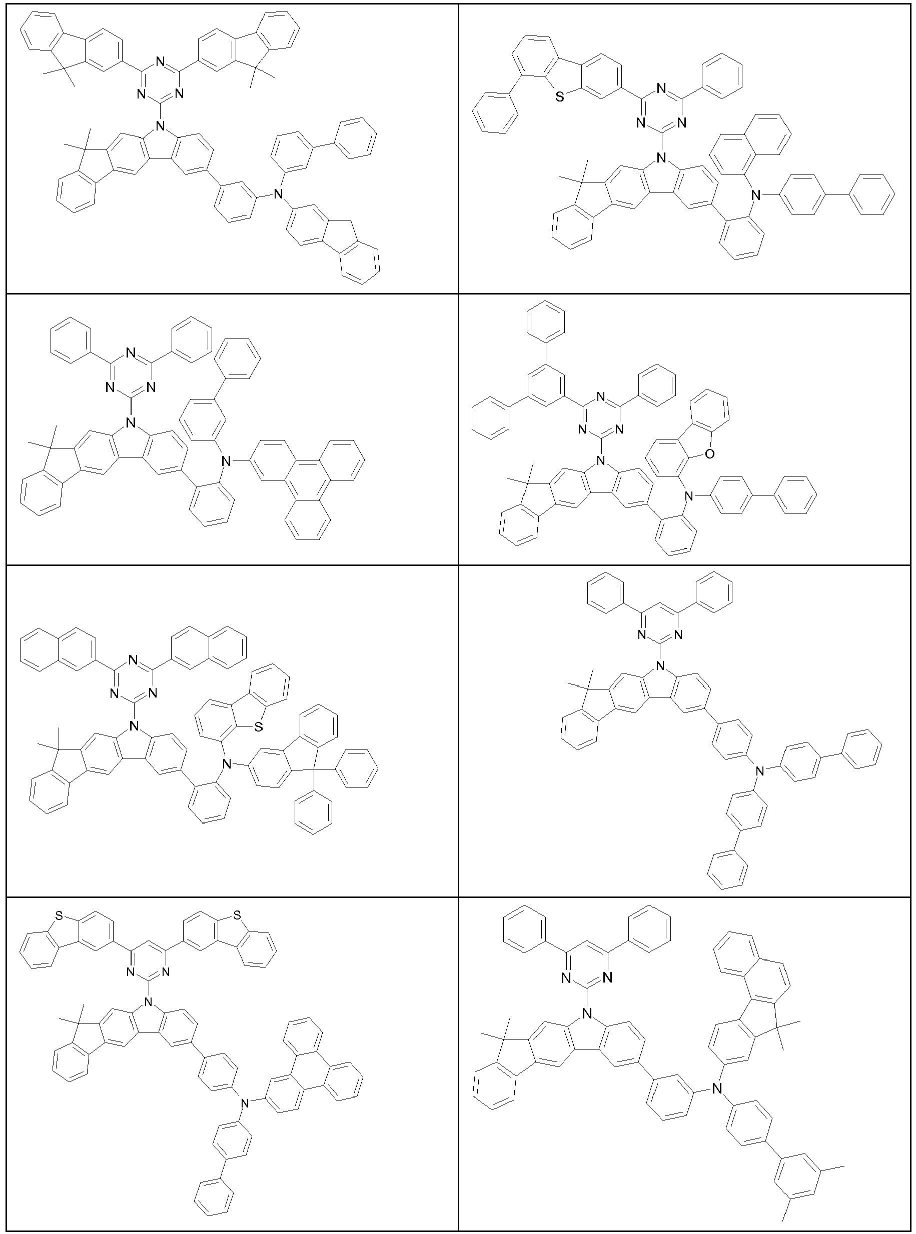

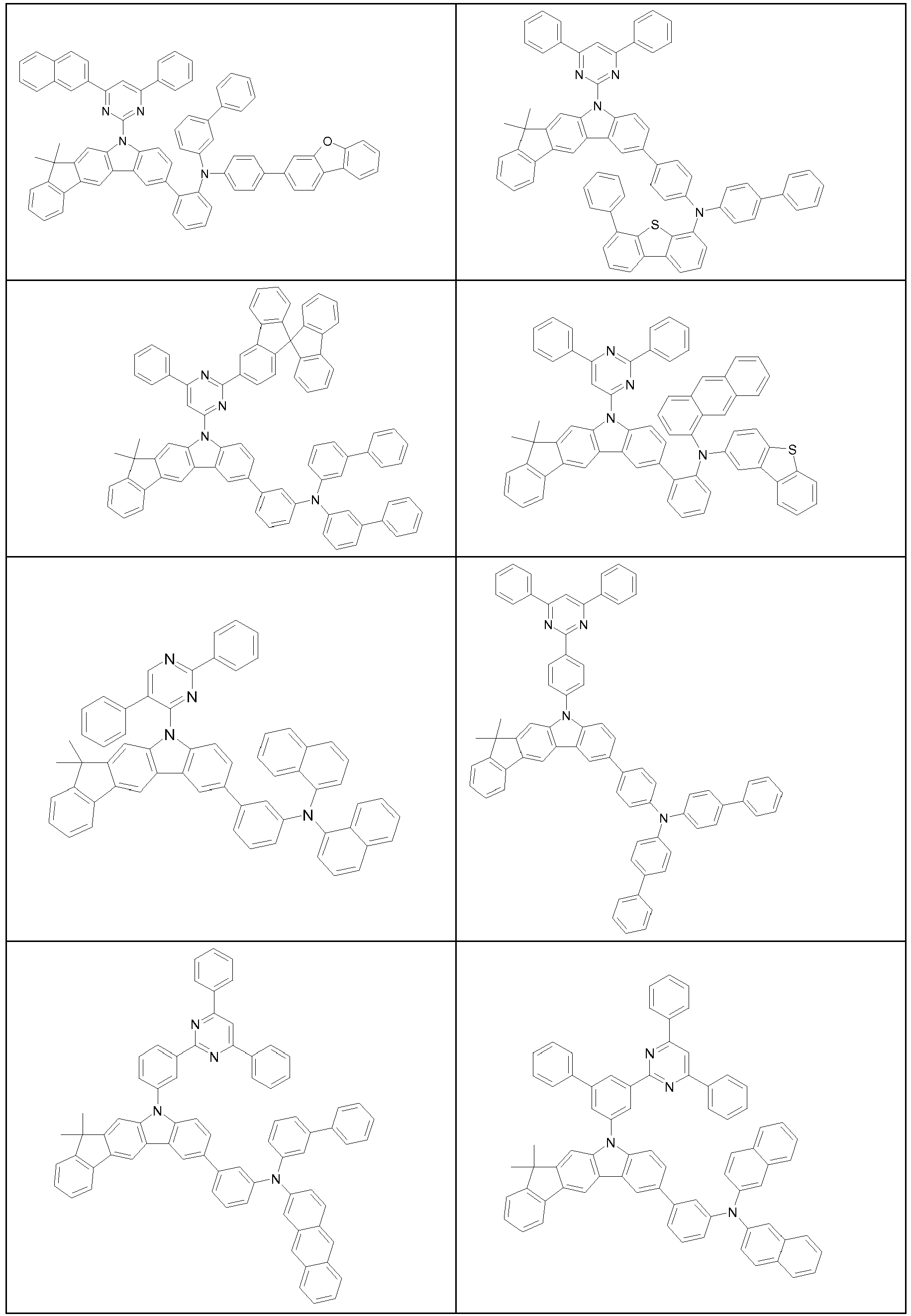

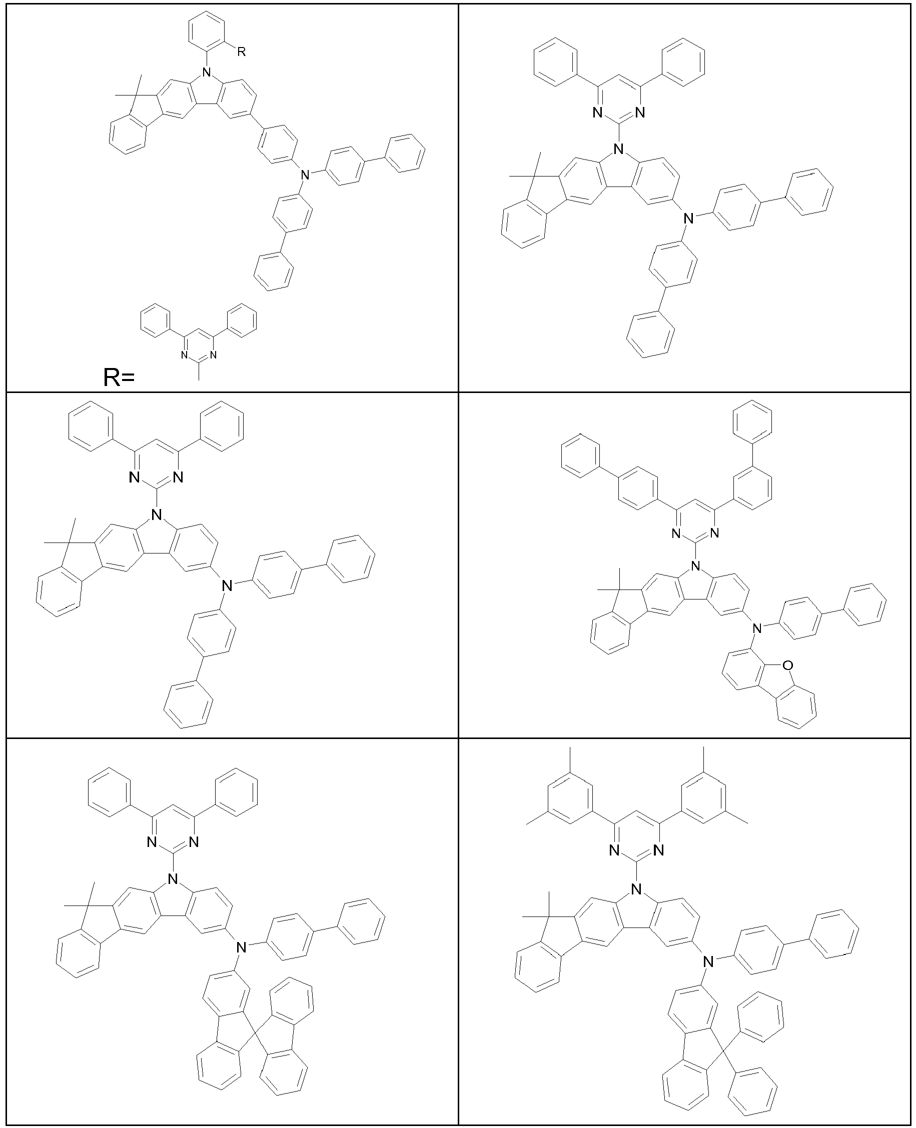

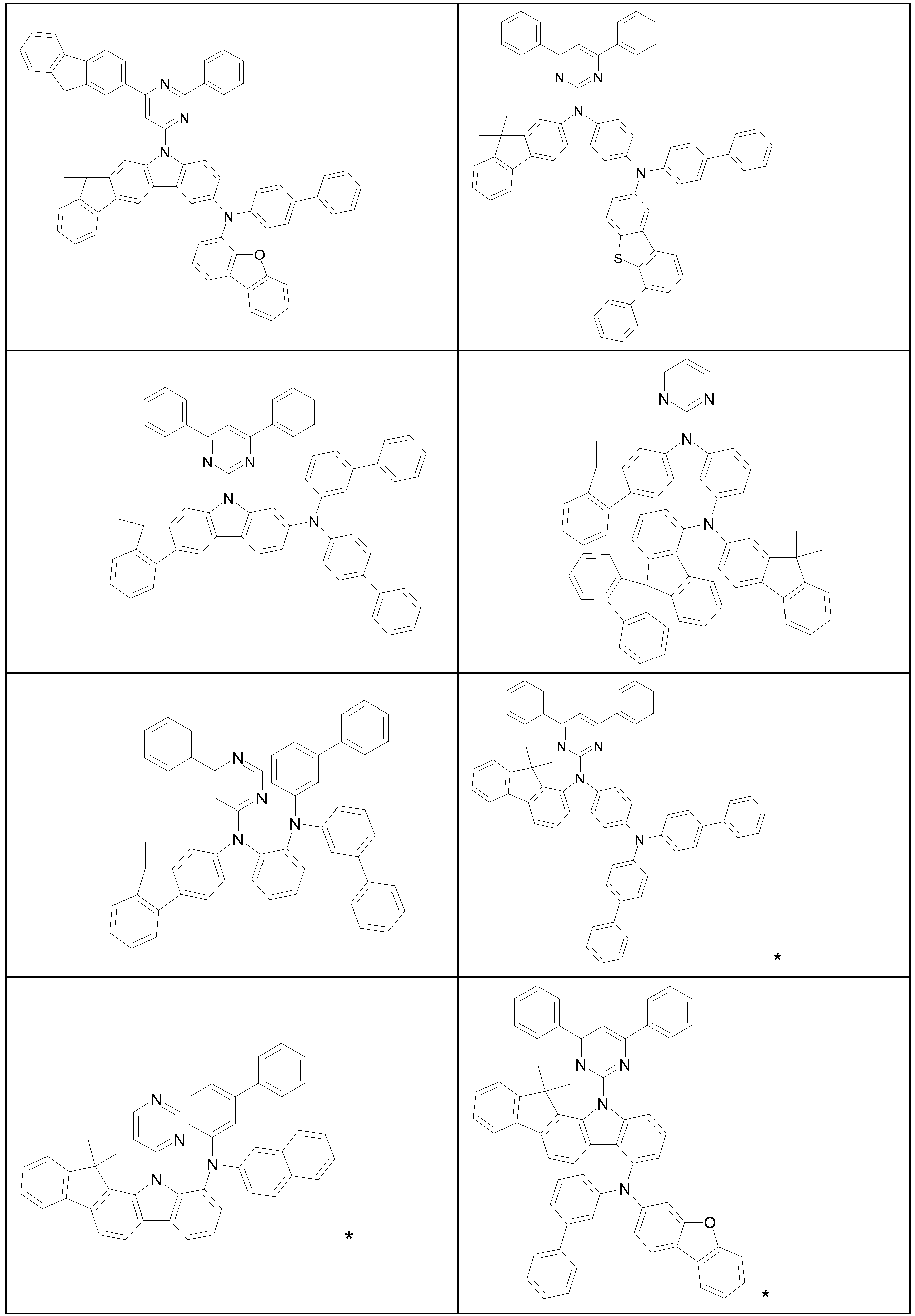

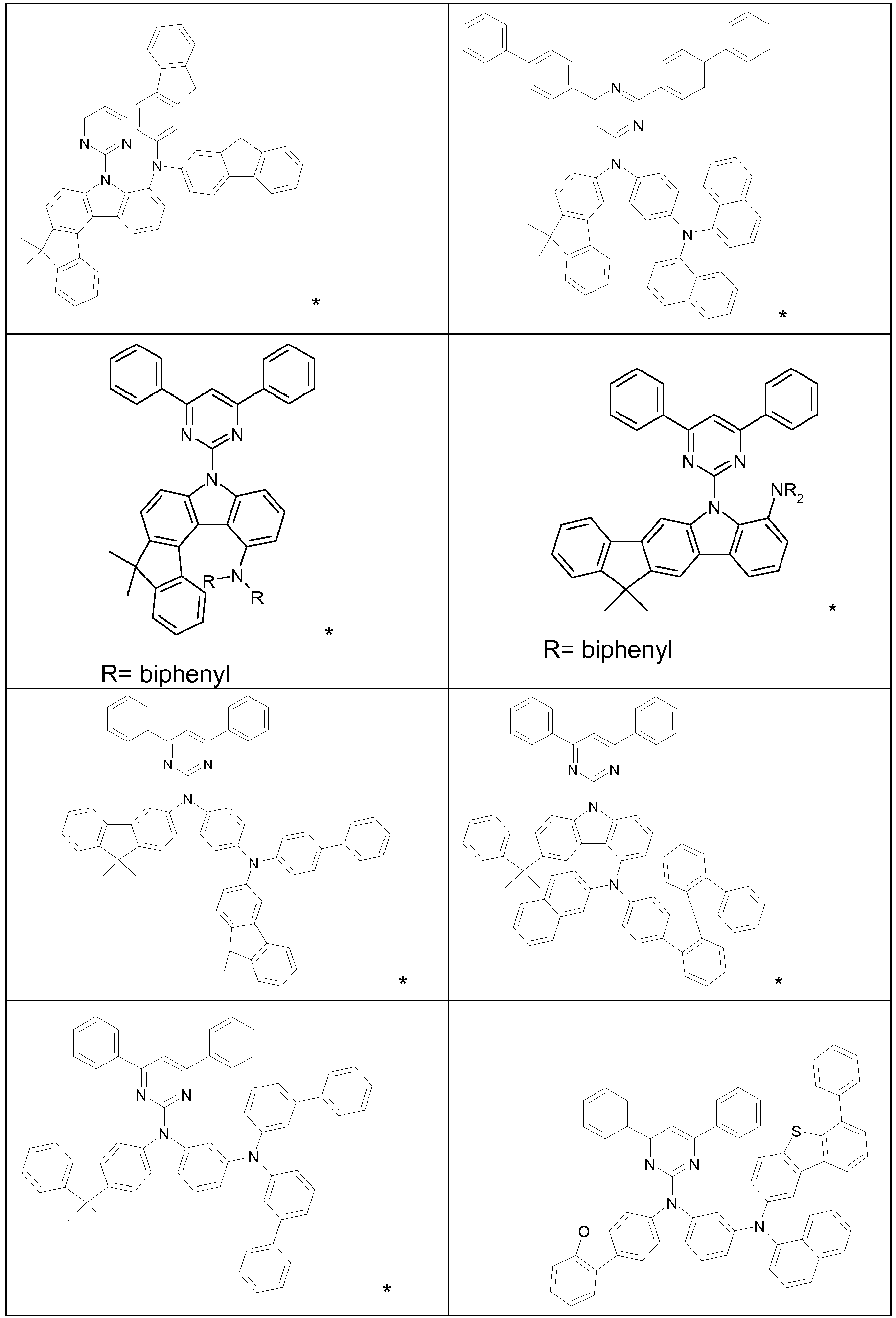

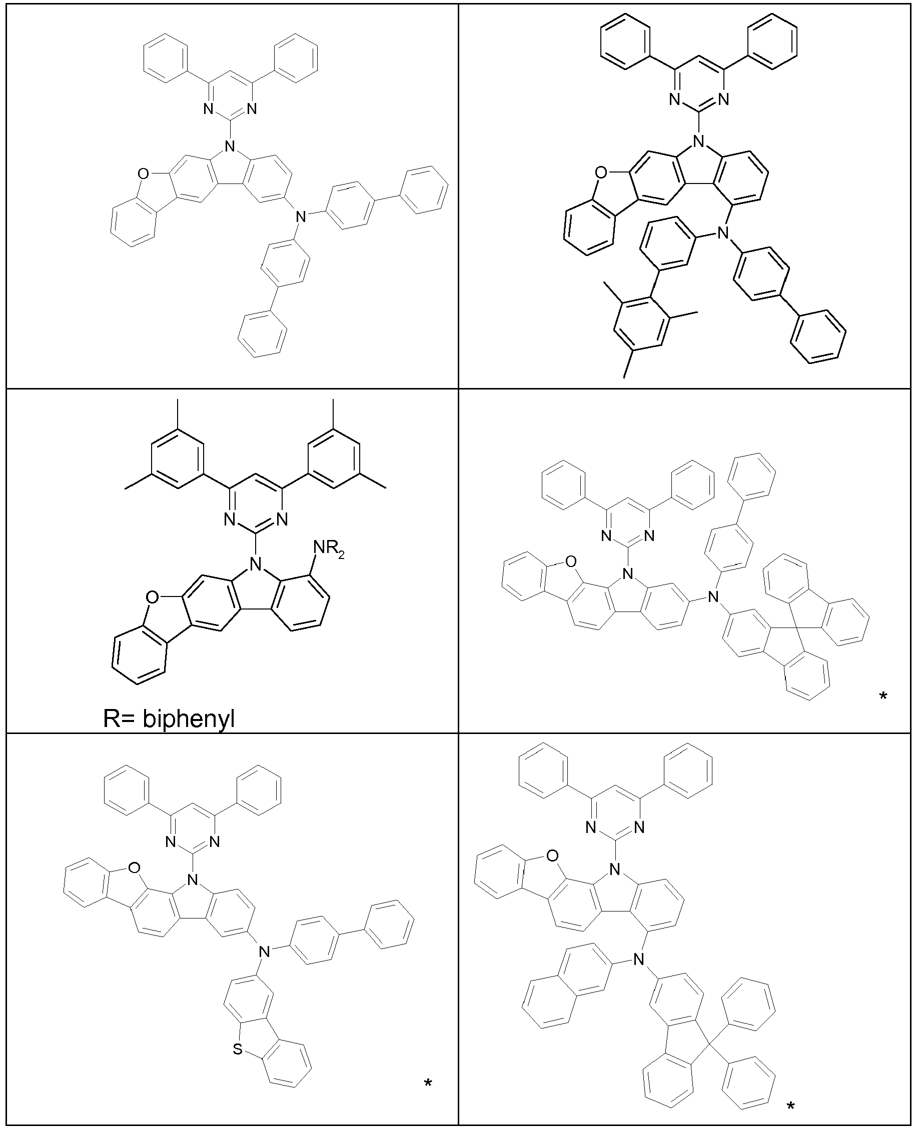

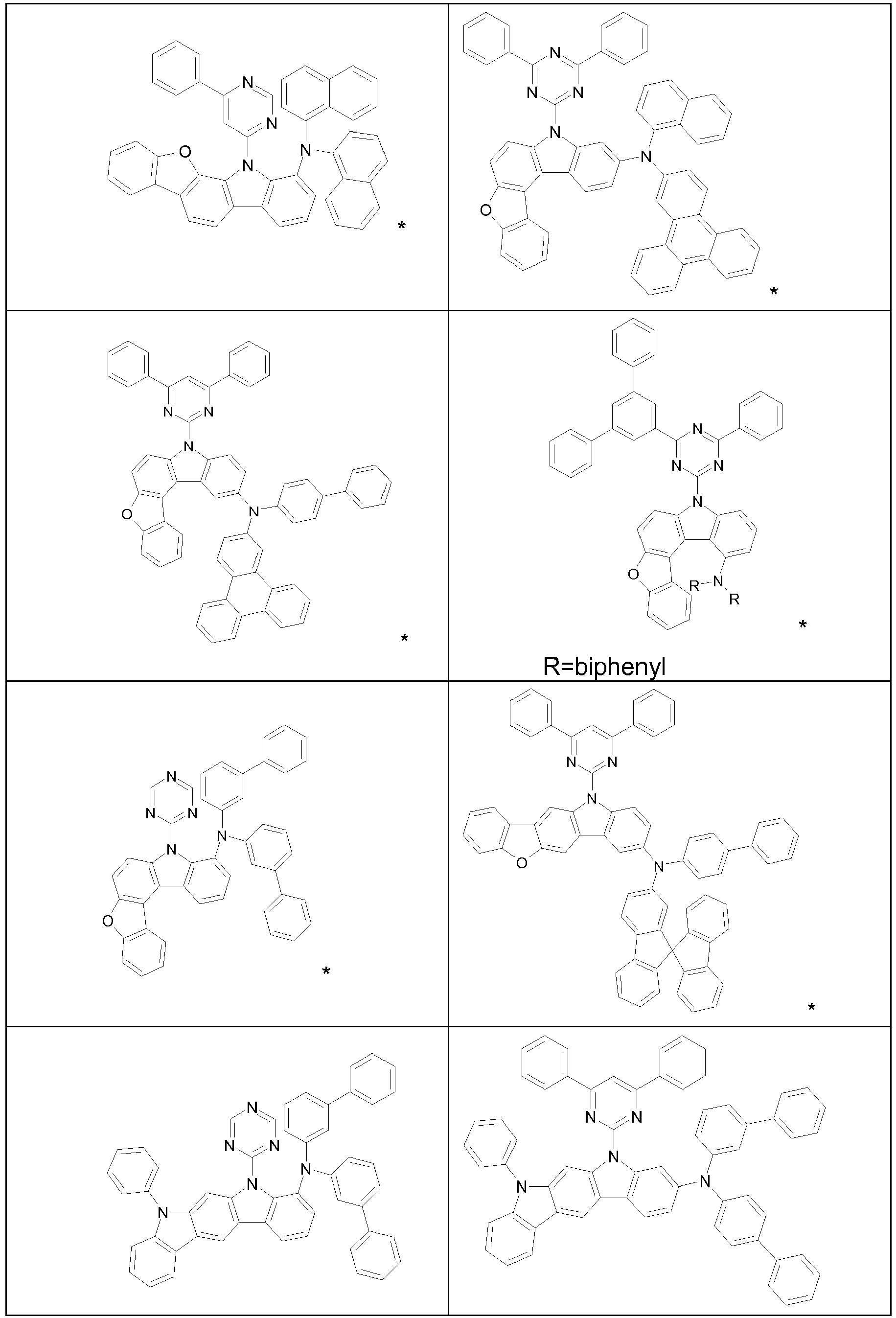

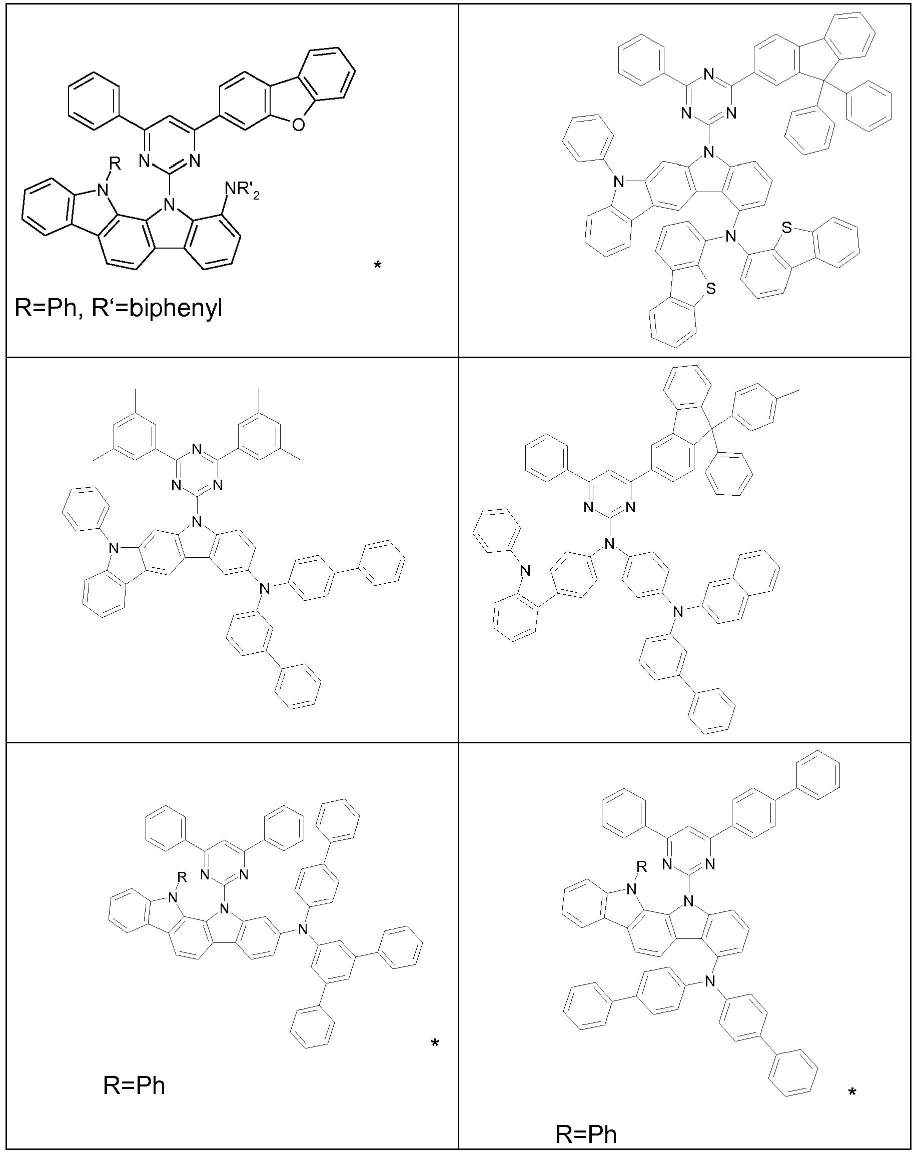

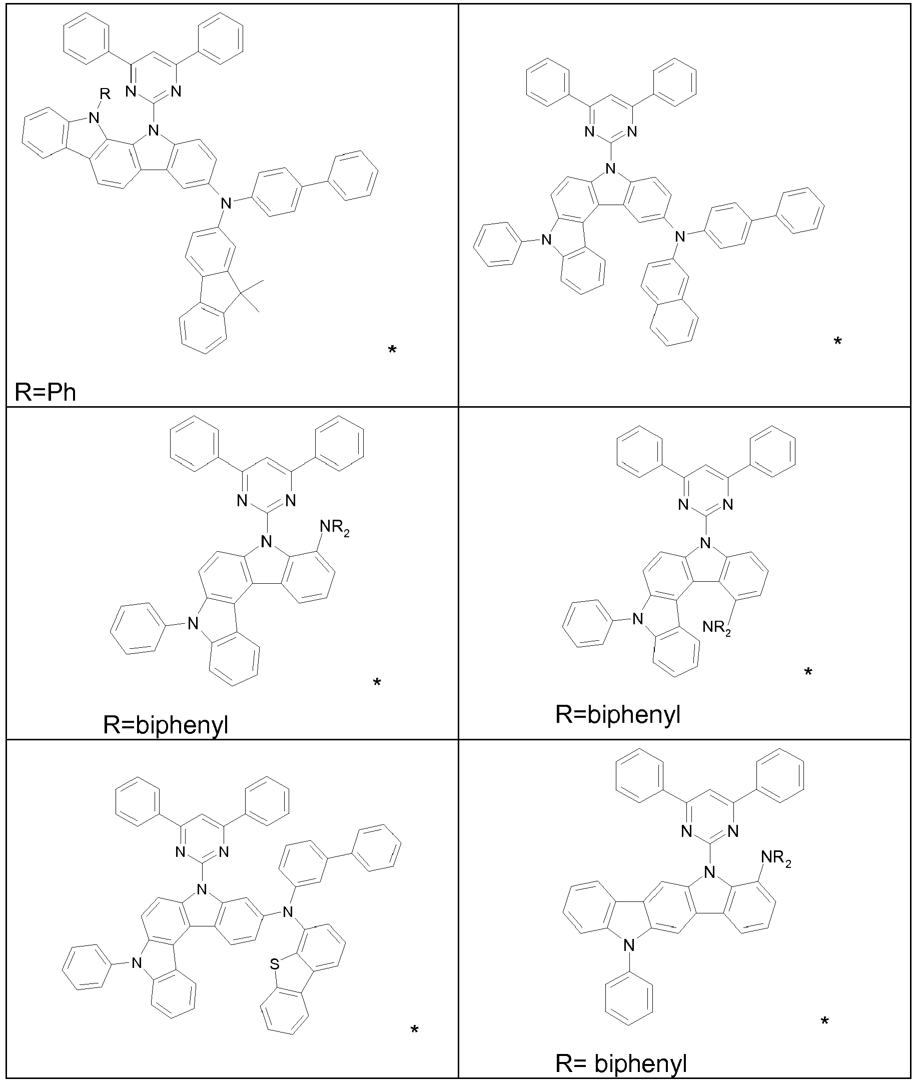

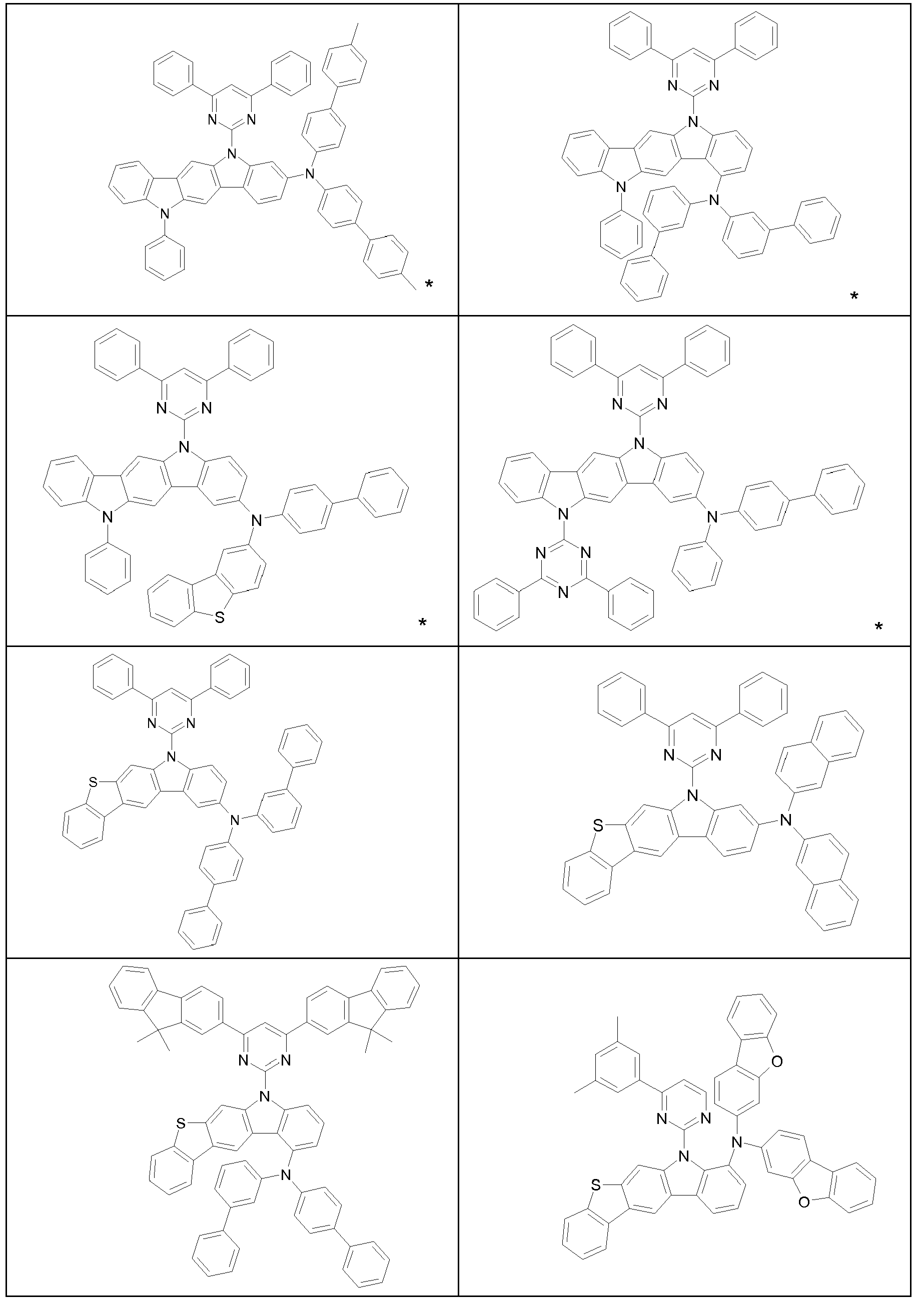

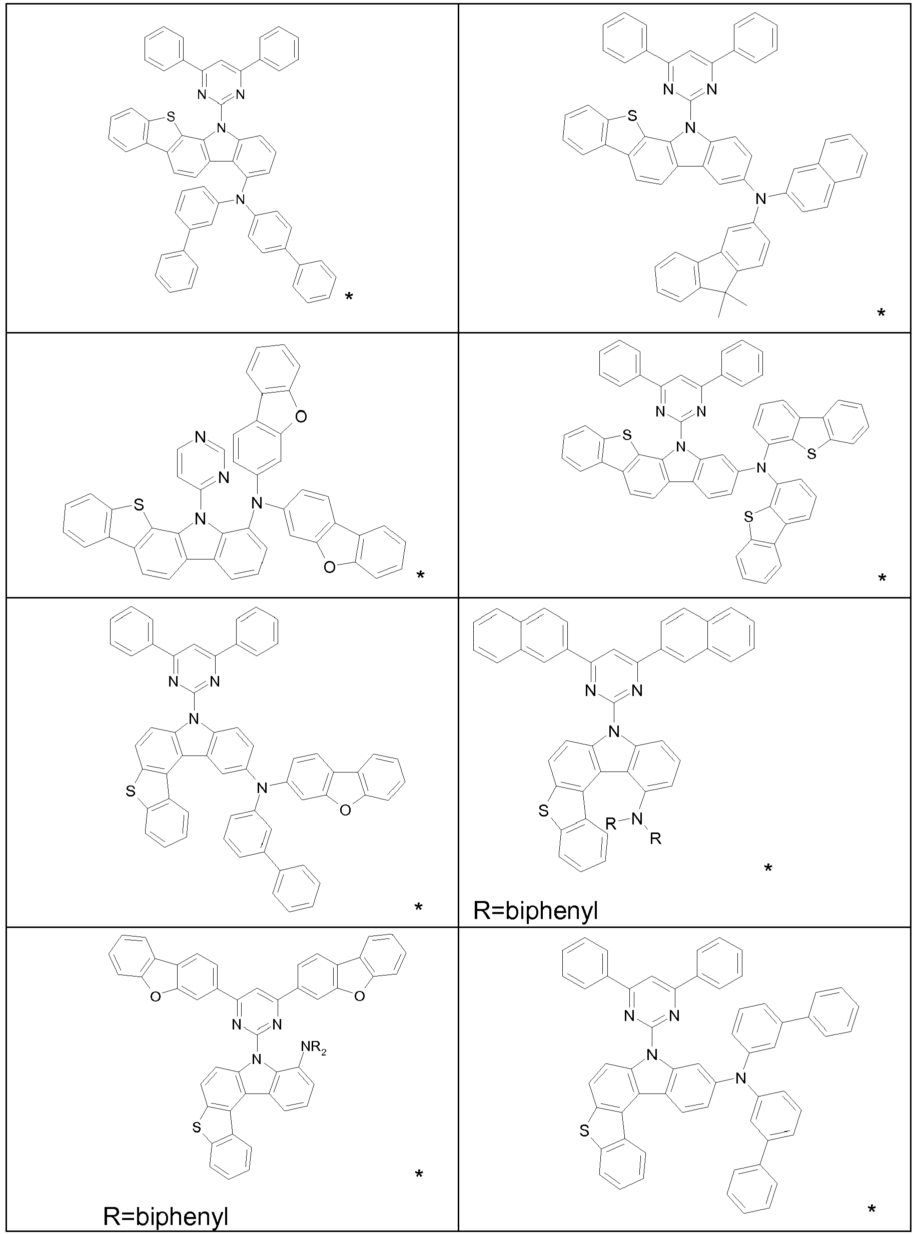

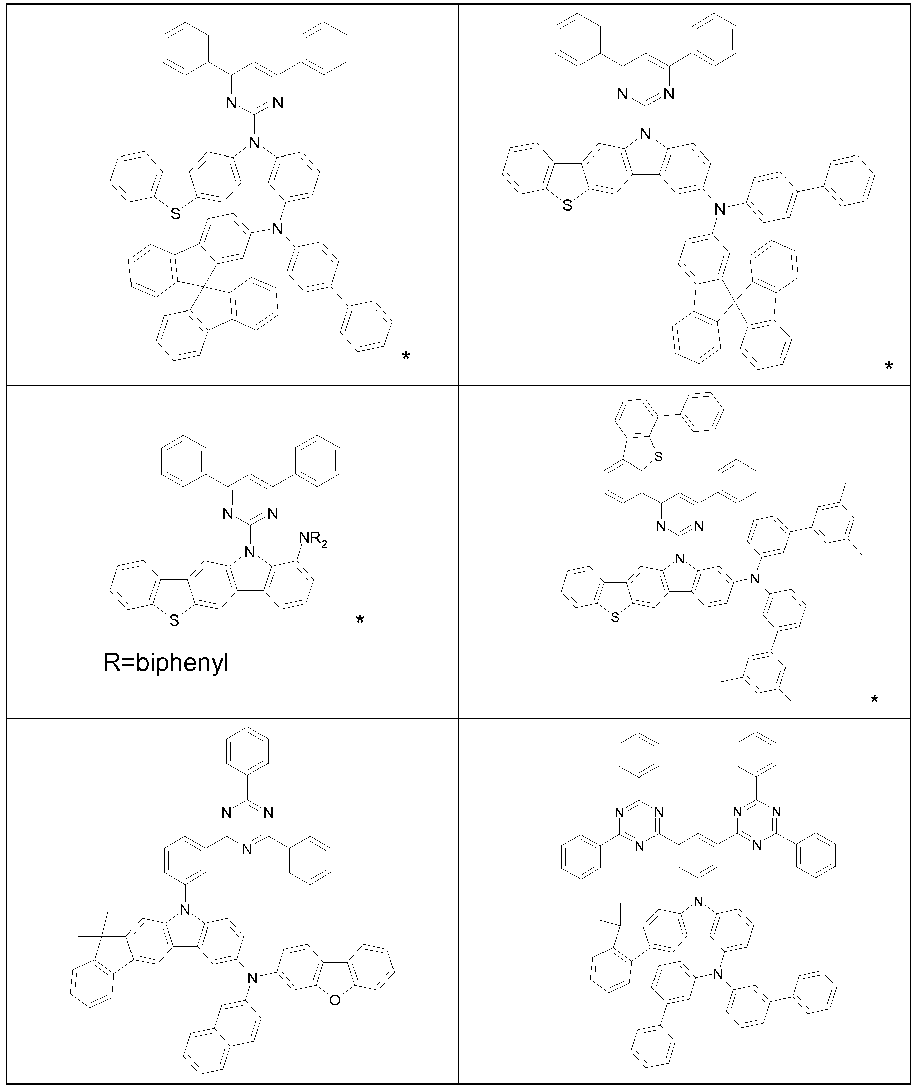

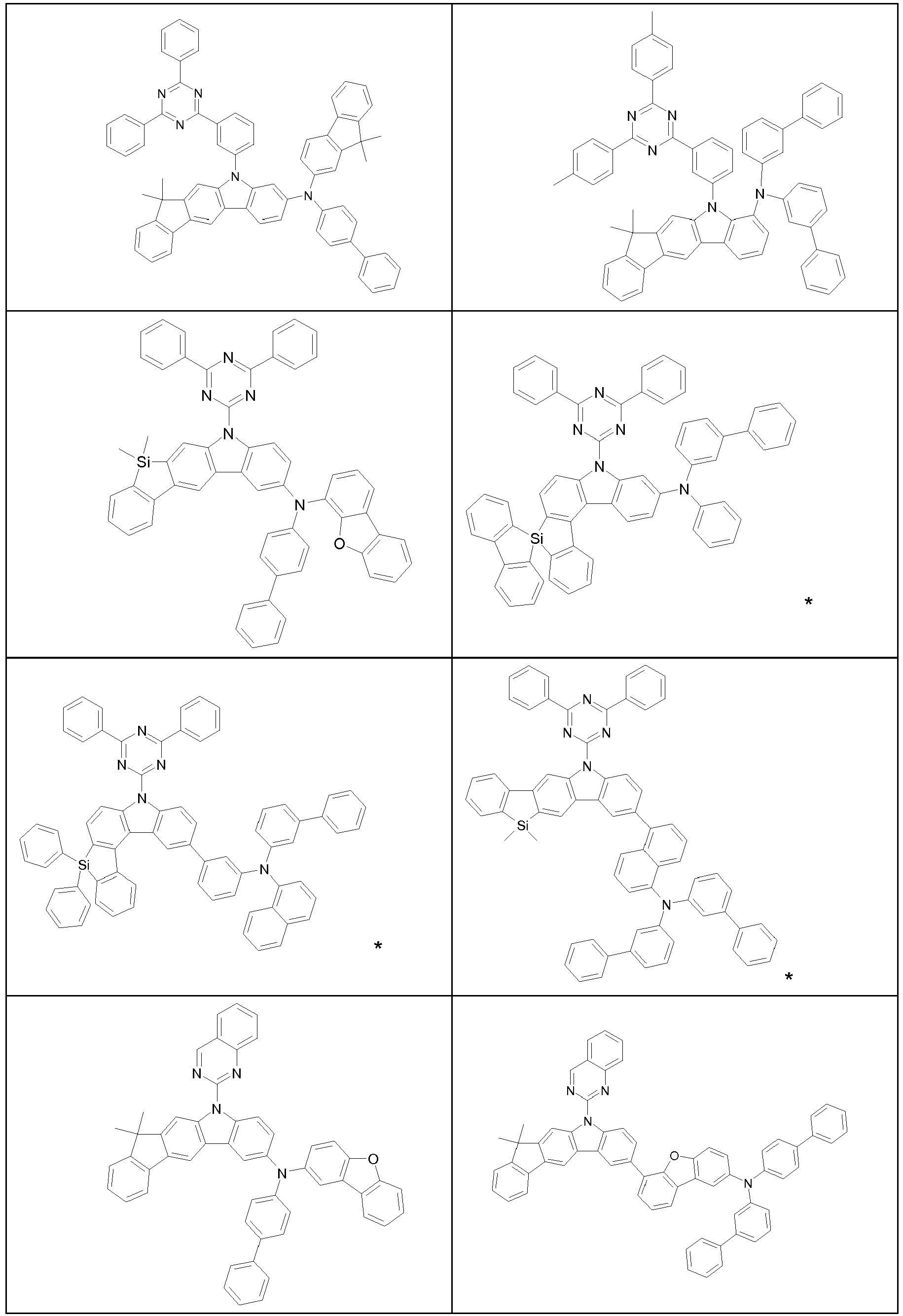

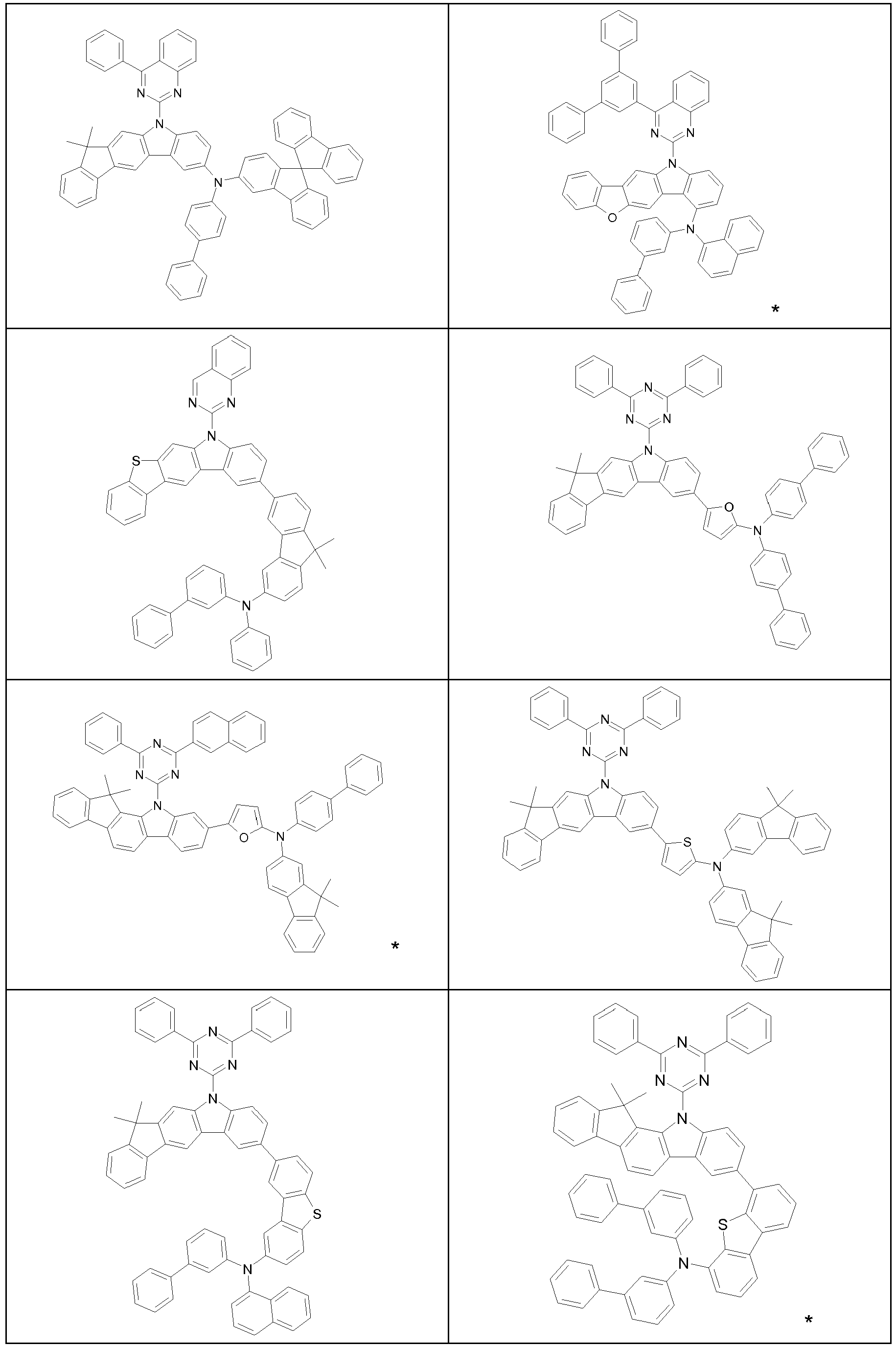

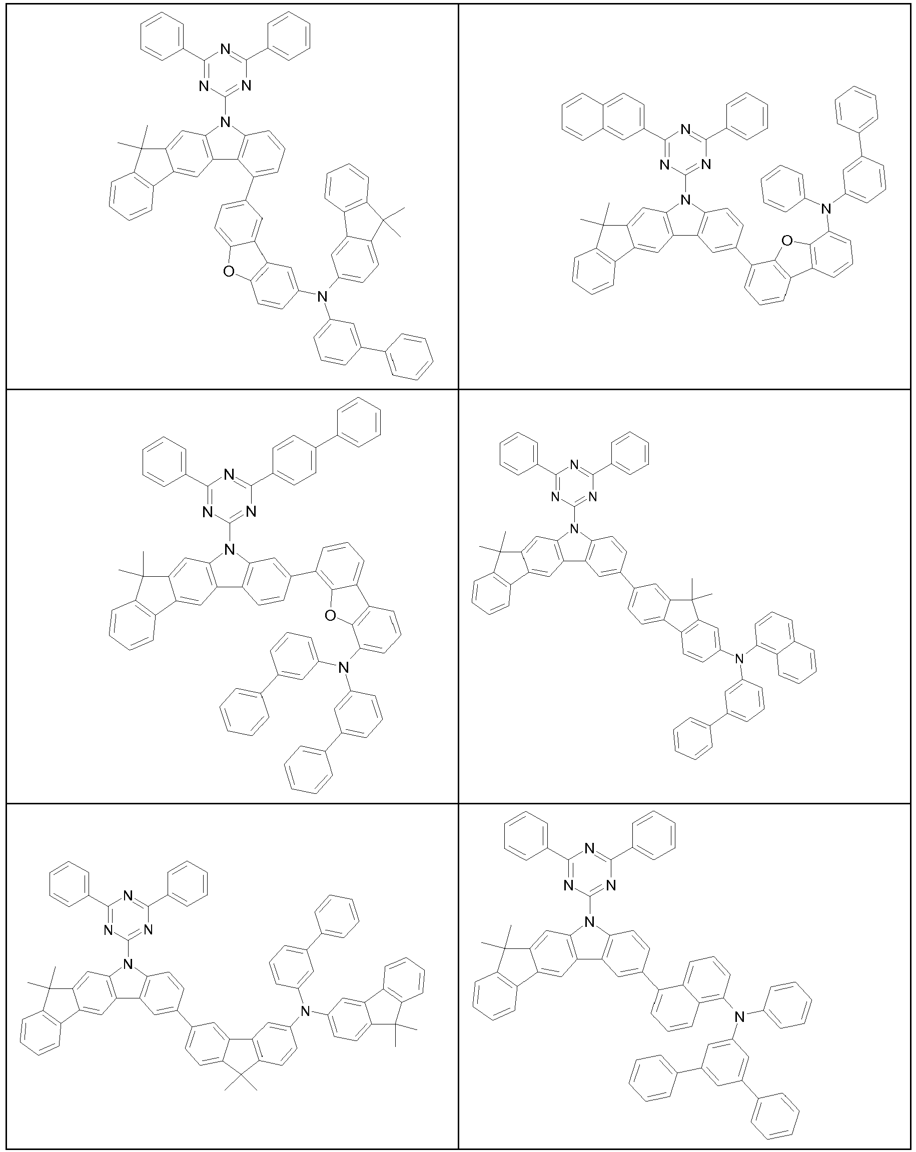

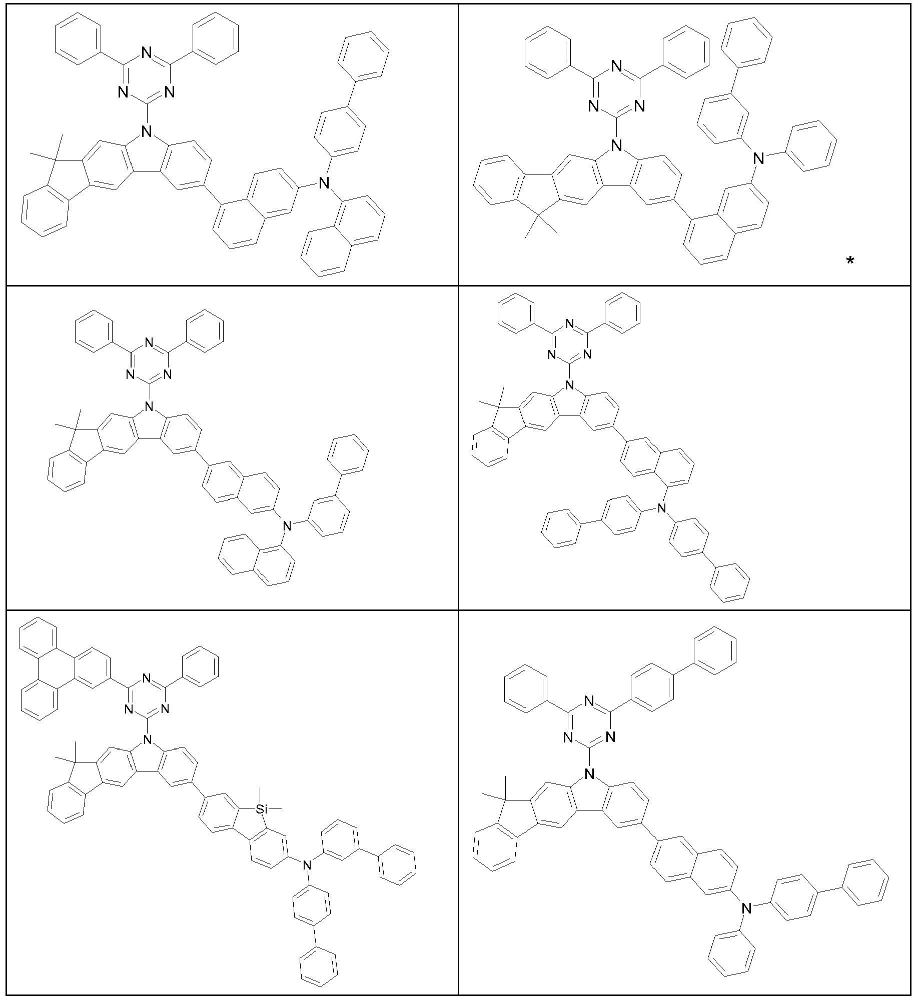

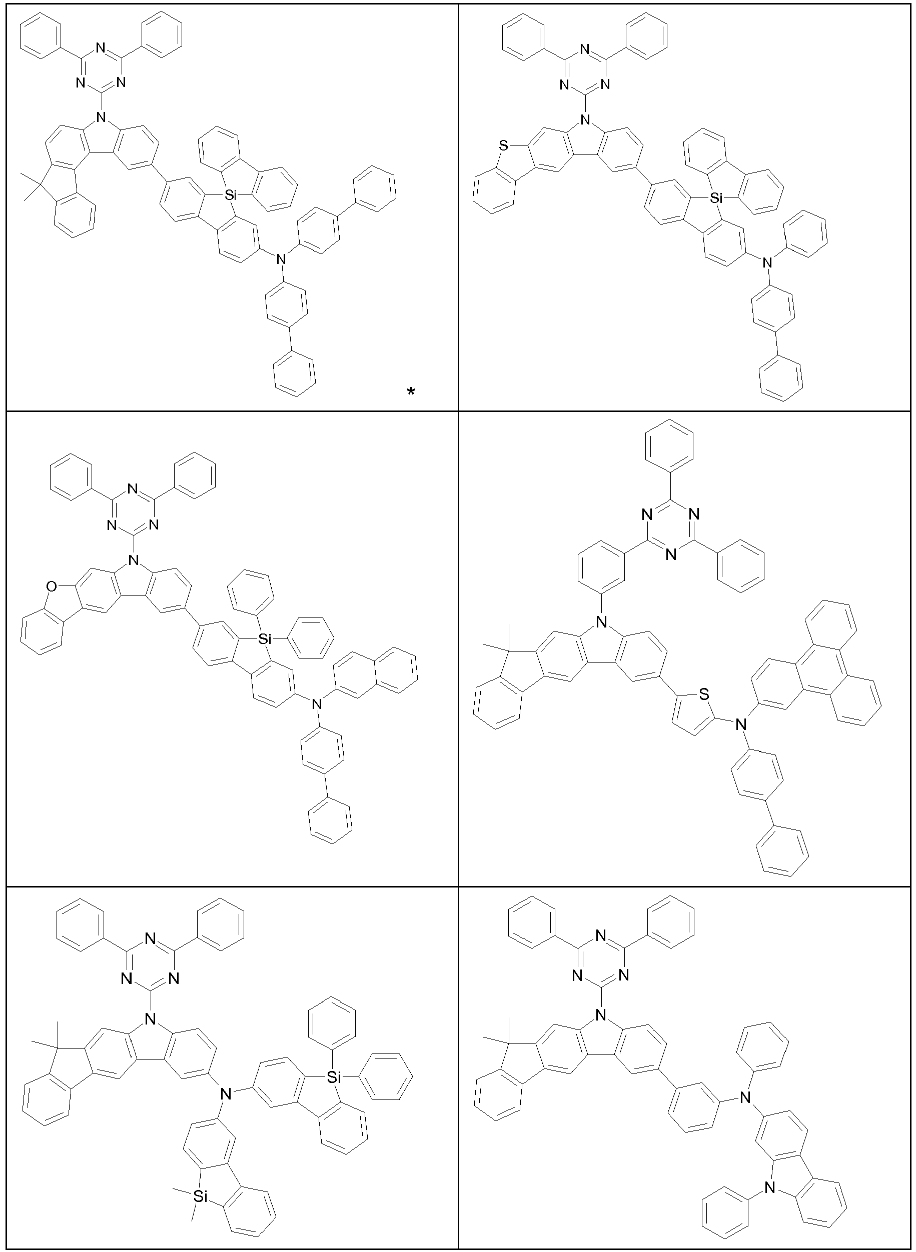

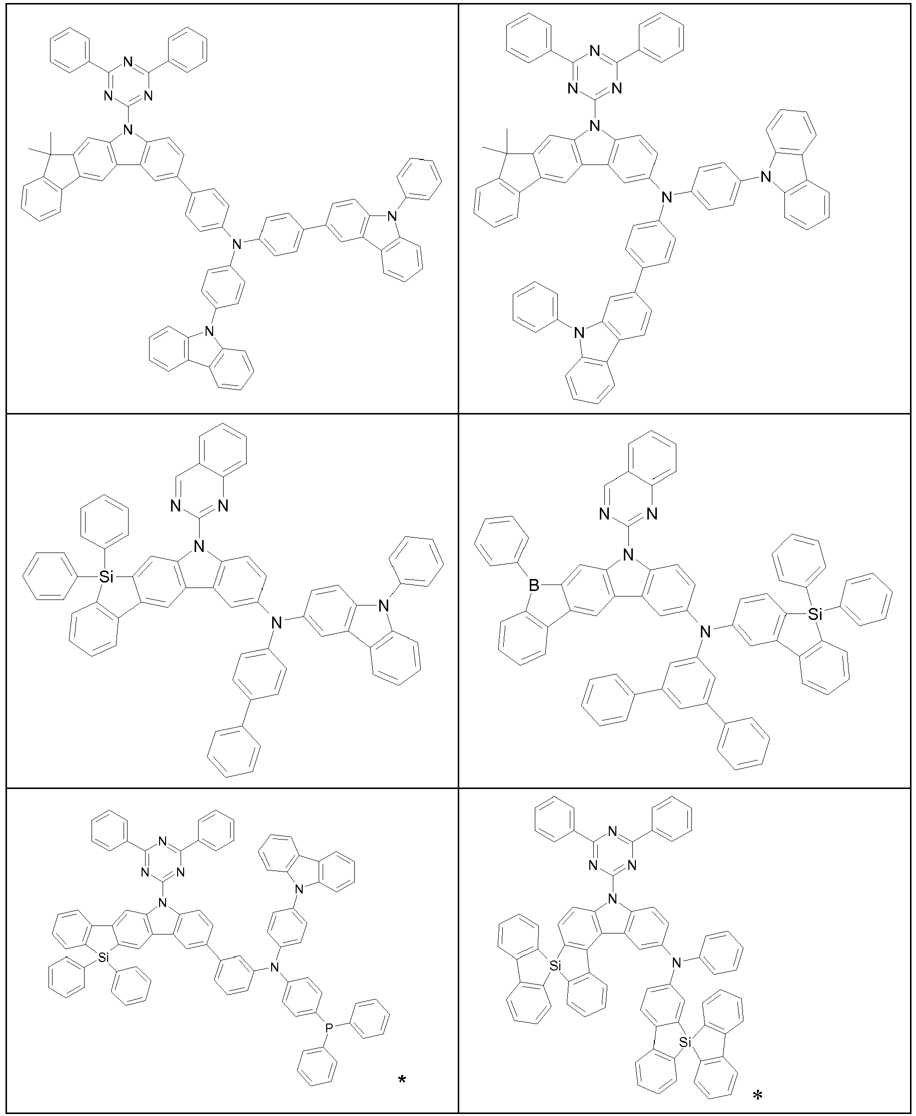

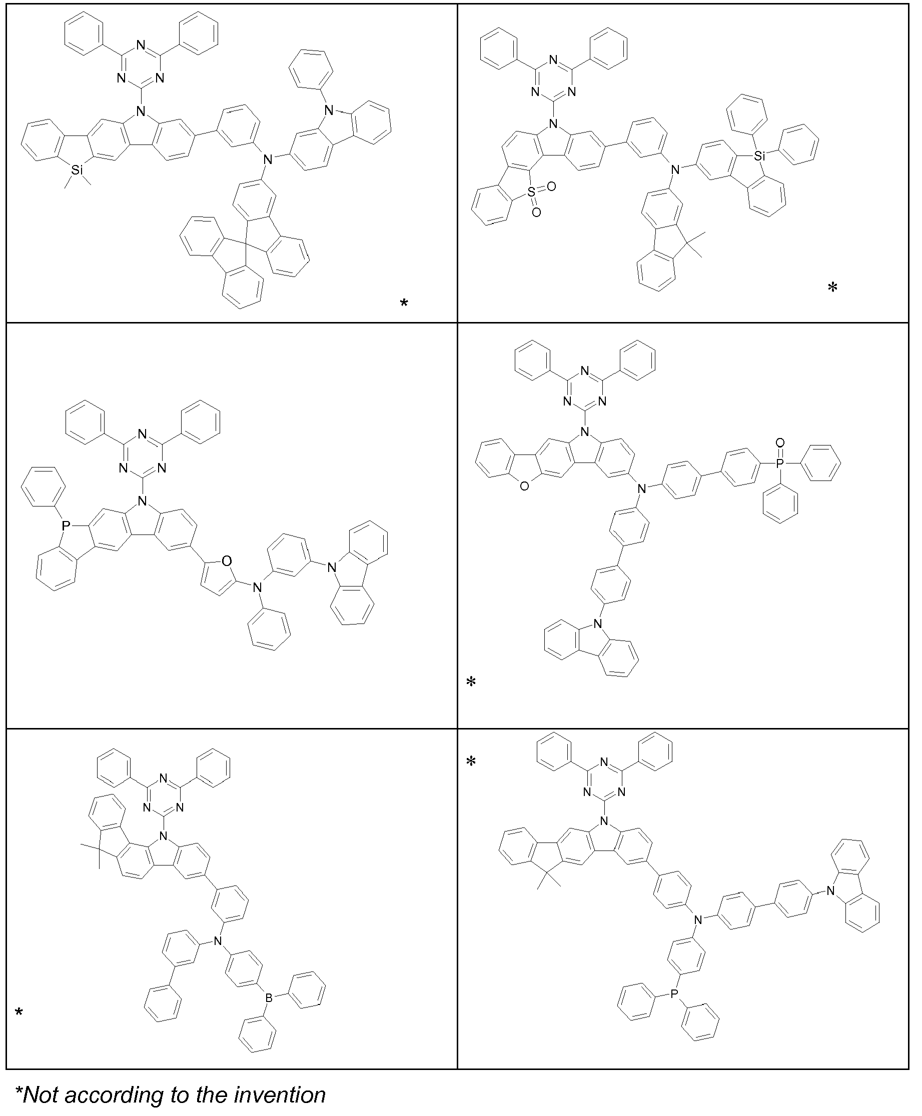

*

The compounds according to the invention can be prepared by means of known synthetic steps of organic chemistry. These include, for example, transition metal-catalysed coupling reactions, such as Suzuki and Buch-wald coupling, brominations and halogenations.

Illustrative processes for the preparation of the compounds according to the invention are presented below. The processes shown are particularly suitable for the preparation of the compounds according to the invention. However, alternative processes are conceivable and possibly preferable in certain cases. Accordingly, the person skilled in the art will be able to modify the processes shown below within the scope of his general expert knowledge.

The compounds according to the invention are preferably synthesised as shown in Scheme 1. The compounds in Scheme 1 may be substituted at free positions by any desired organic radicals R.

An indenocarbazole compound is reacted in a first step here with a halogenated aryl compound containing an electron-deficient heteroaryl group. The way in which indenocarbazole compounds of this type can be prepared is known from the prior art and is illustrated by the working examples in this application. The first step is preferably carried out under the conditions of a Buchwald coupling. A halogenation on the indenocarbazole skeleton is subsequently carried out. This is preferably a bromination, particularly preferably using the reagent NBS.

This step is preferably followed by a Suzuki coupling to a triarylamine derivative which carries a boronic acid substituent or this step is preferably followed by a Buchwald coupling with a diarylamine derivative.

The product obtained may already represent the target compound and conforms to formula (1). However, further steps may follow, for example in order to introduce further functional groups or radicals.

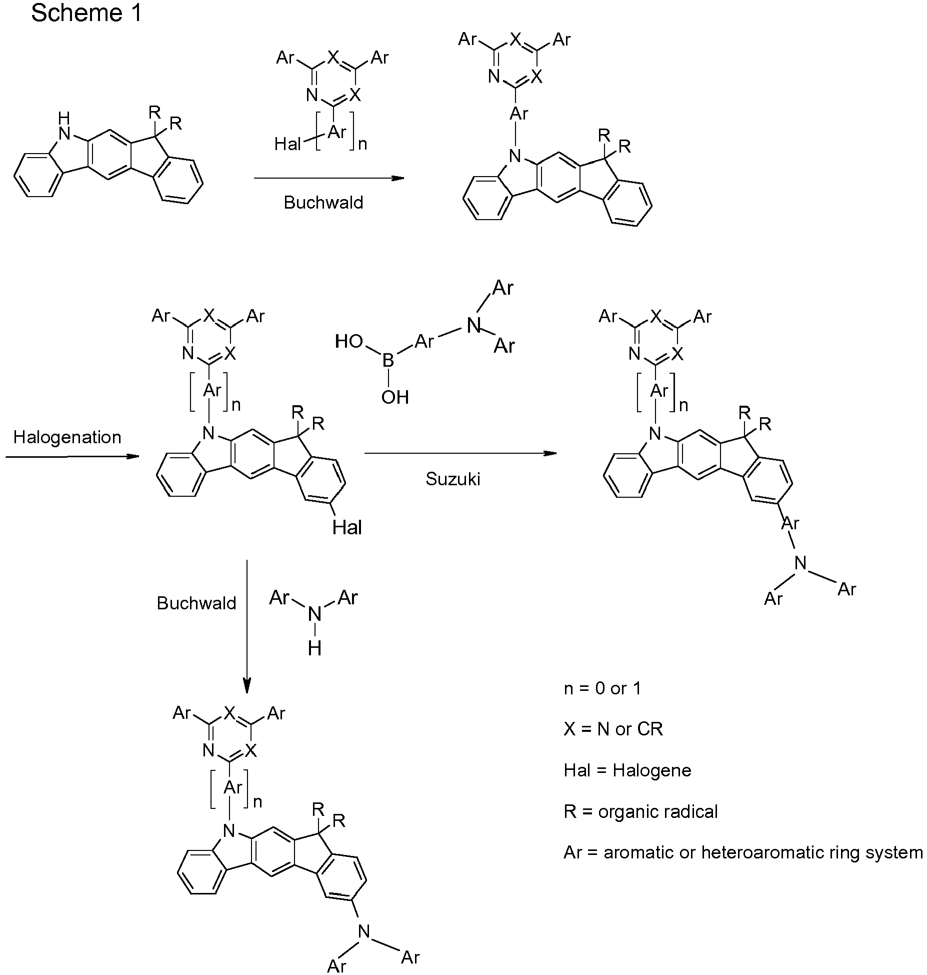

The invention thus furthermore relates to a process for the preparation of a compound of the formula (1), characterised in that an indenocarbazole compound is reacted with an aromatic or heteroaromatic ring system which contains an electron-deficient heteroaryl group, where the aromatic or heteroaromatic ring system is coupled to the nitrogen atom of the indenocarbazole. The reaction is preferably a Buchwald coupling between an indenocarbazole and a halogenated aromatic or heteroaromatic ring system.

Furthermore, the reaction product is preferably subsequently provided with a reactive functional group, for example by halogenation or by conversion into a boronic acid. Furthermore, a Suzuki coupling to a triarylamino derivative or a Buchwald coupling to diarylamino group is preferably subsequently carried out.

The compounds according to the invention described above, in particular compounds which are substituted by reactive leaving groups, such as bromine, iodine, chlorine, boronic acid or boronic acid ester, can be used as monomers for the preparation of corresponding oligomers, dendrimers or polymers. Suitable reactive leaving groups are, for example, bromine, iodine, chlorine, boronic acids, boronic acid esters, amines, alkenyl or alkynyl groups containing a terminal C-C double or triple bond respectively, oxiranes, oxetanes, groups which undergo a cycloaddition, for example a 1,3-dipolar cycloaddition, such, as, for example, dienes or azides, carboxylic acid derivatives, alcohols and silanes.

The invention therefore furthermore relates to oligomers, polymers or dendrimers comprising one or more compounds of the formula (1), where the bond(s) to the polymer, oligomer or dendrimer may be localised at any desired positions in formula (1) which are substituted by R¹ or R². Depending on the linking of the compound of the formula (1), the compound is part of a side chain of the oligomer or polymer or part of the main chain. An oligomer in the sense of this invention is taken to mean a compound which is built up from at least three monomer units. A polymer in the sense of the invention is taken to mean a compound which is built up from at least ten monomer units. The polymers, oligomers or dendrimers according to the invention may be conjugated, partially conjugated or non-conjugated. The oligomers or polymers according to the invention may be linear, branched or dendritic. In the structures linked in a linear manner, the units of the formula (1) may be linked directly to one another or linked to one another via a divalent group, for example via a substituted or unsubstituted alkylene group, via a heteroatom or via a divalent aromatic or heteroaromatic group. In branched and dendritic structures, three or more units of the formula (1) may, for example, be linked via a trivalent or polyvalent group, for example via a trivalent or polyvalent aromatic or heteroaromatic group, to give a branched or dendritic oligomer or polymer.

The same preferences as described above for compounds of the formula (1) apply to the recurring units of the formula (1) in oligomers, dendrimers and polymers.

For the preparation of the oligomers or polymers, the monomers according to the invention are homopolymerised or copolymerised with further monomers. Suitable and preferred comonomers are selected from fluorenes (for example in accordance with EP 842208 or WO 00/22026), spirobifluorenes (for example in accordance with EP 707020, EP 894107 or WO 06/ 061181), para-phenylenes (for example in accordance with WO 1992/ 18552), carbazoles (for example in accordance with WO 04/070772 or WO 2004/113468), thiophenes (for example in accordance with
EP 1028136), dihydrophenanthrenes (for example in accordance with WO 2005/014689 or WO 2007/006383), cis- and trans-indenofluorenes (for example in accordance with WO 2004/041901 or WO 2004/113412), ketones (for example in accordance with WO 2005/040302), phen-anthrenes (for example in accordance with WO 2005/104264 or WO 2007/ 017066) or also a plurality of these units. The polymers, oligomers and dendrimers usually also contain further units, for example emitting (fluorescent or phosphorescent) units, such as, for example, vinyltriarylamines (for example in accordance with WO 2007/068325) or phosphorescent metal complexes (for example in accordance with WO 2006/003000), and/or charge-transport units, in particular those based on triarylamines.

The polymers and oligomers according to the invention are generally prepared by polymerisation of one or more types of monomer, at least one monomer of which results in recurring units of the formula (1 -1) in the polymer. Suitable polymerisation reactions are known to the person skilled in the art and are described in the literature. Particularly suitable and preferred polymerisation reactions which result in C-C or C-N links are the following:
(A) SUZUKI polymerisation;
(B) YAMAMOTO polymerisation;
(C) STILLE polymerisation; and
(D) HARTWIG-BUCHWALD polymerisation.

The way in which the polymerisation can be carried out by these methods and the way in which the polymers can then be separated off from the reaction medium and purified is known to the person skilled in the art and is described in detail in the literature, for example in WO 2003/048225, WO 2004/037887 and WO 2004/037887.

The present invention thus also relates to a process for the preparation of the polymers, oligomers and dendrimers according to the invention, which is characterised in that they are prepared by SUZUKI polymerisation, YAMAMOTO polymerisation, STILLE polymerisation or HARTWIG-BUCHWALD polymerisation. The dendrimers according to the invention can be prepared by processes known to the person skilled in the art or analogously thereto. Suitable processes are described in the literature, such as, for example, in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/ 067343 A1 and WO 2005/026144 A1.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetol, 1,4-diisopropylbenzene, dibenzyl ether, diethylene glycol butyl methyl ether, triethylene glycol butyl methyl ether, diethylene glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The invention therefore furthermore relates to a formulation, in particular a solution, dispersion or emulsion, comprising at least one compound of the formula (1) or at least one polymer, oligomer or dendrimer containing at least one unit of the formula (1), and at least one solvent, preferably an organic solvent. The way in which solutions of this type can be prepared is known to the person skilled in the art and is described, for example, in WO 2002/072714, WO 2003/019694 and the literature cited therein.

The compound of the formula (1) is suitable for use in an electronic device, in particular an organic electroluminescent device (OLED). Depending on the substitution, the compound of the formula (1) can be employed in different functions and layers. Preference is given to the use as matrix material in an emitting layer, particularly preferably in combination with a phosphorescent emitter, and/or the use as electron-transporting material, and/or the use as hole-blocking material.

The invention therefore furthermore relates to the use of a compound of the formula (1) in an electronic device. The electronic device here is preferably selected from the group consisting of organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and particularly preferably organic electroluminescent devices (OLEDs).

The invention furthermore relates to an electronic device comprising at least one compound of the formula (1). The electronic device here is preferably selected from the devices mentioned above.

Particular preference is given to an organic electroluminescent device comprising anode, cathode and at least one emitting layer, characterised in that the device comprises at least one organic layer which comprises at least one compound of the formula (1). Preference is given to an organic electroluminescent device comprising anode, cathode and at least one emitting layer, characterised in that at least one layer in the device, selected from emitting layers, electron-transport layers, electron-injection layers and hole-blocking layers, comprises at least one compound of the formula (1).

An electron-transport layer is taken to mean any desired organic layer, arranged between cathode and an emitting layer, which has electron-transporting properties.

An electron-injection layer is taken to mean any desired organic layer, arranged between cathode and an emitting layer, which has electron-injecting properties and is directly adjacent to the cathode.

A hole-blocking layer is taken to mean any desired organic layer which is located between emitting layer and cathode and has hole-blocking properties. A hole-blocking layer in accordance with the present application is preferably located between an emitting layer and an electron-transporting layer, particularly preferably directly adjacent to an emitting layer on the cathode side. Materials of the hole-blocking layer are typically distinguished by a low HOMO.

Apart from cathode, anode and emitting layer, the electronic device may also comprise further layers. These are selected, for example, from in each case one or more hole-injection layers, hole-transport layers, hole-blocking layers, electron-transport layers, electron-injection layers, electron-blocking layers, exciton-blocking layers, interlayers, charge-generation layers (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer*)* and/or organic or inorganic p/n junctions. However, it should be pointed out that each of these layers does not necessarily have to be present and the choice of layers is always dependent on the compounds used and in particular also on whether the electroluminescent device is fluorescent or phosphorescent.

The sequence of the layers of the electronic device is preferably as follows:
-anode-
-hole-injection layer-
-hole-transport layer-
-optionally further hole-transport layers-
-emitting layer-
-electron-transport layer-
-electron-injection layer-
-cathode-.
It should again be pointed out here that not all the said layers have to be present, and/or that further layers may additionally be present.

The electronic device according to the invention may comprise a plurality of emitting layers. In this case, these emitting layers particularly preferably have in total a plurality of emission maxima between 380 nm and 750 nm, resulting overall in white emission, i.e. various emitting compounds which are able to fluoresce or phosphoresce and which emit blue or yellow or orange or red light are used in the emitting layers. Particular preference is given to three-layer systems, i.e. systems having three emitting layers, where at least one of these layers preferably comprises at least one compound of the formula (1-1) and where the three layers exhibit blue, green and orange or red emission (for the basic structure see, for example, WO 2005/011013). The compounds according to the invention may alternatively and/or additionally also be present in the electron-transport layer or in another layer. It should be noted that, for the generation of white light, an emitter compound used individually which emits in a broad wavelength range may also be suitable instead of a plurality of emitter compounds emitting in colour.

The compound of the formula (1) is preferably present in the electronic device as matrix material in an emitting layer, particularly preferably in combination with one or more phosphorescent emitter compounds. The emitter compounds are preferably in the form of dopants.

The terms dopant, matrix material and phosphorescent emitter compound here are defined as described above.

Emitter compounds preferably used as matrix material in combination with the compound of the formula (1) are the phosphorescent emitter compounds indicated below.

The emitting layer of the electronic device may also comprise systems comprising a plurality of matrix materials (mixed-matrix systems) and/or a plurality of dopants. In this case too, the dopants are generally the materials whose proportion in the system is the smaller and the matrix materials are the materials whose proportion in the system is the greater. In individual cases, however, the proportion of an individual matrix material in the system may be smaller than the proportion of an individual dopant.

In a further preferred embodiment of the invention, the compound of the formula (1) is used as a component of mixed-matrix systems. The mixed-matrix systems preferably comprise two or three different matrix materials, particularly preferably two different matrix materials. One of the two materials here is preferably a material having hole-transporting properties and the other material is a material having electron-transporting properties. However, the desired electron-transporting and hole-transporting properties of the mixed-matrix components may also be combined principally or completely in a single mixed-matrix components, where the further mixed-matrix component(s) fulfil other functions. The two different matrix materials here may be present in a ratio of 1:50 to 1:1, preferably 1:20 to 1:1, particularly preferably 1:10 to 1:1 and very particularly preferably 1:4 to 1:1. Mixed-matrix systems are preferably employed in phosphorescent organic electroluminescent devices. More precise information on mixed-matrix systems is given, inter alia, in the application WO 2010/108579.

The mixed-matrix systems may comprise one or more dopants, preferably one or more phosphorescent dopants. In general, mixed-matrix systems are preferably employed in phosphorescent organic electroluminescent devices.

Particularly suitable matrix materials which can be used in combination with the compounds according to the invention as matrix components of a mixed-matrix system are selected from the preferred matrix materials for phosphorescent dopants indicated below or the preferred matrix materials for fluorescent dopants, depending on what type of dopant is employed in the mixed-matrix system.

Preferred phosphorescent dopants for use in mixed-matrix systems are the phosphorescent dopants shown in the table above.

The proportion of the matrix material in the emitting layer in the electronic device according to the invention is preferably between 50.0 and 99.9% by vol., particularly preferably between 80.0 and 99.5% by vol. and very particularly preferably between 92.0 and 99.5% by vol. for fluorescent emitting layers and between 85.0 and 97.0% by vol. for phosphorescent emitting layers. Correspondingly, the proportion of the dopant is preferably between 0.1 and 50.0% by vol., particularly preferably between 0.5 and 20.0% by vol. and very particularly preferably between 0.5 and 8.0% by vol. for fluorescent emitting layers and between 3.0 and 15.0% by vol. for phosphorescent emitting layers.

In a further preferred embodiment of the invention, the compound of the formula (1) is employed as electron-transport material in an electron-transport layer or electron-injection layer or hole-blocking layer.

Materials which are preferably present in the above-mentioned functional layers of the electronic device according to the invention are indicated below.

Suitable phosphorescent emitters are, in particular, compounds which emit light, preferably in the visible region, on suitable excitation and in addition contain at least one atom having an atomic number greater than 20, preferably greater than 38 and less than 84, particularly preferably greater than 56 and less than 80. The phosphorescent emitters used are preferably compounds which contain copper, molybdenum, tungsten, rhenium, ruthenium, osmium, rhodium, iridium, palladium, platinum, silver, gold or europium, in particular compounds which contain iridium, platinum or copper.

All luminescent iridium, platinum or copper complexes are regarded as phosphorescent compounds in the sense of the present invention.

Examples of phosphorescent emitters are revealed by the applications WO 2000/70655, WO 2001/41512, WO 2002/02714, WO 2002/15645, EP 1191613, EP 1191612, EP 1191614, WO 2005/033244, WO 2005/ 019373 and US 2005/0258742. In general, all phosphorescent complexes as are used in accordance with the prior art for phosphorescent OLEDs and as are known to the person skilled in the art in the area of organic electroluminescent devices are suitable for use in the devices according to the invention.

The compounds shown in the following table are particularly suitable phosphorescent dopants:

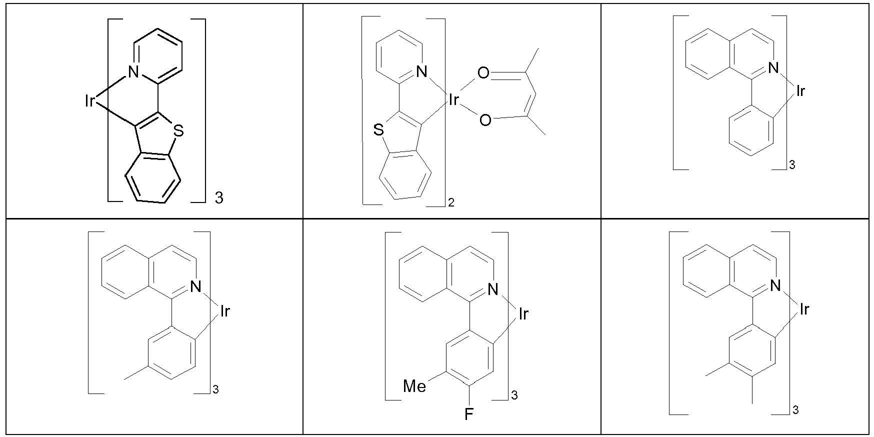

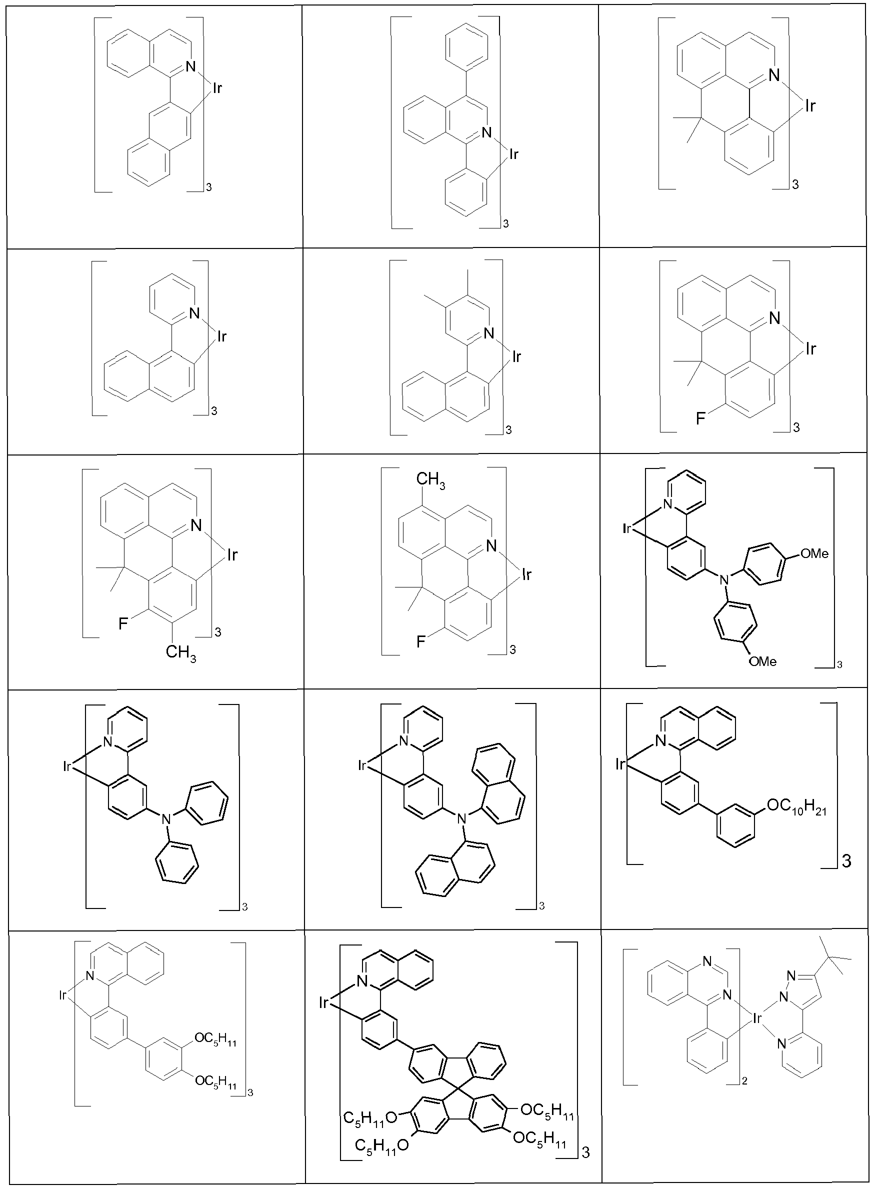

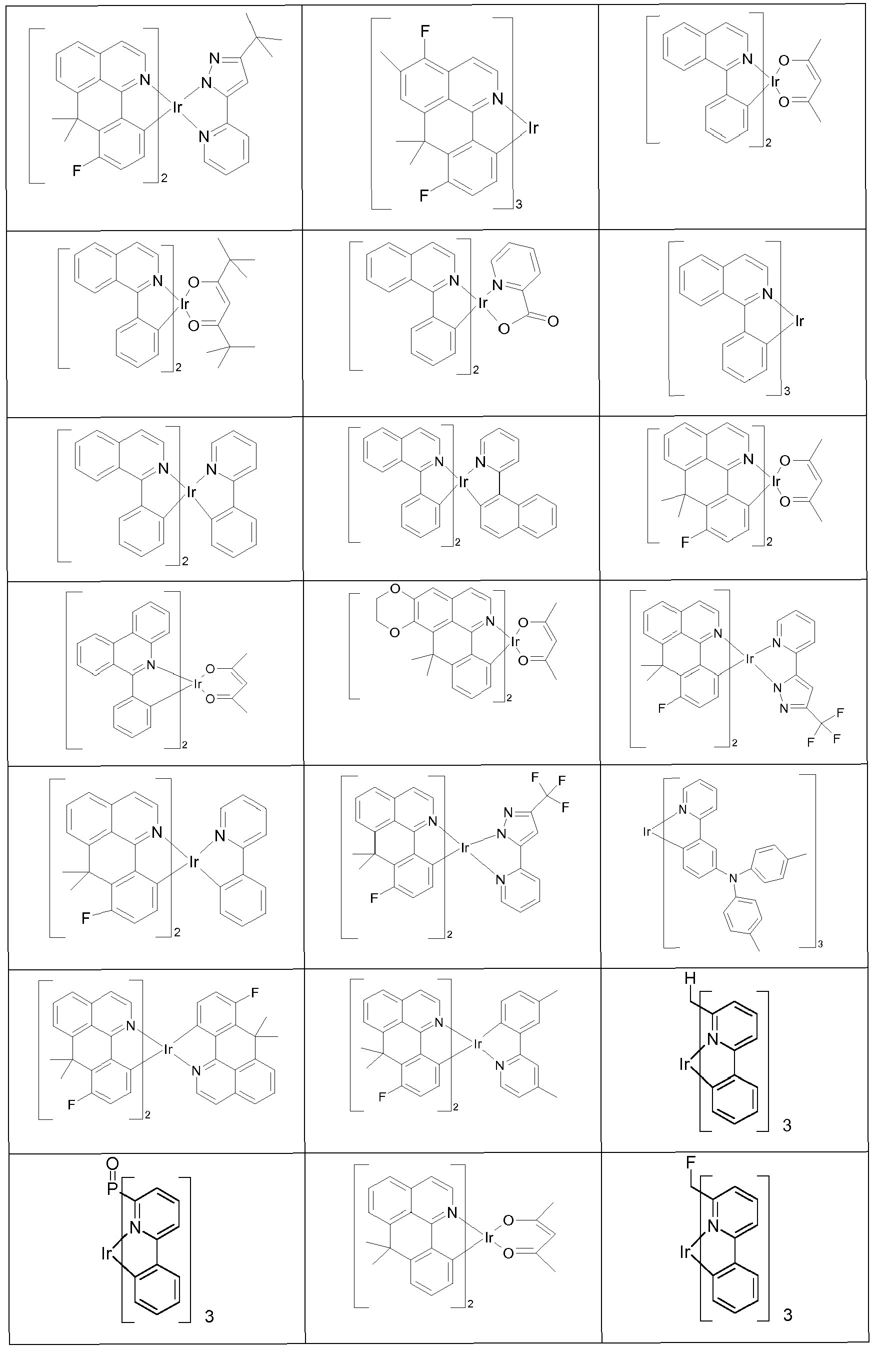

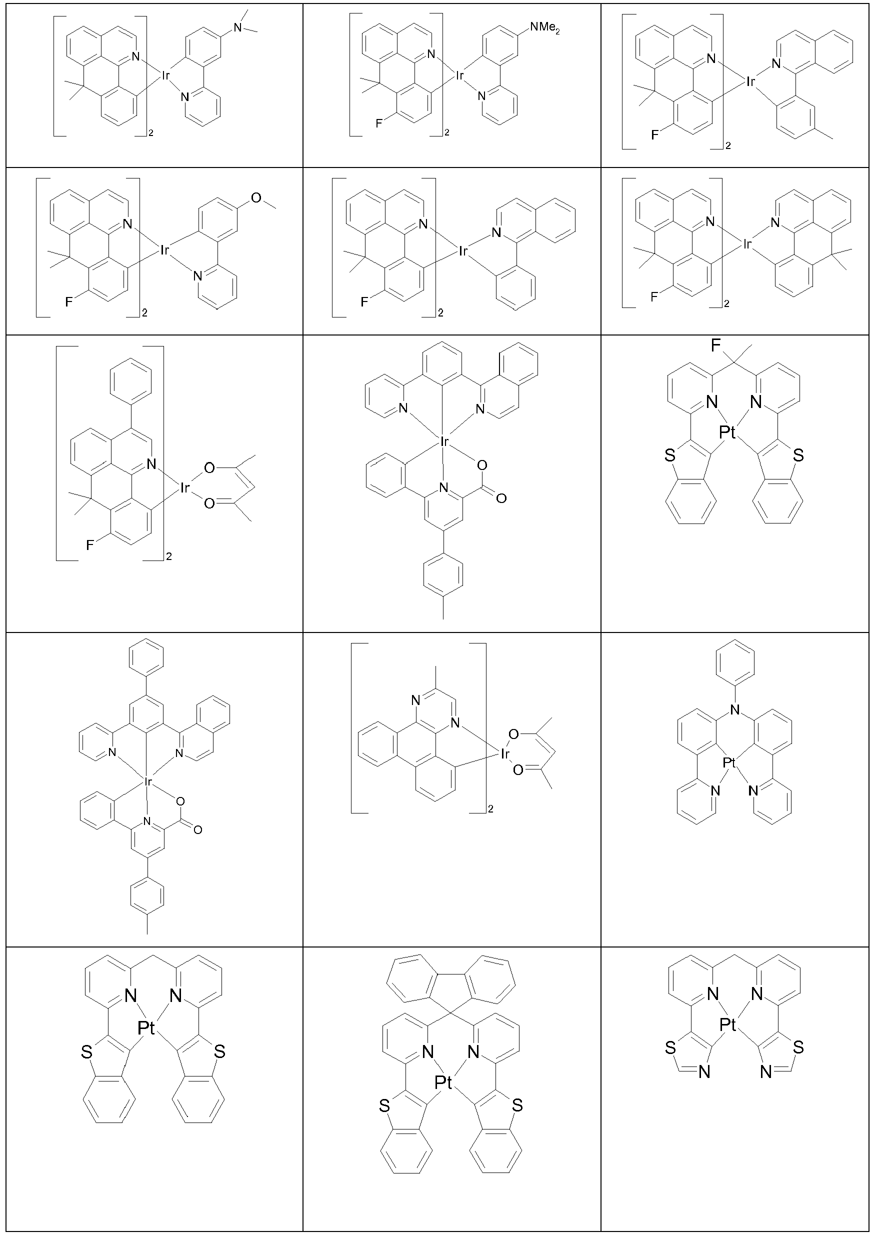

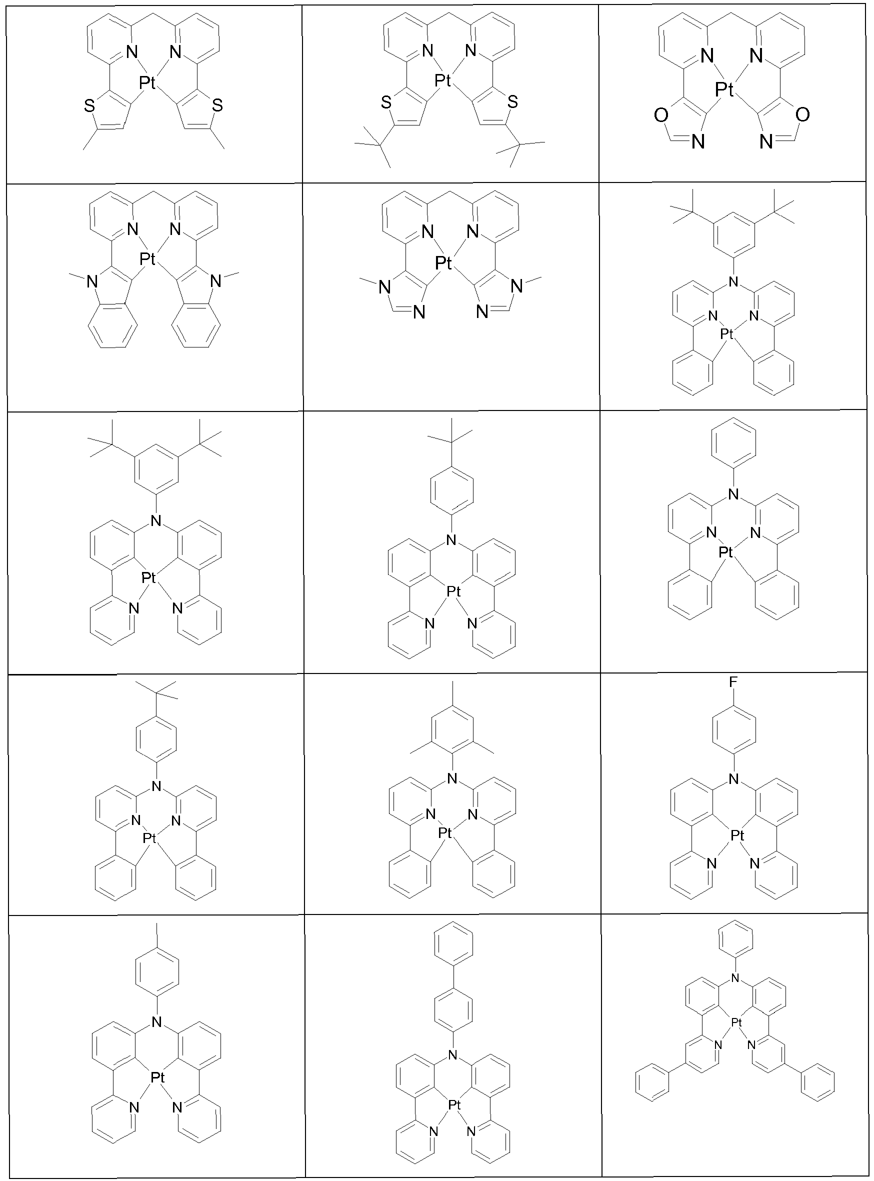

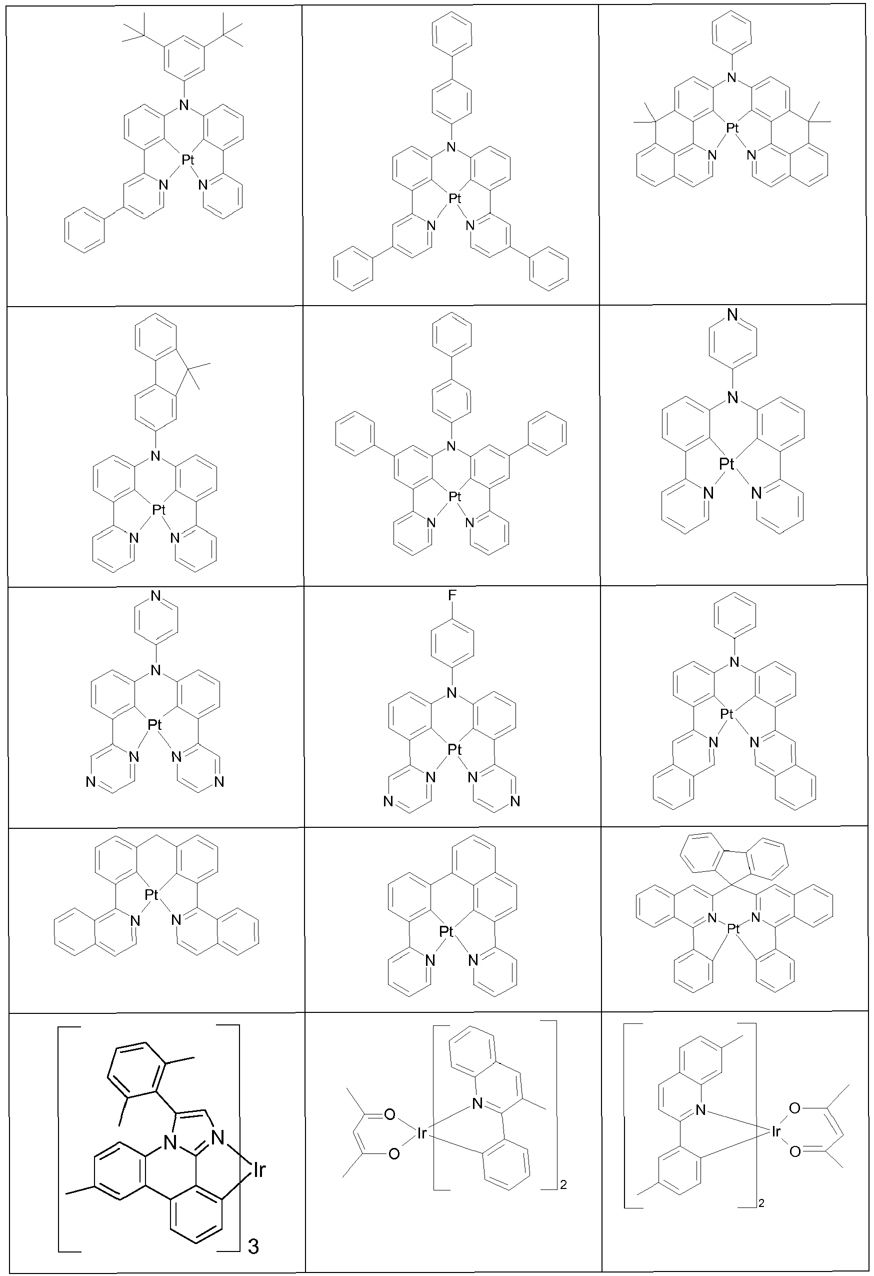

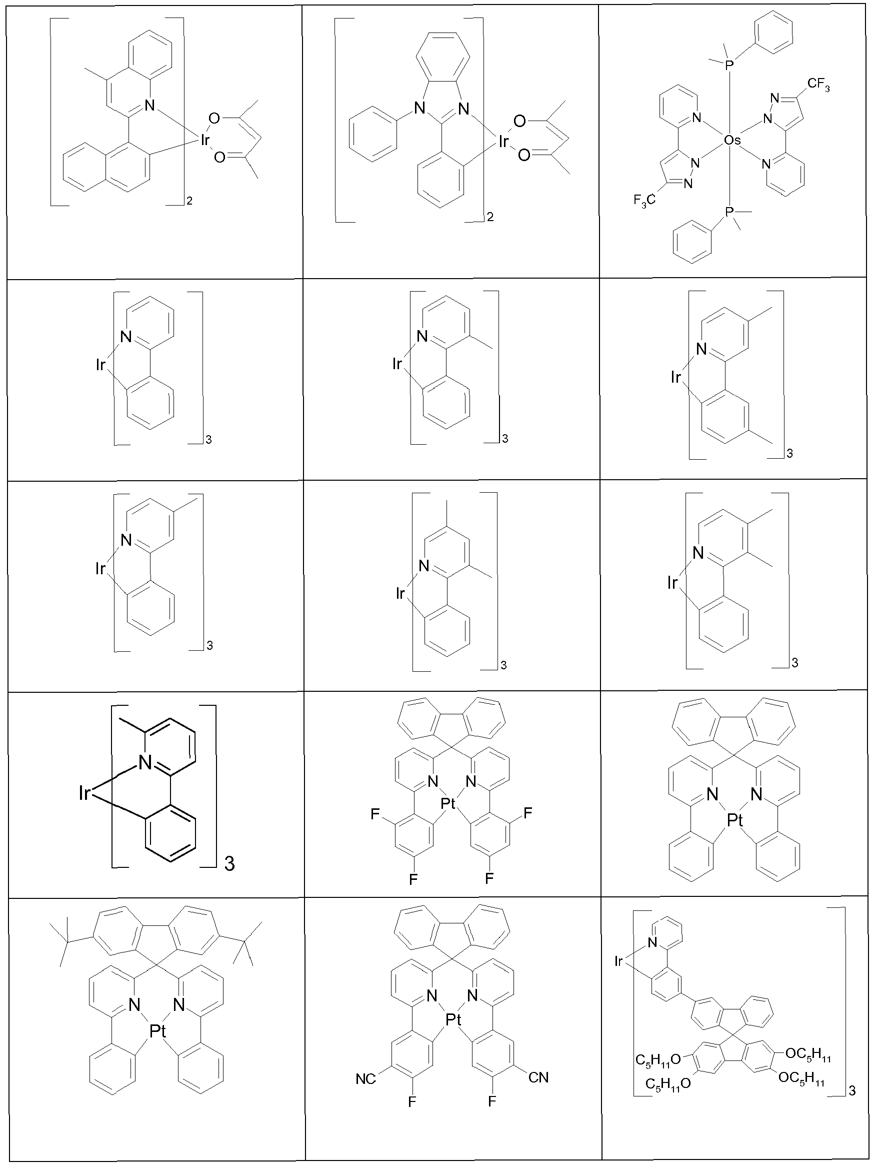

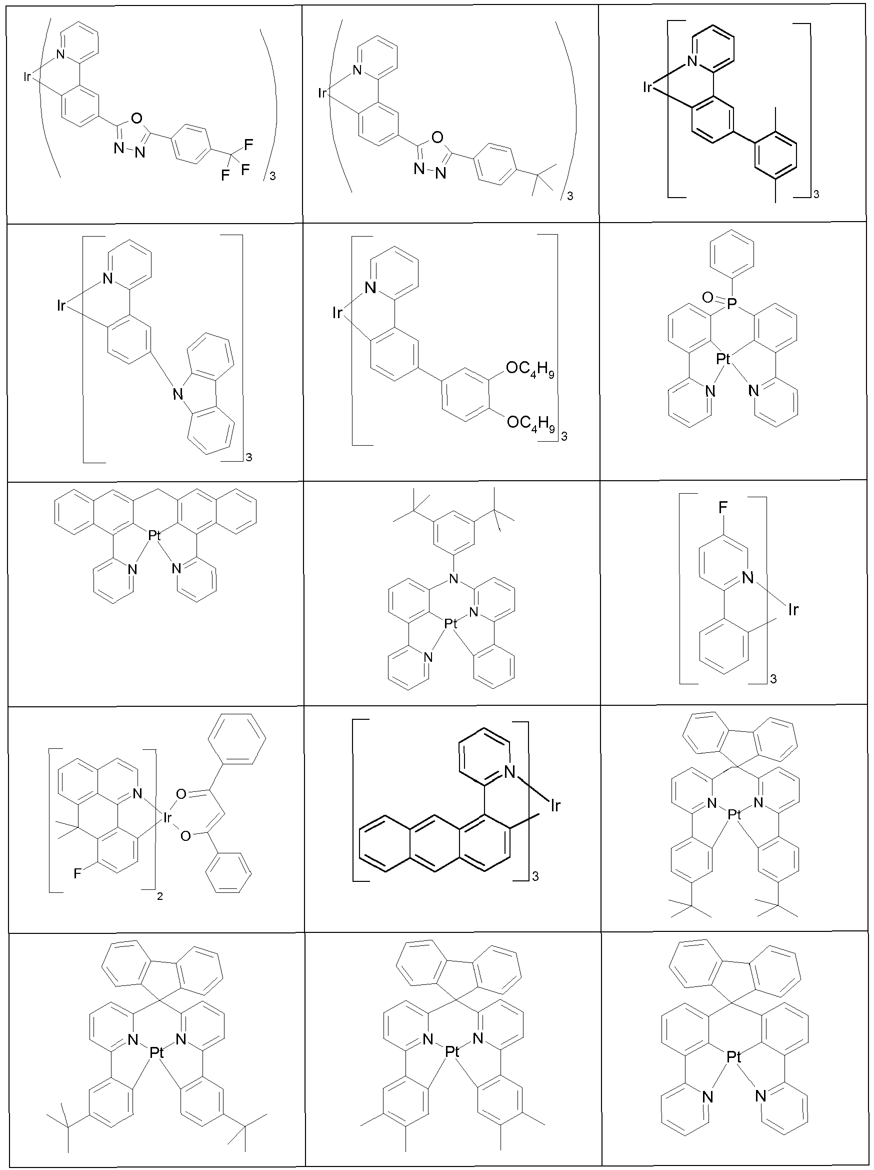

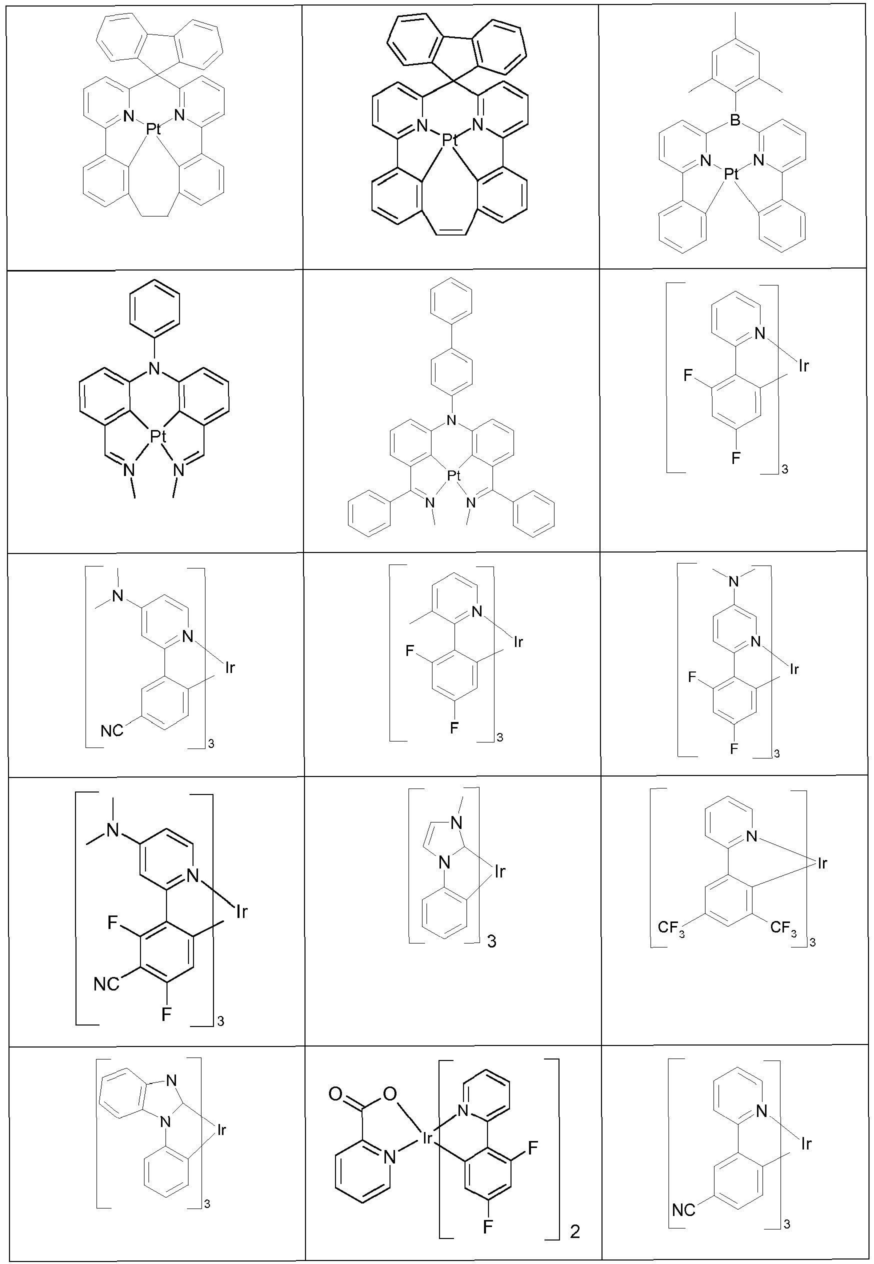

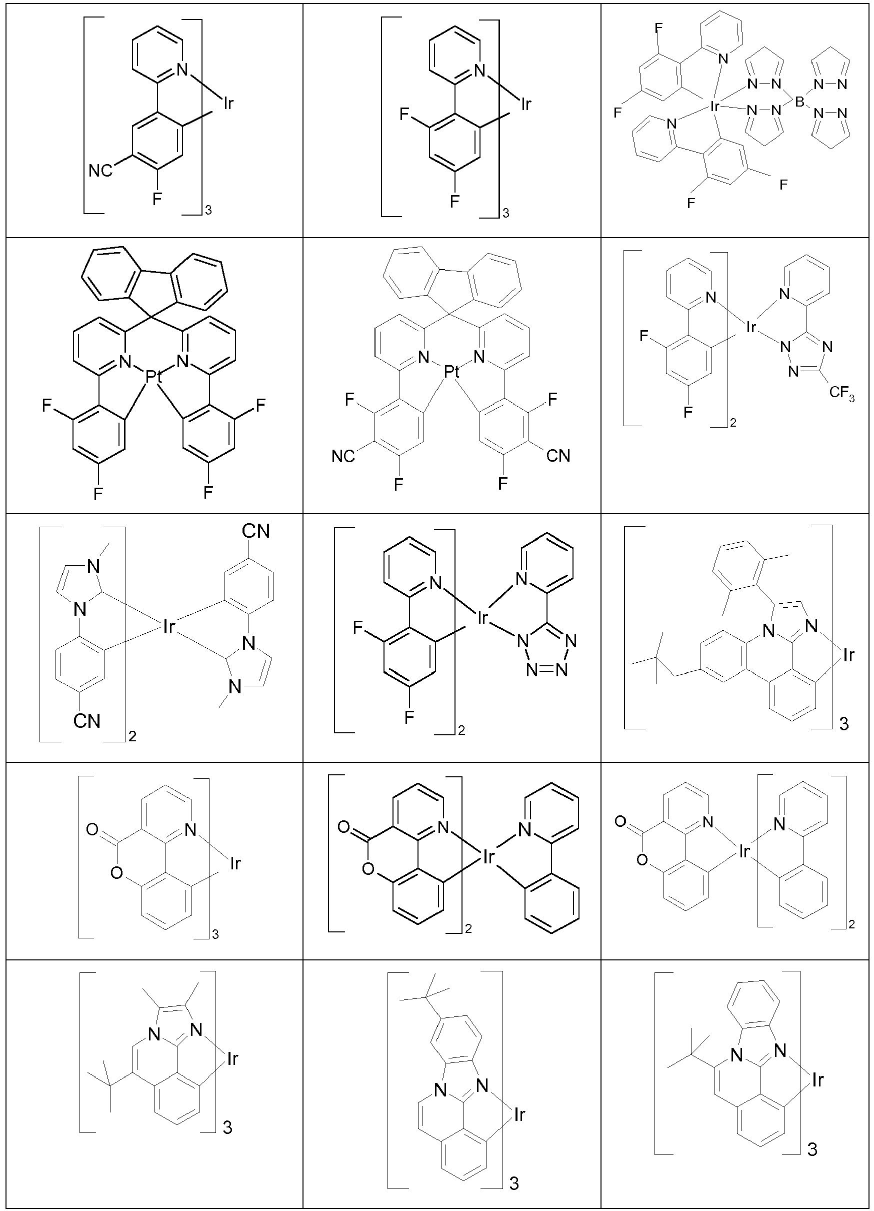

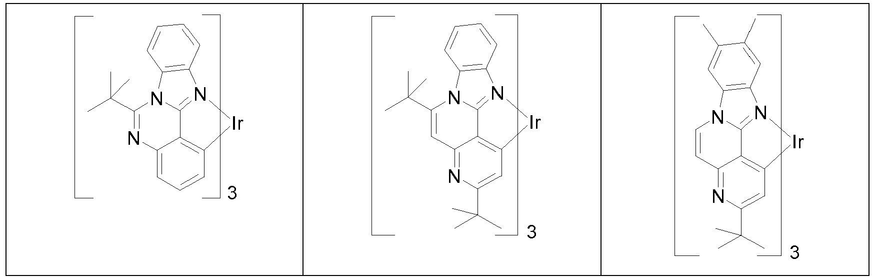

Preferred fluorescent emitters are selected from the class of the arylamines. An arylamine or aromatic amine in the sense of this invention is taken to mean a compound which contains three substituted or unsubstituted aromatic or heteroaromatic ring systems bonded directly to the nitrogen. At least one of these aromatic or heteroaromatic ring systems is preferably a condensed ring system, particularly preferably having at least 14 aromatic ring atoms. Preferred examples thereof are aromatic anthracen-amines, aromatic anthracenediamines, aromatic pyrenamines, aromatic pyrenediamines, aromatic chrysenamines or aromatic chrysenediamines. An aromatic anthracenamine is taken to mean a compound in which one diarylamino group is bonded directly to an anthracene group, preferably in the 9-position. An aromatic anthracenediamine is taken to mean a compound in which two diarylamino groups are bonded directly to an anthracene group, preferably in the 9,10-position. Aromatic pyrenamines, pyrenediamines, chrysenamines and chrysenediamines are defined analogously thereto, where the diarylamino groups are preferably bonded to the pyrene in the 1-position or in the 1,6-position. Further preferred emitter compounds are indenofluorenamines or indenofluorenediamines, for example in accordance with WO 2006/108497 or WO 2006/122630, benzoindenofluoren-amines or benzoindenofluorenediamines, for example in accordance with WO 2008/006449, and dibenzoindenofluorenamines or dibenzoindeno-fluorenediamines, for example in accordance with WO 2007/140847, and the indenofluorene derivatives containing condensed aryl groups which are disclosed in WO 2010/012328. Preference is likewise given to the pyren-arylamines disclosed in WO 2012/048780 and the as yet unpublished EP 12004426.8. Preference is likewise given to the benzoindenofluoren-amines disclosed in the as yet unpublished EP 12006239.3.

Suitable matrix materials, preferably for fluorescent emitters, besides the compounds according to the invention, are materials from various classes of substance. Preferred matrix materials are selected from the classes of the oligoarylenes (for example 2,2',7,7'-tetraphenylspirobifluorene in accordance with EP 676461 or dinaphthylanthracene), in particular the oligoarylenes containing condensed aromatic groups, the oligoarylenevinylenes (for example DPVBi or spiro-DPVBi in accordance with EP 676461), the polypodal metal complexes (for example in accordance with WO 2004/ 081017), the hole-conducting compounds (for example in accordance with WO 2004/058911), the electron-conducting compounds, in particular ketones, phosphine oxides, sulfoxides, etc. (for example in accordance with WO 2005/084081 and WO 2005/084082), the atropisomers (for example in accordance with WO 2006/048268), the boronic acid derivatives (for example in accordance with WO 2006/117052) or the benz-anthracenes (for example in accordance with WO 2008/145239). Particularly preferred matrix materials are selected from the classes of the oligoarylenes, comprising naphthalene, anthracene, benzanthracene and/or pyrene or atropisomers of these compounds, the oligoarylenevinylenes, the ketones, the phosphine oxides and the sulfoxides. Very particularly preferred matrix materials are selected from the classes of the oligoarylenes, comprising anthracene, benzanthracene, benzophenanthrene and/or pyrene or atropisomers of these compounds. An oligoarylene in the sense of this invention is intended to be taken to mean a compound in which at least three aryl or arylene groups are bonded to one another. Preference is furthermore given to the anthracene derivatives disclosed in WO 2006/ 097208, WO 2006/131192, WO 2007/065550, WO 2007/110129, WO 2007/065678, WO 2008/145239, WO 2009/100925, WO 2011/054442 and EP 1553154, and the pyrene compounds disclosed in EP 1749809, EP 1905754 and US 2012/0187826.

Preferred matrix materials for phosphorescent emitters, besides the compounds according to the invention, are aromatic amines, in particular triarylamines, for example in accordance with US 2005/0069729, carbazole derivatives (for example CBP, N,N-biscarbazolylbiphenyl) or compounds in accordance with WO 2005/039246, US 2005/0069729, JP 2004/288381, EP 1205527 or WO 2008/086851, bridged carbazole derivatives, for example in accordance with WO 2011/088877 and WO 2011/128017, indenocarbazole derivatives, for example in accordance with WO 2010/ 136109 and WO 2011/000455, azacarbazole derivatives, for example in accordance with EP 1617710, EP 1617711, EP 1731584, JP 2005/347160, indolocarbazole derivatives, for example in accordance with WO 2007/ 063754 or WO 2008/056746, ketones, for example in accordance with WO 2004/093207 or WO 2010/006680, phosphine oxides, sulfoxides and sulfones, for example in accordance with WO 2005/003253, oligophenyl-enes, bipolar matrix materials, for example in accordance with WO 2007/ 137725, silanes, for example in accordance with WO 2005/ 111172, aza-boroles or boronic esters, for example in accordance with WO 2006/ 117052, triazine derivatives, for example in accordance with WO 2010/ 015306, WO 2007/063754 or WO 2008/056746, zinc complexes, for example in accordance with EP 652273 or WO 2009/062578, aluminium complexes, for example BAlq, diazasilole and tetraazasilole derivatives, for example in accordance with WO 2010/054729, and diazaphosphole derivatives, for example in accordance with WO 2010/054730.

Suitable charge-transport materials, as can be used in the hole-injection or hole-transport layer or in the electron-transport layer of the organic electroluminescent device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer, besides the compounds according to the invention, are all materials as are used in accordance with the prior art as electron-transport materials in the electron-transport layer. Particularly suitable are aluminium complexes, for example Alq₃, zirconium complexes, for example Zrq₄, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore suitable materials are derivatives of the above-mentioned compounds, as disclosed in JP 2000/ 053957, WO 2003/060956, WO 2004/028217, WO 2004/080975 and WO 2010/072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluoren-amines (for example in accordance with WO 08/006449), dibenzoindenofluorenamines (for example in accordance with WO 07/140847), spiro-bifluorenamines (for example in accordance with WO 2012/034627 or WO 2013/120577), fluorenamines (for example in accordance with the as yet unpublished applications EP 12005369.9, EP 12005370.7 and EP 12005371.5), spirodibenzopyranamines (for example in accordance with WO 2013/083216) and dihydroacridine derivatives (for example in accordance with WO 2012/150001).

The cathode of the organic electroluminescent device preferably comprises metals having a low work function, metal alloys or multilayered structures comprising various metals, such as, for example, alkaline-earth metals, alkali metals, main-group metals or lanthanoids (for example Ca, Ba, Mg, Al, In, Mg, Yb, Sm, etc.). Also suitable are alloys comprising an alkali metal or alkaline-earth metal and silver, for example an alloy comprising magnesium and silver. In the case of multilayered structures, further metals which have a relatively high work function, such as, for example, Ag or Al, can also be used in addition to the said metals, in which case combinations of the metals, such as, for example, Ca/Ag, Mg/Ag or Ag/Ag, are generally used. It may also be preferred to introduce a thin interlayer of a material having a high dielectric constant between a metallic cathode and the organic semiconductor. Suitable for this purpose are, for example, alkali metal fluorides or alkaline-earth metal fluorides, but also the corresponding oxides or carbonates (for example LiF, Li₂O, BaF₂, MgO, NaF, CsF, Cs₂CO₃, etc.). Furthermore, lithium quinolinate (LiQ) can be used for this purpose. The layer thickness of this layer is preferably between 0.5 and 5 nm.

The anode preferably comprises materials having a high work function. The anode preferably has a work function of greater than 4.5 eV vs. vacuum. Suitable for this purpose are on the one hand metals having a high redox potential, such as, for example, Ag, Pt or Au. On the other hand, metal/metal oxide electrodes (for example Al/Ni/NiOₓ, Al/PtOₓ) may also be preferred. For some applications, at least one of the electrodes must be transparent or partially transparent in order to facilitate either irradiation of the organic material (organic solar cells) or the coupling-out of light (OLEDs, O-lasers). Preferred anode materials here are conductive mixed metal oxides. Particular preference is given to indium tin oxide (ITO) or indium zinc oxide (IZO). Preference is furthermore given to conductive, doped organic materials, in particular conductive, doped polymers. Furthermore, the anode may also consist of a plurality of layers, for example of an inner layer of ITO and an outer layer of a metal oxide, preferably tungsten oxide, molybdenum oxide or vanadium oxide.

The device is appropriately (depending on the application) structured, provided with contacts and finally sealed, since the lifetime of the devices according to the invention is shortened in the presence of water and/or air.

In a preferred embodiment, the electronic device according to the invention is characterised in that one or more layers are coated by means of a sublimation process, in which the materials are applied by vapour deposition in vacuum sublimation units at an initial pressure of less than 10⁻⁵ mbar, preferably less than 10⁻⁶ mbar. However, it is also possible here for the initial pressure to be even lower, for example less than 10⁻⁷ mbar.

Preference is likewise given to an electronic device, characterised in that one or more layers are coated by means of the OVPD (organic vapour phase deposition) process or with the aid of carrier-gas sublimation, in which the materials are applied at a pressure of between 10⁻⁵ mbar and 1 bar. A special case of this process is the OVJP (organic vapour jet printing) process, in which the materials are applied directly through a nozzle and are thus structured (for example M. S. Arnold et al., Appl. Phys. Lett. 2008, 92, 053301).

Preference is furthermore given to an electronic device, characterised in that one or more layers are produced from solution, such as, for example, by spin coating, or by means of any desired printing process, such as, for example, screen printing, flexographic printing, nozzle printing or offset printing, but particularly preferably LITI (light induced thermal imaging, thermal transfer printing) or ink-jet printing. Soluble compounds of the formula (1-1) are necessary for this purpose. High solubility can be achieved through suitable substitution of the compounds.

For the production of an electronic device according to the invention, it is furthermore preferred to apply one or more layers from solution and one or more layers by a sublimation process.

In accordance with the invention, the electronic devices comprising one or more compounds of the formula (1-1) can be employed in displays, as light sources in lighting applications and as light sources in medical and/or cosmetic applications (for example light therapy).

### Working examples

The following working examples serve to explain the invention. They are not intended to be interpreted as restrictive.

The following syntheses are, unless indicated otherwise, carried out under a protective-gas atmosphere in dried solvents. Compounds (I), (XIII) and (XII) can be prepared in accordance with WO2010136109. Compounds (II), (XV), (XVI), (XX), and (XXIV) are commercially available. Compounds (IV), (VI) and (XIII) can be prepared in accordance with WO201155912. Comparative compounds (A), (B), and (C) can be prepared in accordance with WO2010136109. (D) can be prepared in accordance with WO2012014500A1.

### Example 1:

### Preparation of compound (V)

### Synthetic procedure for the preparation of compound (V):

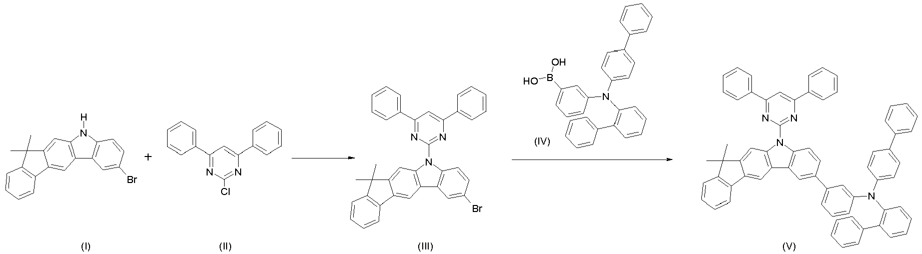

### Preparation of compound (III)

To the suspension of 2.4 g (60.7 mmol, 60% in oil) of NaH in 100 mL of DMF, 20.0 g (55.2 mmol) of compound(I) in 180 mL of DMF is slowly added to the suspension and stirred for 1 h at room temperature. 17.7 g (66.4 mmol) of compound(II) is dissolved in 80 mL of DMF and slowly added to the suspension, and the reaction mixture is stirred for 16 h. The reaction mixture is poured on to ice, the organic phase is separated off, extracted three times with 200 mL of dichloromethane, dried over magnesium sulfate and subsequently evaporated to dryness. The residue is washed with ethanol, filtered off and finally dried under reduced pressure. The yield is 28.2 g (47.6 mmol), corresponding to 78% of theory.

### Preparation of compound (V)

30.0 g (50.6 mmol) of compound (III), 24.6 g (55.8 mmol) of compound (IV) and 5.8 g (5.5 mmol) of sodium carbonate are suspended in 600 mL of toluene and 220 mL of water. 590 mg (0.51 mmol) of tetrakis(triphenylphosphine)palladium(0) are added to this suspension, and the reaction mixture is stirred under reflux for 16 h. After cooling, the organic phase is separated off, extracted three times with 300 mL of dichloromethane, dried over magnesium sulfate and subsequently evaporated to dryness. The residue was washed with heptane, filtered off and finally dried under reduced pressure. The yield is 37.2 g (40.9 mmol), corresponding to 84% of theory.

### Example 2

### Preparation of compound (VII)

### Synthetic procedure for the preparation of compound (VII):

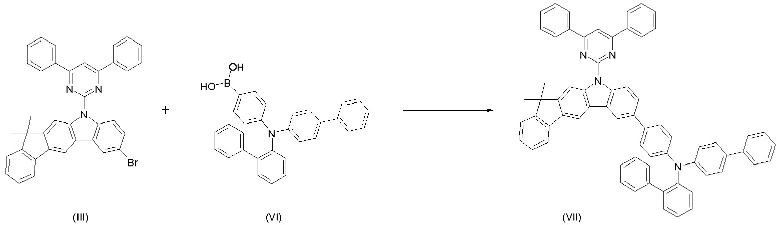

### Preparation of compound (VII)

28.5 g (48.1 mmol) of compound (III), 23.3 g (52.9 mmol) of compound (VI) and 5.5 g (52.1 mmol) of sodium carbonate are suspended in 600 mL of toluene and 220 mL of water. 0.56 g (0.5 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 21.7 g (23.9 mmol), corresponding to 50% of theory.

### Example 3

### Preparation of compound (IX)

### Synthetic procedure for the preparation of compound (IX):

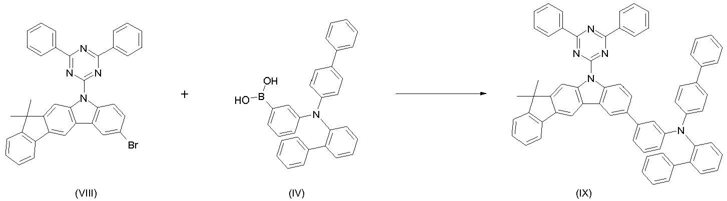

25 g (42.1 mmol) of compound (VIII), 20.5 g (52.9 mmol) of compound (IV) and 4.8 g (45.6 mmol) of sodium carbonate are suspended in 600 mL of toluene and 220 mL of water. 0.42 g (0.4 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 26 g (28.6 mmol), corresponding to 68% of theory.

### Example 4

### Preparation of compound (X)

### Synthetic procedure for the preparation of compound (X):

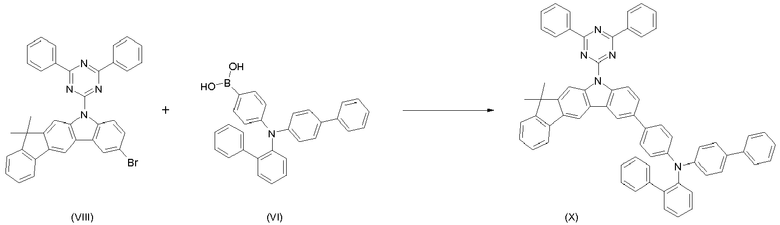

### Preparation of compound (X)

30 g (50.5 mmol) of compound (VIII), 24.5 g (55.6 mmol) of compound (VI) and 5.8 g (54.7 mmol) of sodium carbonate are suspended in 600 mL of toluene, and 220 mL of water. 0.58 g (0.5 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 27 g (29.7 mmol), corresponding to 64% of theory.

### Example 5

### Preparation of compound (XIV)

### Synthetic procedure for the preparation of compound (XIV):

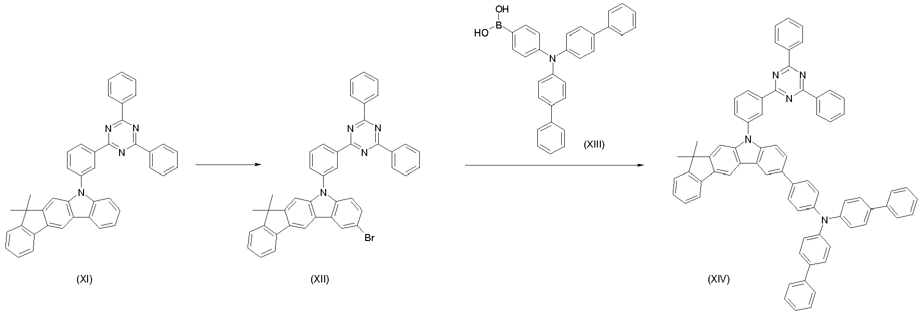

### Preparation of compound (XII)

30.0 g (50.8 mmol) of compound (XI) is suspended in 750 mL of THF. 12.6 g (70.8 mmol) of NBS is slowly added to this suspension, and the reaction mixture is stirred at room temperature for overnight. The reaction mixture is quenched by water. The organic phase is separated off, extracted three times with 300 mL of dichloromethane, dried over magensium sulfate and subsequently evaporated to dryness. The residue is recrystallized from toluene. The yield is 21.6 g (32.2 mmol), corresponding to 63% of theory.

### Preparation of compound (XIV)

20 g (29.9 mmol) of compound (III), 14.5 g (32.9 mmol) of compound (VIII) and 3.4 g (32.4 mmol) of sodium carbonate are suspended in 600 mL of toluene, and 220 mL of water. 0.35 g (0.3 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 18 g (18.3 mmol), corresponding to 61% of theory.

### Example 6

### Preparation of compound (XIX)

### Synthetic procedure for the preparation of compound (XIX):

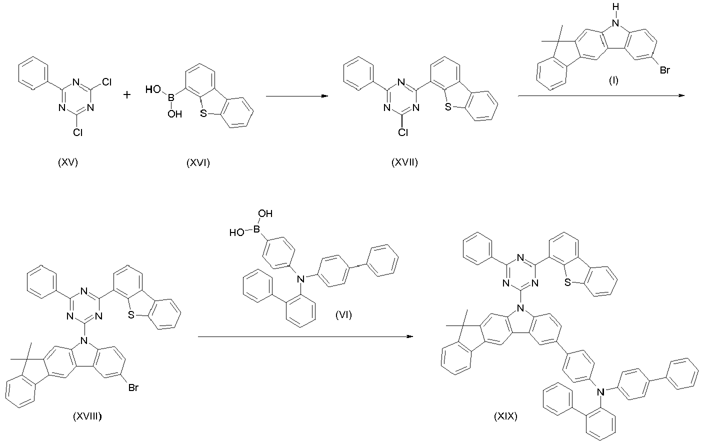

### Preparation of compound (XVII)

34.7 g (153.5 mmol) of compound (XV), 35.0 g (153.5 mmol) of compound (XVI) and 17.9 g (168.8 mmol) of sodium carbonate are suspended in 120 mL of toluene, 300 mL of dioxane and 300 mL of water. 1.8 g (1.5 mmol) of tetrakis(triphenylphosphine)palladium(0) are added to this suspension, and the reaction mixture is heated at 110 °C for 16 h. After cooling, the organic phase is separated off, extracted three times with 300 mL of dichloromethane, dried over magensium sulfate and subsequently evaporated to dryness. The residue is recrystallized from toluene and heptane. The yield is 32 g (85.6 mmol), corresponding to 56% of theory.

### Preparation of compound (XVIII)

To the suspension of 2.4 g (60.7 mmol, 60% in oil) of NaH in in 100 mL of DMF, 20.0 g (55.2 mmol) of compound(I) in 180 mL of DMF and slowly added to the suspension and stirred for 1 h at room temperature. 24.8 g (66.4 mmol) of compound(II) is dissolved in 80 mL of DMF and slowly added to the suspension, and the reaction mixture is stirred for 16 h. The reaction mixture is poured on to ice, the organic phase is separated off, extracted three times with 200 mL of dichloromethane, dried over magnesium sulfate and subsequently evaporated to dryness. The residue is washed with ethanol, filtered off and finally dried under reduced pressure. The yield is 29 g (41.4 mmol), corresponding to 75% of theory.

### Preparation of compound (XIX)

20 g (28.6 mmol) of compound (VIII), 13.8 g (31.5 mmol) of compound (IV) and 3.3 g (31 mmol) of sodium carbonate are suspended in 600 mL of toluene and 220 mL of water. 0.33 g (0.3 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 18 g (17.7 mmol), corresponding to 62% of theory.

### Example 7

### Preparation of compound (XXIII)

### Synthetic procedure for the preparation of compound (XXIII):

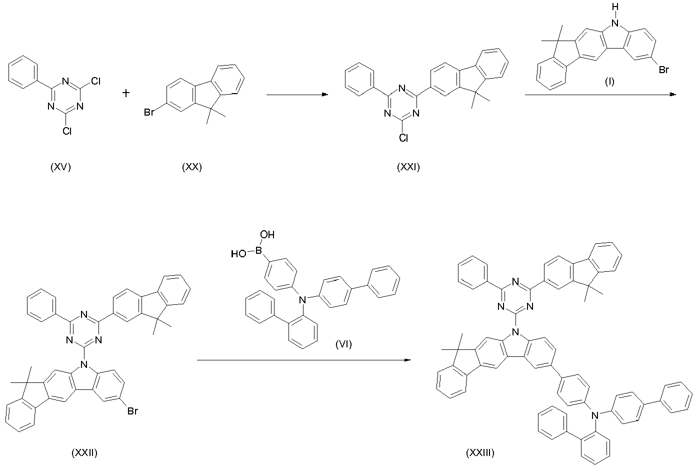

### Preparation of compound (XXI)

The solution of 50.8 g (186.0 mmol) of compound (XV) in 580 mL of THF is added to 4.5 g (185.1 mmol) of Mg turnings and the reaction mixture is stirred for 16 h. The reaction mixture is added to the solution of 35.0 g (154.8 mmol) of compound (XX) in 190 mL of THF at 0 °C. The reaction mixture is slowly warmed up to room temperature. After 24 h, the reaction mixture is quenched by 12% of HCI aqueous solution, then the organic phase is separated off, extracted three times with 500 mL of dichloromethane, dried over magensium sulfate and subsequently evaporated to dryness. The residue is purified by column chromatography on silica gel using a mixture of dichloromethane/heptanes (1/5). The yield is 24 g (62.5 mol), corresponding to 40% of theory.

### Preparation of compound (XXII)

To the suspension of 2.4 g (60.7 mmol, 60% in oil) of NaH in in 100 mL of DMF, 20.0 g (55.2 mmol) of compound(I) in 180 mL of DMF and slowly added to the suspension and stirred for 1 h at room temperature. 25.4 g (66.3 mmol) of compound(XXI) is dissolved in 80 mL of DMF and slowly added to the suspension, and the reaction mixture is stirred for 16 h. The reaction mixture is poured on to ice, the organic phase is separated off, extracted three times with 200 mL of dichloromethane, dried over magnesium sulfate and subsequently evaporated to dryness. The residue is washed with ethanol, filtered off and finally dried under reduced pressure. The yield is 30 g (42.3 mmol), corresponding to 77% of theory.

### Preparation of compound (XXIII)

20 g (28.21 mmol) of compound (XXII), 13.7 g (31.0 mmol) of compound (VI) and 3.23 g (30.5 mmol) of sodium carbonate are suspended in 600 mL of toluene and 220 mL of water. 0.33 g (0.3 mmol) of tetrakis(triphenylphosphine)palladium(0) is added to this suspension, and the reaction mixture is stirred under reflux for 16 h. After cooling, the organic phase is separated off, extracted three times with 300 mL of dichloromethane, dried over magensium sulfate and subsequently evaporated to dryness. The residue is recrystallized from toluene and heptane. The yield is 21 g (20.5 mmol), corresponding to 72% of theory.

### Example 8

### Preparation of compound (XXV)

### Synthetic procedure for the preparation of compound (XXV):

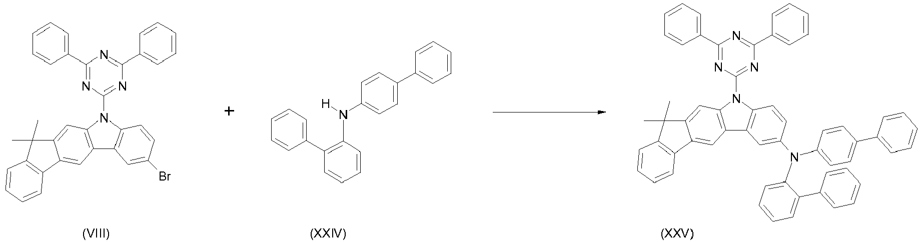

### Preparation of compound (XXV)

30.0 g (50.6 mmol) of compound (VIII) and 16.8 g (50.6 mmol) of compound (XXIV) are suspended in 500 mL of toluene under Ar atmosphere. 226 mg (1.0 mmol) of Pd(OAc)₂ are added to the flask and stirred under Ar atmosphere then 2.0 mL of a 1 M tri-tert-butylphosphine solution and 7.3 g (75.5 mmol) of sodium t-butoxide are added to the flask. The reaction mixture is stirred under reflux for 24 h. After cooling, the organic phase is separated off, washed three times with 200 mL of water, dried over magnesium sulfate, filtered and subsequently evaporated to dryness. The residue is purified by column chromatography on silica gel using a mixture of ethylacetate/heptane (1:3). The yield is 33.0 g (39.6 mmol), corresponding to 78% of theory

### Examples 9 to 12

### Preparation of a solution-processed OLED devices

Compounds related to the present invention can be processed from solution to fabricate simple OLED devices with good performances. The preparation of these solution-processed OLED devices is similar to the preparation of polymer light-emitting diodes (PLEDs), which has been already widely described in the literature (eg. Example, in WO 2004/037887). In this case the compounds according to the invention (V), (VII), (IX), (X), (XIV), (XIX), (XXIII) and (XXV) were dissolved in toluene. The typical solids content of such solutions is between 16 and 25 g / L in order to achieve for a typical device a layer thickness of 80 nm by spin coating. The OLED devices show the following structure: ITO / PEDOT: PSS / interlayer / EML / cathode, where EML is the emissive layer. The emissive layer (EML) includes not only the compounds related to this invention (V), (VII), (IX), (X), (XIV), (XIX), (XXIII) and (XXV), which are present in a concentration of 80 wt%, but also a dopant, TEG-001 (commercially available by Merck), which is present in a concentration of 20%. Structured ITO substrates and the material for the so-called buffer layer (PEDOT, actually PEDOT: PSS) are commercially available (ITO of Technoprint and others, PEDOT: PSS as an aqueous dispersion Clevios Baytron® P from HC Starck. The interlayer is used for the hole injection; in this case, HIL-012 (commercially available by Merck) was used. HIL-012 is a polymer consisting of the following monomers:

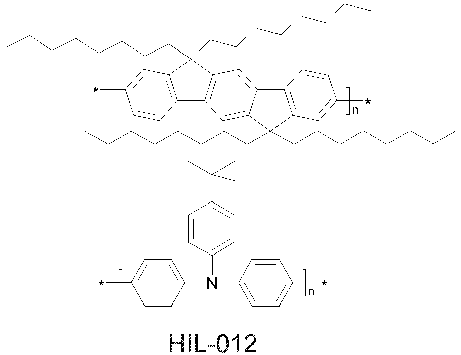

In an inert gas, argon atmospere in this case, the emission layer is spin coated and heated at 120 °C for 10 minutes. Finally, a cathode of barium and aluminum is evaporated in vacuo. Between the emitting layer and the cathode, a hole blocking layer and / or an electron transport layer can also be evaporated. Also the interlayer can be replaced by one or more layers.

The devices are characterized by standard method and OLED examples are not optimized yet.

### Structure of compounds related to the present invention and comparative examples.

| | |
|---|---|
| 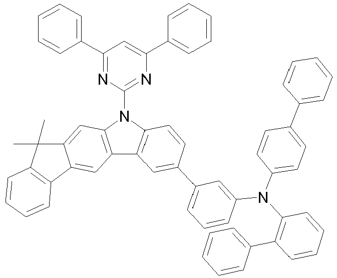 | 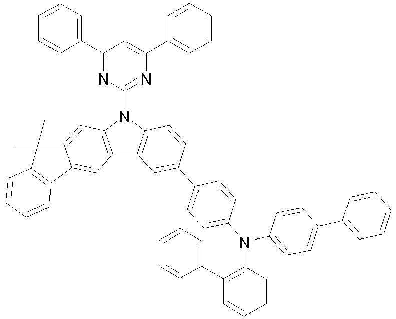 |
| (V) | (VII) |
| 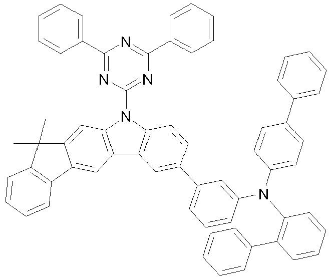 | 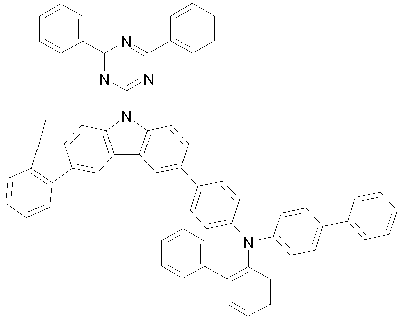 |
| (IX) | (X) |
| 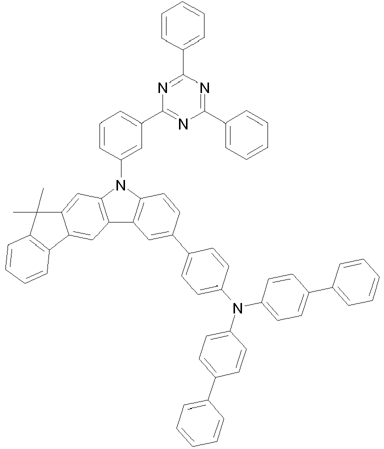 | 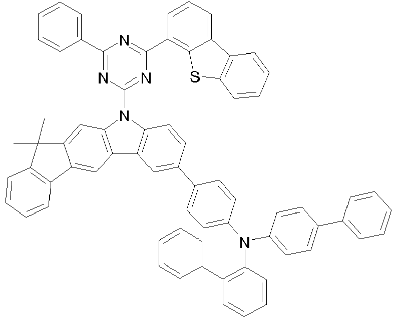 |
| (XIV) | (XIX) |
| 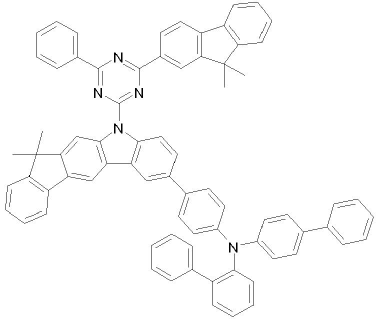 | 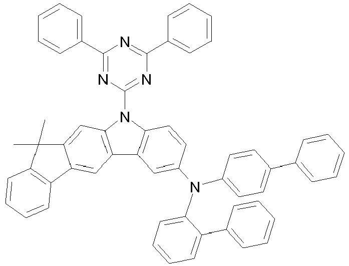 |
| (XXIII) | (XXV) |
| 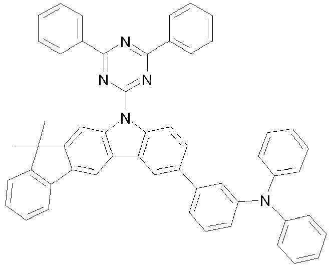 | 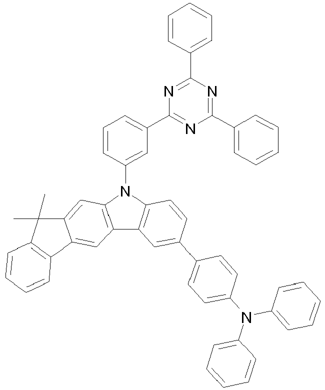 |
| A** | B** |
| 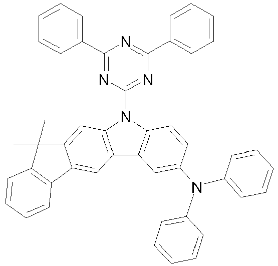 | 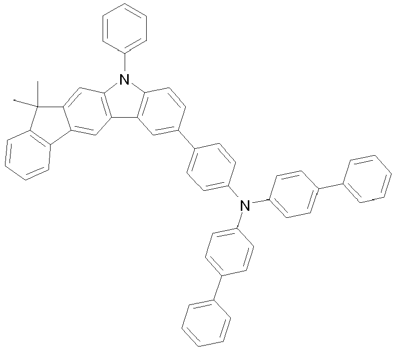 |
| C** | D** |

| | |
|---|---|
| ***for comparative purposes only* | |

### Example 9

| Compound | Max.Eff [Cd/A] | U@1000cd/m² [V] | CIE [x/y] | LT95 [h] |
|---|---|---|---|---|
| A | 48.7 | 6.2 | 0.32/0.62 | 52@8000 |
| (IX) | 63.8 | 4.2 | 0.32/0.62 | 88@8000 |

As can be seen from the results, the performances (in particular efficiency, lifetime and voltage) of a simple solution-processed OLED device fabricated with compound IX related to the present invention are better than the one obtained with comparative compound A.

### Example 10

| Compound | Max.Eff [Cd/A] | U@1000cd/m² [V] | CIE [x/y] | LT95 [h] |
|---|---|---|---|---|
| B | 50.3 | 5.9 | 0.33/0.63 | 49@8000 |
| (XIV) | 65.0 | 3.9 | 0.31/0.63 | 90@8000 |

As can be seen from the results, the performances (in particular efficiency, lifetime and voltage) of a simple solution-processed OLED device fabricated with compound XIV related to the present invention are better than the one obtained with comparative compound B.

### Example 11

| Compound | Max.Eff [Cd/A] | U@1000cd/m² [V] | CIE [x/y] | LT95 [h] |
|---|---|---|---|---|
| C | 47.9 | 5.8 | 0.33/0.61 | 50@8000 |
| (XXV) | 72.7 | 4.0 | 0.33/0.62 | 93@8000 |

As can be seen from the results, the performances (in particular efficiency, lifetime and voltage) of a simple solution-processed OLED device fabricated with compound XXV related to the present invention are better than the one obtained with comparative compound C.

### Example 12

| Compound | Max.Eff [Cd/A] | U@1000cd/m² [V] | CIE [x/y] | LT95 [h] |
|---|---|---|---|---|
| D | 48.5 | 6.0 | 0.32/0.63 | 48@8000 |
| (VII) | 66.2 | 3.8 | 0.33/0.62 | 92@8000 |
| (X) | 64.3 | 4.0 | 0.33/0.60 | 87@8000 |
| (XIV) | 65.0 | 3.9 | 0.31/0.63 | 90@8000 |
| (XIX) | 66.1 | 3.7 | 0.31/0.63 | 95@8000 |

As can be seen from the results, the performances (in particular efficiency, lifetime and voltage) of a simple solution-processed OLED device fabricated with compounds VII, X, XIV and XIX related to the present invention are better than the one obtained with comparative compound D.

## Claims

1. Compound of the formula (1-1), 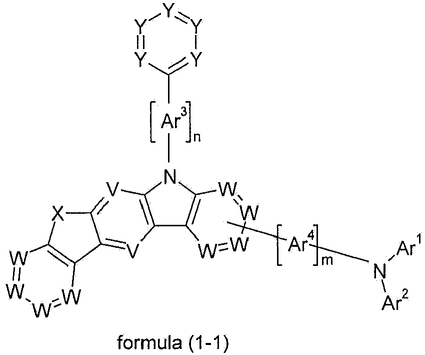 where the following applies to the symbols and indices used:
Y is, identically or differently, equal to N or CR¹, with the proviso that the group of the formula (Y), 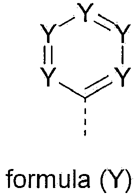 as constituent of the formula (1-1) conforms to one of the following formulae (Y-1) to (Y-6), where the dashed line denotes the bond to the remainder of the compound,
| | |
|---|---|
| 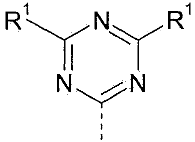 | 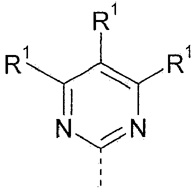 |
| formula (Y-1) | formula (Y-2) |
| 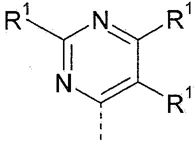 | 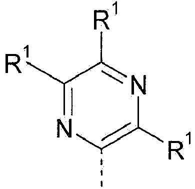 |
| formula (Y-3) | formula (Y-4) |
| 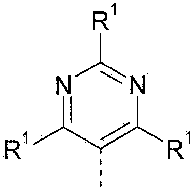 | 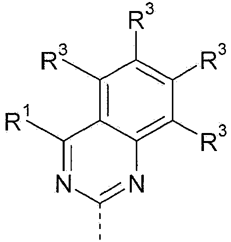 |
| formula (Y-5) | formula (Y-6) |
W is, identically or differently, equal to CR¹ or N;
V is, identically or differently, equal to CR¹ or N;
X is a divalent bridge selected from N(R²), B(R²), O, C(R²)₂, Si(R²)₂, C=NR², C=C(R²)₂, S, S=O, SO₂, P(R²) and P(=O)R²;
Ar¹, Ar² are, identically or differently, an aromatic or heteroaromatic ring system, which may be substituted by one or more radicals R¹; wherein at least one of the group Ar¹ or Ar² stands for an aromatic ring system having 12 to 30 aromatic C atoms, an heteroaryl group having 13 to 30 aromatic ring atoms or an aryl group having 10 to 20 aromatic C atoms, each of which may be substituted by one or more radicals R¹, Ar¹ and Ar² here may also be connected to one another by a group E;
Ar³, Ar⁴ are on each occurrence, identically or differently, an aromatic ring system having 6 to 40 aromatic C atoms or an heteroaromatic ring system having 5 to 40 aromatic ring atoms, which may be substituted by one or more radicals R¹; with the proviso that Ar⁴ is not an anthracenylene group;
E is on each occurrence, identically or differently, a single bond, N(R³), O, S, C(R³)₂, C(R³)₂-C(R³)₂, Si(R³)₂ or B(R³);
R¹ is on each occurrence, identically or differently, H, D, F, Br, Cl, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³, where two adjacent radicals R¹ located on Ar¹ or two adjacent radicals R¹ located on Ar² may be linked to one another and may form a ring;
R² is on each occurrence, identically or differently, H, D, F, Br, Cl, I, C(=O)R³, CN, Si(R³)₃, N(R³)₂, P(=O)(R³)₂, S(=O)R³, S(=O)₂R³, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R³ and where one or more CH₂ groups in the above-mentioned groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂, or an aromatic or heteroaromatic ring system having 5 to 30 aromatic ring atoms, which may in each case be substituted by one or more radicals R³, or an aryloxy or heteroaryloxy group having 5 to 30 aromatic ring atoms, which may be substituted by one or more radicals R³; where two radicals R² may be linked to one another and may form a ring;
R³ is on each occurrence, identically or differently, H, D, F, Br, Cl, I, C(=O)R⁴, CN, Si(R⁴)₃, N(R⁴)₂, P(=O)(R⁴)₂, S(=O)R⁴, S(=O)²R⁴, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may each be substituted by one or more radicals R⁴ and where one or more CH₂ groups in the above-mentioned groups may be replaced by -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂, or an aromatic or heteroaromatic ring system having 5 to 30 aromatic ring atoms, which may in each case be substituted by one or more radicals R⁴, or an aryloxy or heteroaryloxy group having 5 to 30 aromatic ring atoms, which may be substituted by one or more radicals R⁴, where two or more radicals R³ may be linked to one another and may form a ring;
R⁴ is on each occurrence, identically or differently, H, D, F or an aliphatic, aromatic or heteroaromatic organic radical having 1 to 20 C atoms, in which, in addition, one or more H atoms may be replaced by D or F; two or more substituents R⁴ here may be linked to one another and may form a ring;
n is equal to 0 or 1;
m is equal to 0 or 1.

2. Compound according to Claim 1, **characterised in that** the index m is equal to 1.

3. Compound according to Claim 1 or 2, **characterised in that** the index n is equal to 0.

4. Compound according to one or more of Claims 1 to 3, **characterised in that** W is equal to CR¹.

5. Compound according to one or more of Claims 1 to 4, **characterised in that** X is equal to C(R²)₂.

6. Compound according to one or more of Claims 1 to 5, **characterised in that** precisely three groups Y in the ring are equal to N, and the remaining groups Y are equal to CR¹.

7. Compound according to one or more of Claims 1 to 6, **characterised in that** Ar⁴ represents a group of the following formula (Ar⁴-I) 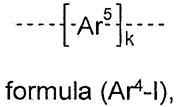 where the dashed lines represent the bonds to the indenocarbazole group and the group -NAr¹Ar²,
Ar⁵ is on each occurrence, identically or differently, an aryl or heteroaryl group having 6 to 18 aromatic ring atoms, which may be substituted by one or more radicals R¹, where R¹ is defined as in Claim 1; and
k is 1, 2, 3 or 4, where the index k is selected so that the number of aromatic ring atoms in the entire group Ar⁴ does not exceed the number 40.

8. Compound according to one or more of Claims 1 to 7, **characterised in that** at least one of the groups Ar¹ and Ar² is selected, identically or differently, from the group consisting of biphenyl, terphenyl, quaterphenyl, fluorenyl, spirobifluorenyl, carbazolyl, dibenzofuranyl, dibenzothiophenyl, naphtyl, anthracyl, phenantryl, chrysenyl, triphenylenyl, pyrenyl, perylenyl, each of which may be substituted by one or more radicals R³.

9. Compound according to one or more of the preceding claims, **characterised in that** at least one of the groups Ar¹ and Ar² is selected, identically or differently, from the groups of the following formulae (11) to (77), 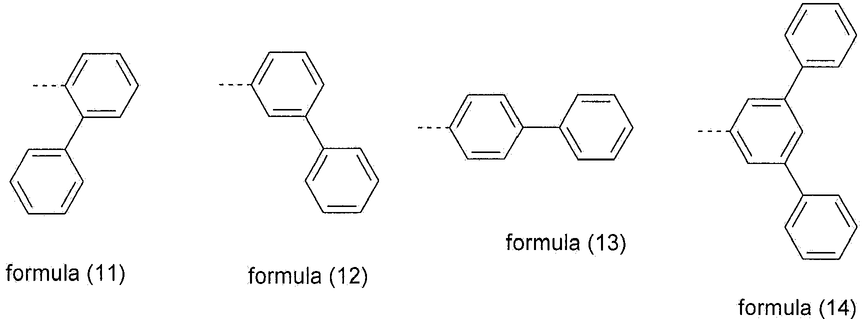 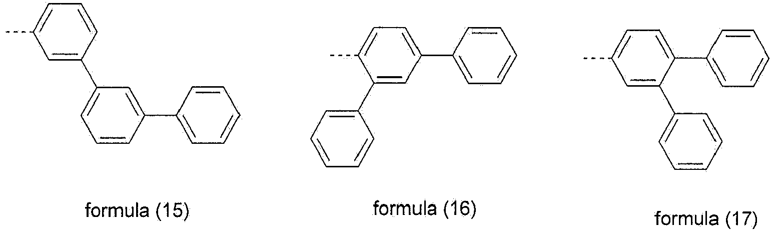 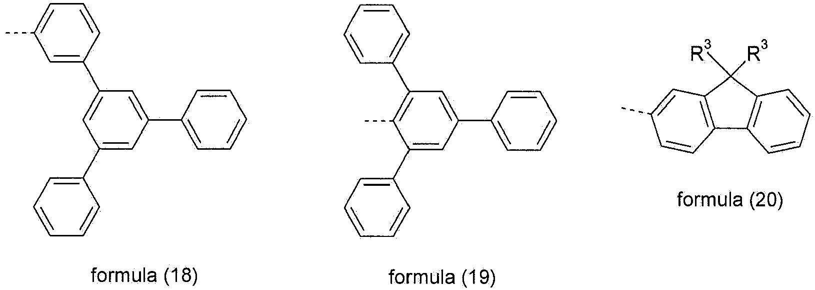 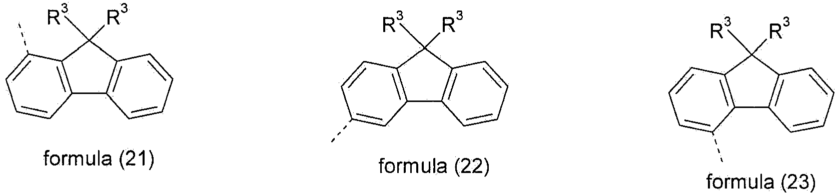 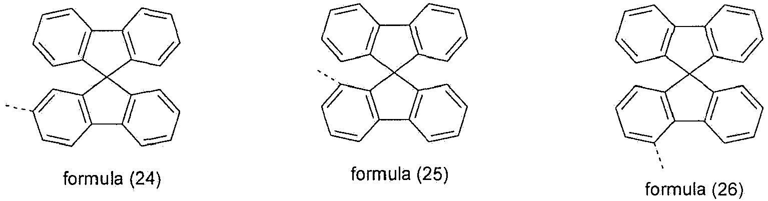 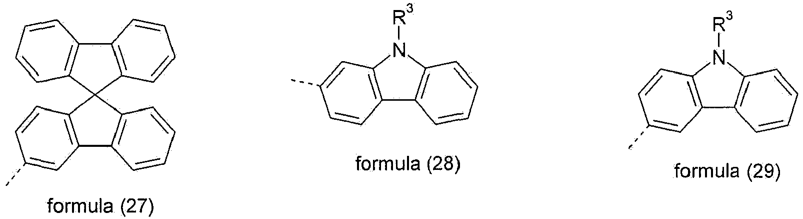 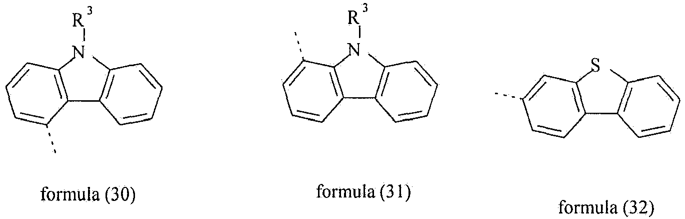 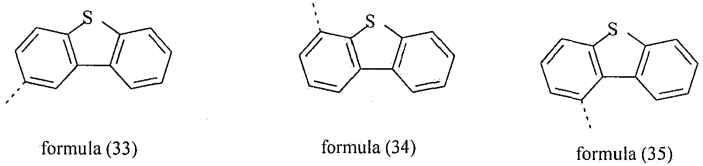 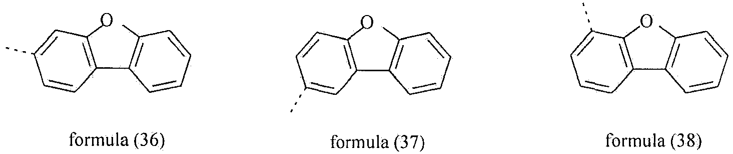 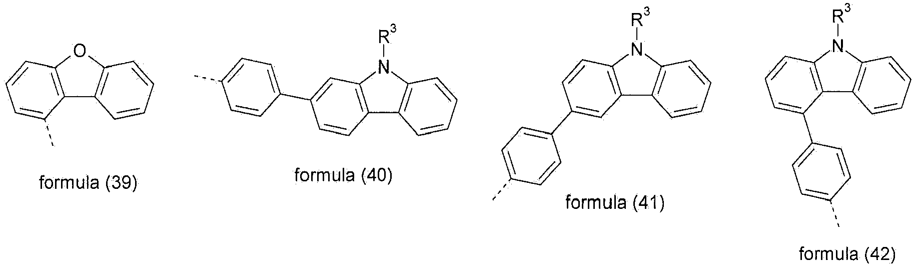 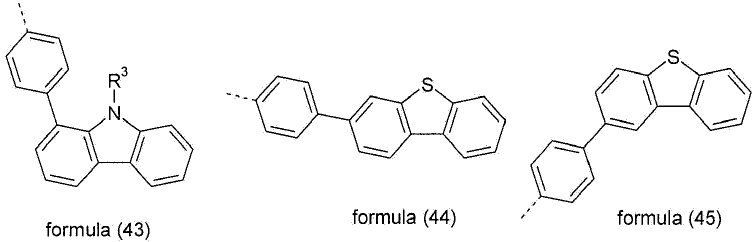 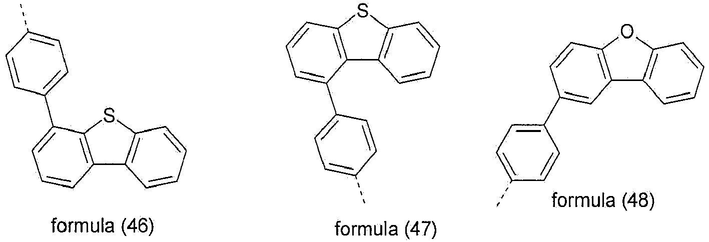 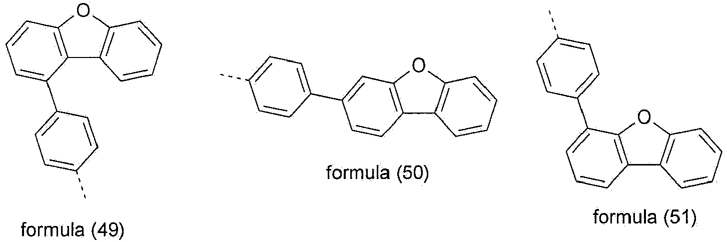 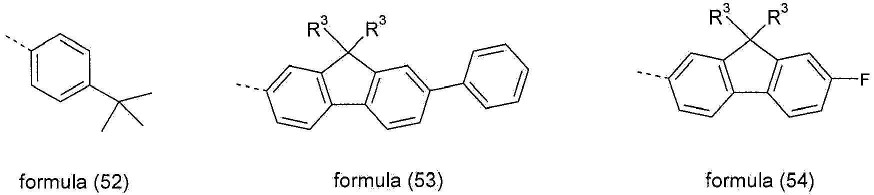 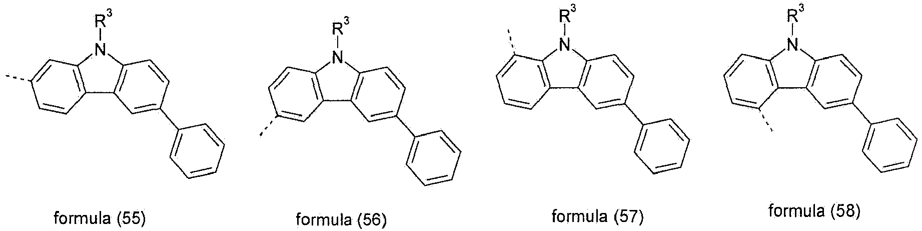 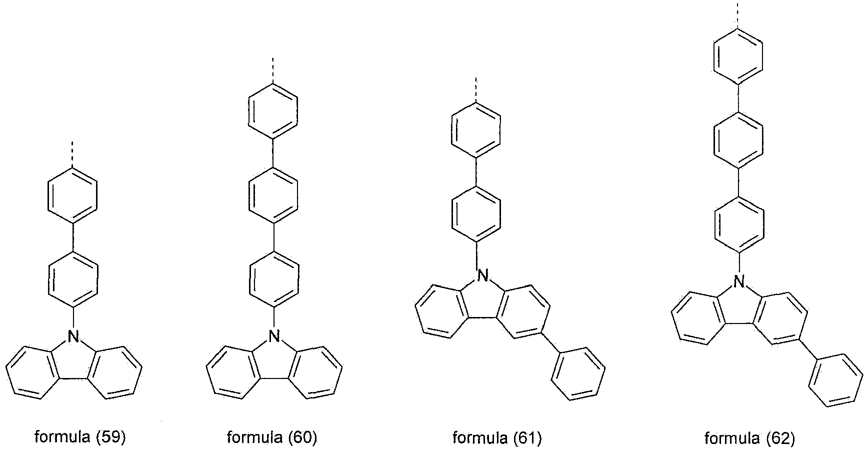 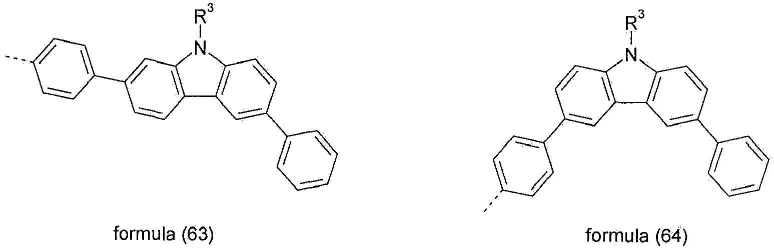 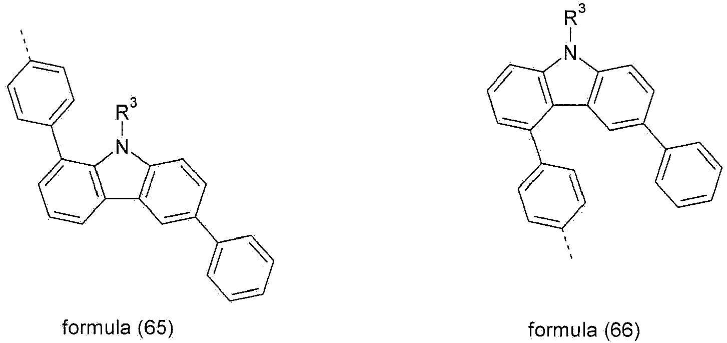 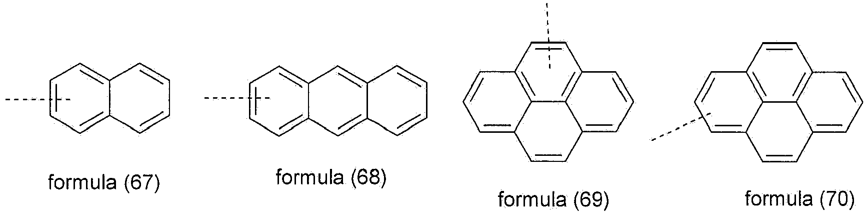 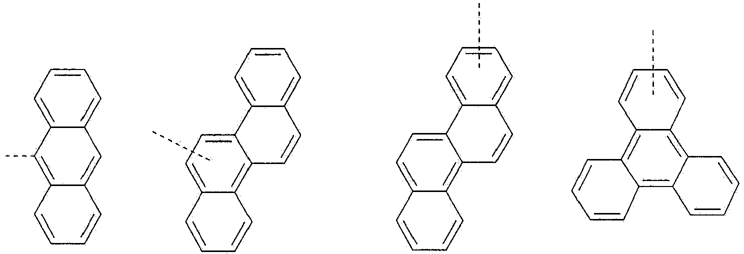 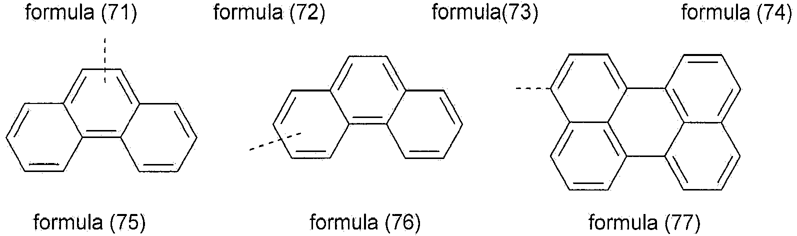 where the dashed lines represent the bonds to the N atom and the group Ar¹ or Ar², and the groups may be substituted by one or more radicals R³ as defined in claim 1.

10. Oligomer, polymer or dendrimer containing one or more compounds according to one or more of Claims 1 to 9, where the bond(s) to the polymer, oligomer or dendrimer may be localised at any desired positions in formula (I) which are substituted by R¹ or R².

11. Formulation comprising at least one compound according to one or more of Claims 1 to 9 or at least one polymer, oligomer or dendrimer according to Claim 10 and at least one solvent.

12. Electronic device selected from organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs), **characterised in that** it comprises at least one compound according to one or more of Claims 1 to 9.

13. Electronic device according to Claim 12, selected from organic electroluminescent devices, **characterised in that** it comprises anode, cathode and at least one emitting layer, where at least one layer of the device, selected from emitting layers, electron-transport layers, electron-injection layers and hole-blocking layers, comprises at least one compound according to one or more of Claims 1 to 9.

14. Electronic device according to Claim 13, **characterised in that** the compound according to one or more of Claims 1 to 9 is present in an emitting layer in combination with one or more phosphorescent emitter compounds.

15. Process for the preparation of a compound according to one or more of Claims 1 to 9, **characterised in that** an indenocarbazole compound is reacted with an aromatic or heteroaromatic ring system which contains an electron-deficient heteroaryl group, where the aromatic or heteroaromatic ring system is coupled to the nitrogen atom of the indenocarbazole.

16. Use of a compound according to one or more of Claims 1 to 9 in an electronic device.

## Patentansprüche

1. Verbindung der Formel (1-1) 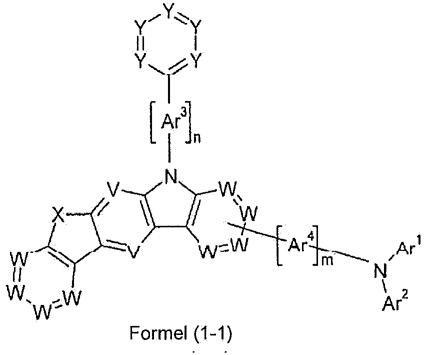 wobei für die verwendeten Symbole und Indizien Folgendes gilt:
Y ist gleich oder verschieden gleich N oder CR¹, mit der Maßgabe, dass die Gruppe der Formel (Y) 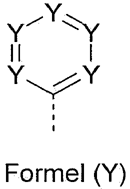 als Bestandteil der Formel (1-1) einer der folgenden Formeln (Y-1) bis (Y-6) entspricht, wobei die gestrichelte Linie die Bindung zum Rest der Verbindung bedeutet,
| | |
|---|---|
| 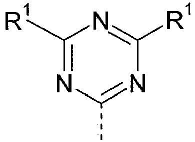 | 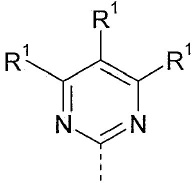 |
| Formel (Y-1) | Formel (Y-2) |
| 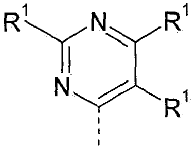 | 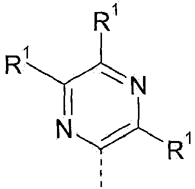 |
| Formel (Y-3) | Formel (Y-4) |
| 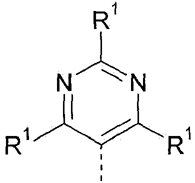 | 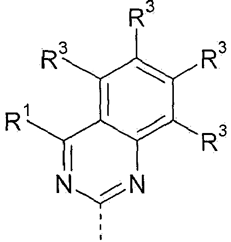 |
| Formel (Y-5) | Formel (Y-6) |
W ist gleich oder verschieden gleich CR¹ oder N;
V ist gleich oder verschieden gleich CR¹ oder N;
X ist eine zweiwertige, aus N(R²), B(R²), O, C(R²)₂, Si(R²)₂, C=NR², C=C(R²)₂, S, S=O, SO₂, P(R²) und P(=O)R² ausgewählte Brücke;
Ar¹, Ar² sind gleich oder verschieden ein aromatisches oder heteroaromatisches Ringsystem, das durch einen oder mehrere Reste R¹ substituiert sein kann; bei denen mindestens eine der Gruppe Ar¹ oder Ar² für ein aromatisches Ringsystem mit 12 bis 30 aromatischen C-Atomen, eine Heteroarylgruppe mit 13 bis 30 aromatischen Ringatomen oder eine Arylgruppe mit 10 bis 20 aromatischen C-Atomen steht, die jeweils durch einen oder mehrere Reste R¹ substituiert sein können, Ar¹ und Ar² können hier auch durch eine Gruppe E miteinander verbunden sein;
Ar³, Ar⁴ sind bei jedem Auftreten gleich oder verschieden ein aromatisches Ringsystem mit 6 bis 40 aromatischen C-Atomen oder ein heteroaromatisches Ringsystem mit 5 bis 40 aromatischen Ringatomen, das durch einen oder mehrere Reste R¹ substituiert sein kann; mit der Maßgabe, dass Ar⁴ keine Anthracenylengruppe ist;
E ist bei jedem Auftreten gleich oder verschieden eine Einfachbindung, N(R³), O, S, C(R³)₂, C(R³)2-C(R³)₂, Si(R³)₂ oder B(R³);
R¹ ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die vorstehend genannten Gruppen jeweils durch einen oder mehrere Reste R³ substituiert sein können und wobei eine oder mehrere CH₂-Gruppen in den vorstehend genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R³ substituiert sein kann, oder eine Aryloxy- oder Heteroaryloxygruppe mit 5 bis 30 aromatischen Ringatomen, die durch einen oder mehrere Reste R³ substituiert sein kann, wobei zwei am Ar¹ befindliche benachbarte Reste R¹ oder zwei am Ar² befindliche benachbarte Reste R¹ miteinander verknüpft sein können und einen Ring bilden können;
R² ist bei jedem Auftreten gleich oder verschieden H, D, F, Br, Cl, I, C(=O)R³, CN, Si(R³)₃, N(R³)₂, P(=O)(R³)₂, S(=O)R³, S(=O)₂R³, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die vorstehend genannten Gruppen jeweils durch einen oder mehrere Reste R³ substituiert sein können und wobei eine oder mehrere CH₂-Gruppen in den vorstehend genannten Gruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁵ substituiert sein kann, oder eine Aryloxy- oder Heteroaryloxygruppe mit 5 bis 30 aromatischen Ringatomen, die durch einen oder mehrere Reste R³ substituiert sein kann; wobei zwei Reste R² miteinander verknüpft sein können und einen Ring bilden können;
R³ ist bei jedem Auftreten gleich oder verschieden H, D, F, Br, Cl, I, C(=O)R⁴, CN, Si(R⁴)₃, N(R⁴)₂, P(=O)(R⁴)₂, S(=O)R⁴, S(=O)²R⁴, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die vorstehend genannten Gruppen jeweils durch einen oder mehrere Reste R⁴ substituiert sein können und wobei eine oder mehrere CH₂-Gruppen in den vorstehend genannten Gruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können, oder ein aromatisches oder heteroaromatisches Ringsystem mit 5 bis 30 aromatischen Ringatomen, das jeweils durch einen oder mehrere Reste R⁴ substituiert sein kann, oder eine Aryloxy- oder Heteroaryloxygruppe mit 5 bis 30 aromatischen Ringatomen, die durch einen oder mehrere Reste R⁴ substituiert sein kann, wobei zwei oder mehr Reste R³ miteinander verknüpft sein können und einen Ring bilden können;
R⁴ ist bei jedem Auftreten gleich oder verschieden H, D, F oder ein aliphatischer, aromatischer oder heteroaromatischer organischer Rest mit 1 bis 20 C-Atomen, in dem zusätzlich ein oder mehrere H-Atome durch D oder F ersetzt sein können; zwei oder mehr Substituenten R⁴ können hier miteinander verknüpft sein und können einen Ring bilden;
n ist gleich 0 oder 1;
m ist gleich 0 oder 1.

2. Verbindung nach Anspruch 1, **dadurch gekennzeichnet, dass** der Index m gleich 1 ist.

3. Verbindung nach Anspruch 1 oder 2, **dadurch gekennzeichnet, dass** der Index n gleich 0 ist.

4. Verbindung nach einem oder mehreren der Ansprüche 1 bis 3, **dadurch gekennzeichnet, dass** W gleich CR¹ ist.

5. Verbindung nach einem oder mehreren der Ansprüche 1 bis 4, **dadurch gekennzeichnet, dass** X gleich C(R²)₂ ist.

6. Verbindung nach einem oder mehreren der Ansprüche 1 bis 5, **dadurch gekennzeichnet, dass** genau drei Gruppen Y in dem Ring gleich N sind und die restlichen Gruppen Y gleich CR¹ sind.

7. Verbindung nach einem oder mehreren der Ansprüche 1 bis 6, **dadurch gekennzeichnet, dass** Ar⁴ eine Gruppe der folgenden Formel (Ar⁴-I) 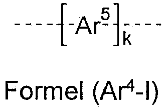 darstellt, wobei die gestrichelten Linien die Bindungen zu der Indenocarbazolgruppe und der Gruppe -NAr¹Ar² darstellen,
Ar⁵ bei jedem Auftreten gleich oder verschieden eine Aryl- oder Heteroarylgruppe mit 6 bis 18 aromatischen Ringatomen ist, die durch einen oder mehrere Reste R¹ substituiert sein kann, wobei R¹ wie in Anspruch 1 definiert ist; und
k 1, 2, 3 oder 4 ist, wobei der Index k so gewählt ist, dass die Anzahl der aromatischen Ringatome in der gesamten Gruppe Ar⁴ die Zahl 40 nicht überschreitet.

8. Verbindung nach einem oder mehreren der Ansprüche 1 bis 7, **dadurch gekennzeichnet, dass** mindestens eine der Gruppen Ar¹ und Ar² gleich oder verschieden aus der Gruppe bestehend aus Biphenyl, Terphenyl, Quaterphenyl, Fluorenyl, Spirobifluorenyl, Carbazolyl, Dibenzofuranyl, Dibenzothiophenyl, Naphtyl, Anthracyl, Phenantryl, Chrysenyl, Triphenylenyl, Pyrenyl, Perylenyl ausgewählt ist, die jeweils durch einen oder mehrere Reste R³ substituiert sein können.

9. Verbindung nach einem oder mehreren der vorhergehenden Ansprüche, **dadurch gekennzeichnet, dass** mindestens eine der Gruppen Ar¹ und Ar² gleich oder verschieden ausgewählt ist aus den Gruppen der folgenden Formeln (11) bis (77), 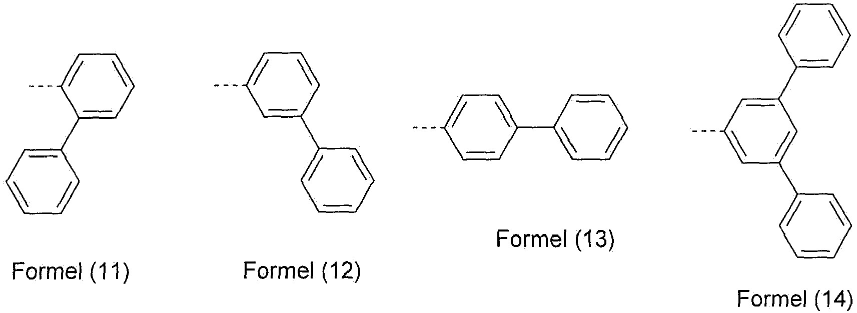 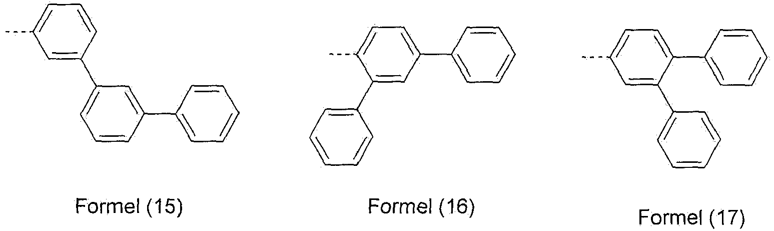 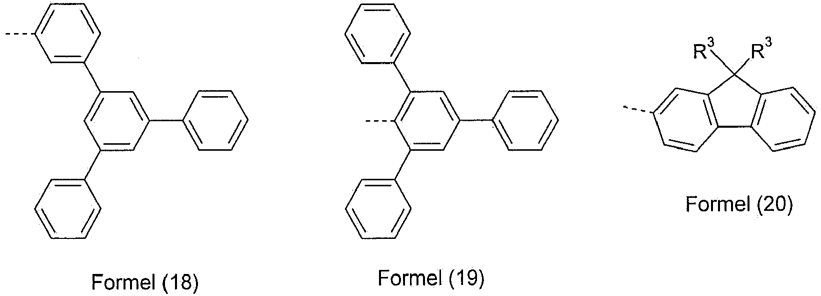 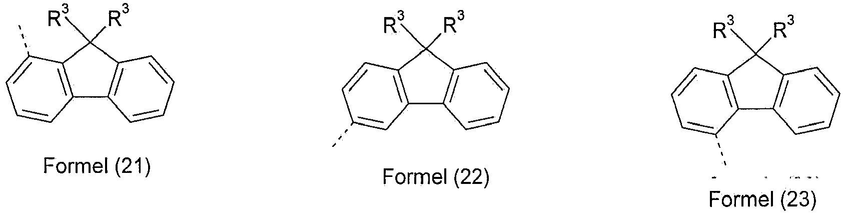 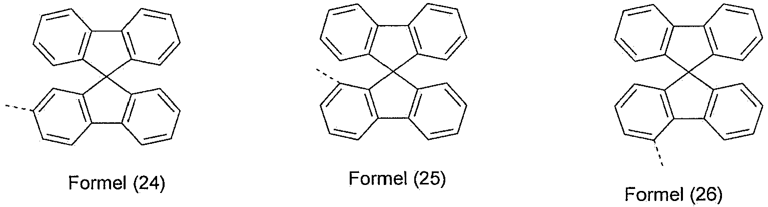 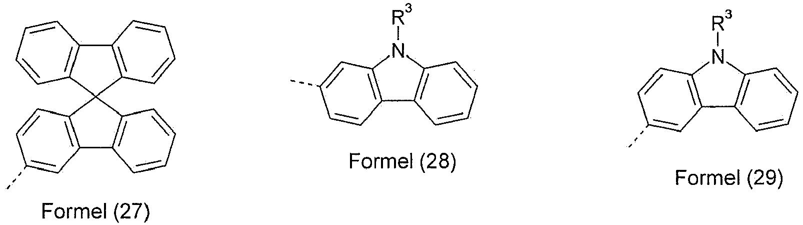 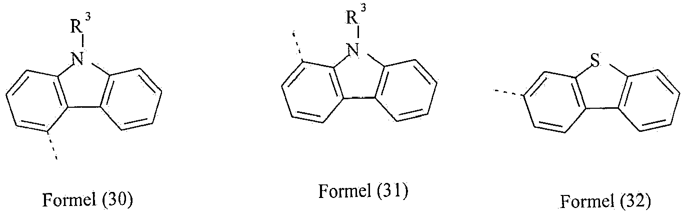 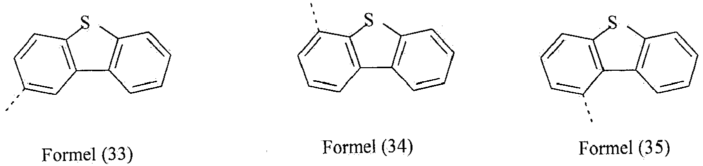 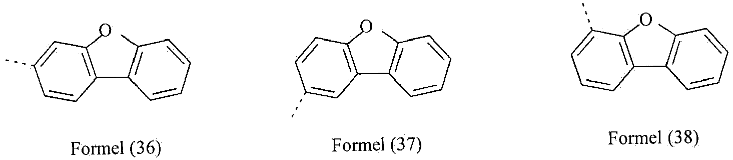 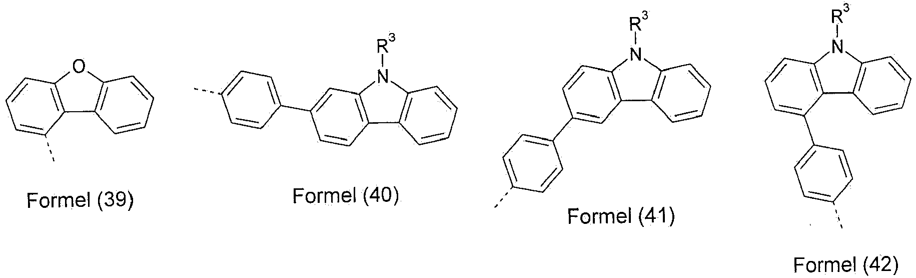 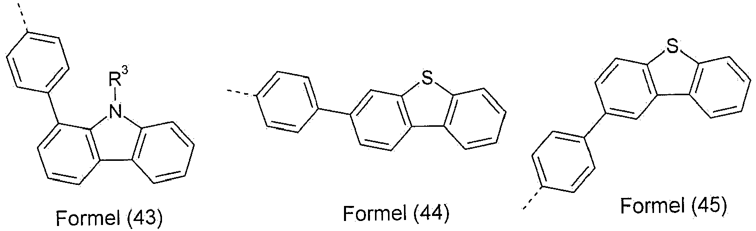 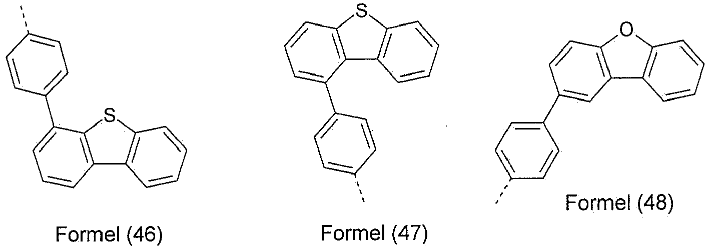 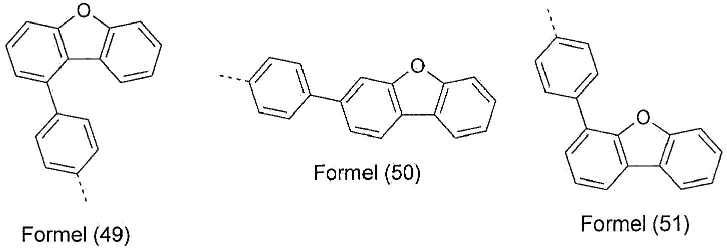 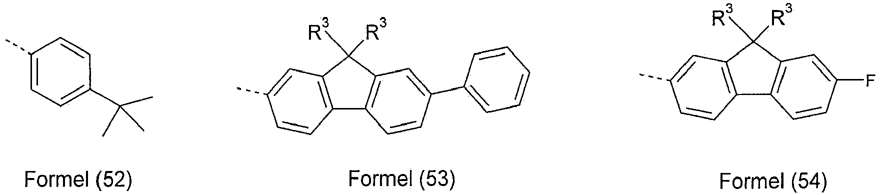 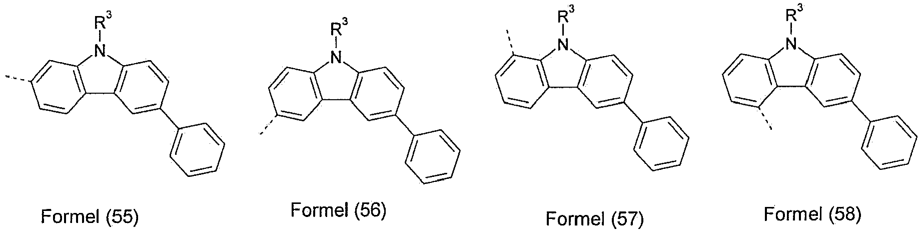 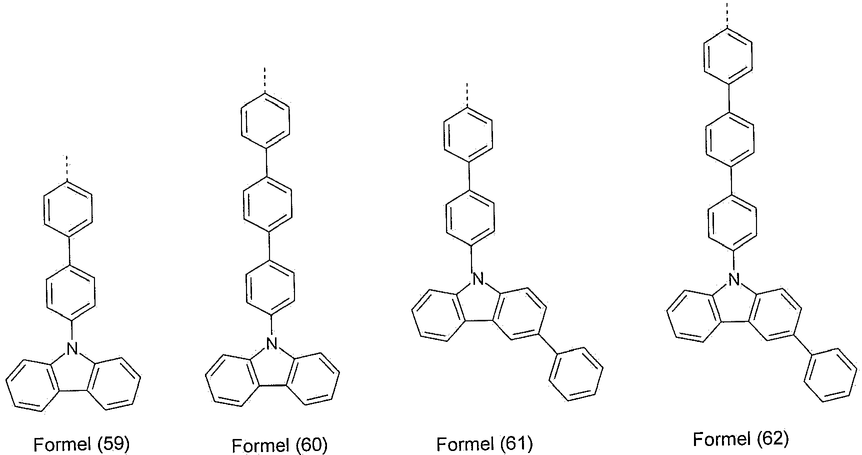 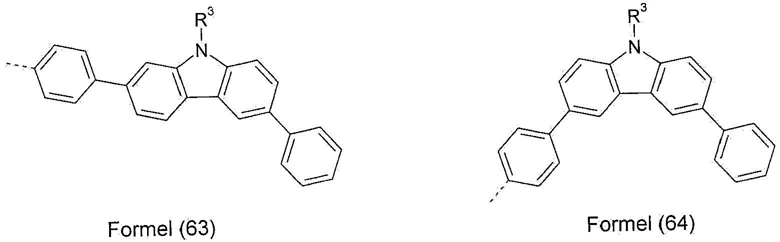 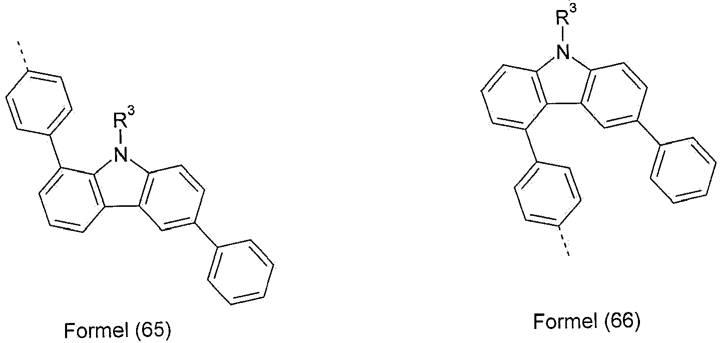 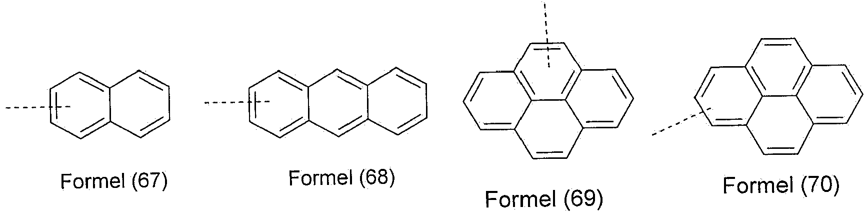 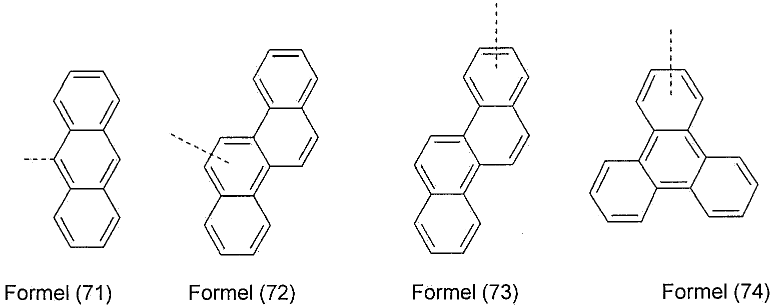 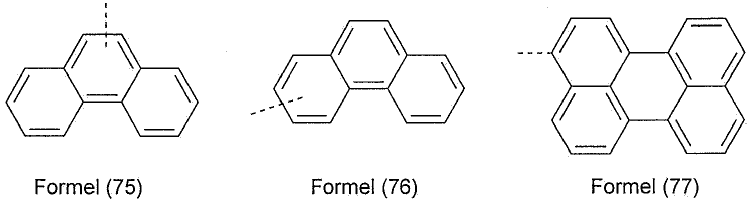 wobei die gestrichelten Linien die Bindung zu dem N-Atom und der Gruppe Ar¹ oder Ar² darstellen und die Gruppen durch einen oder mehrere Reste R³ wie in Anspruch 1 definiert substituiert sein können.

10. Oligomer, Polymer oder Dendrimer enthaltend eine oder mehrere Verbindungen nach einem oder mehreren der Ansprüche 1 bis 9, wobei die Bindung(en) zum Polymer, Oligomer oder Dendrimer an beliebigen mit R¹ oder R² substituierten Positionen in Formel (I) lokalisiert sein können.

11. Formulierung enthaltend mindestens eine Verbindung nach einem oder mehreren der Ansprüche 1 bis 9 oder mindestens ein Polymer, Oligomer oder Dendrimer nach Anspruch 10 sowie mindestens ein Lösungsmittel.

12. Elektronische Vorrichtung ausgewählt aus organischen integrierten Schaltungen (OICs), organischen Feld-Effekt-Transistoren (OFETs), organischen Dünnschichttransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs), **dadurch gekennzeichnet, dass** sie mindestens eine Verbindung nach einem oder mehreren der Ansprüche 1 bis 9 enthält.

13. Elektronische Vorrichtung nach Anspruch 12, ausgewählt aus organischen Elektrolumineszenzvorrichtungen, **dadurch gekennzeichnet, dass** sie Anode, Kathode und mindestens eine emittierende Schicht enthält, wobei mindestens eine aus emittierenden Schichten, Elektronentransportschichten, Elektroneninjektionsschichten und Lochblockierschichten ausgewählte Schicht der Vorrichtung mindestens eine Verbindung nach einem oder mehreren der Ansprüche 1 bis 9 enthält.

14. Elektronische Vorrichtung nach Anspruch 13, **dadurch gekennzeichnet, dass** die Verbindung nach einem oder mehreren der Ansprüche 1 bis 9 in einer emittierenden Schicht in Kombination mit einer oder mehreren phosphoreszierenden Emitterverbindungen vorhanden ist.

15. Verfahren zur Herstellung einer Verbindung nach einem oder mehreren der Ansprüche 1 bis 9, **dadurch gekennzeichnet, dass** man eine Indenocarbazol-Verbindung mit einem aromatischen oder heteroaromatischen Ringsystem umsetzt, das eine elektronenarme Heteroarylgruppe enthält, wobei das aromatische oder heteroaromatische Ringsystem an das Stickstoffatom des Indenocarbazols gekuppelt wird.

16. Verwendung einer Verbindung nach einem oder mehreren der Ansprüche 1 bis 9 in einer elektronischen Vorrichtung.

## Revendications

1. Composé de la formule (1-1) : 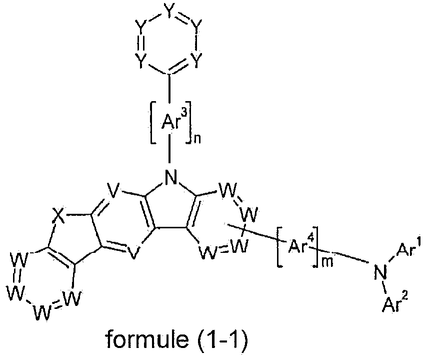 dans laquelle ce qui suit s'applique aux symboles et aux indices qui sont utilisés :
Y est, de manière identique ou différente, égal N ou CR¹, étant entendu que le groupe de la formule (Y) : 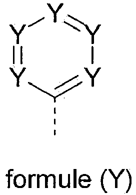 en tant que constituant de la formule (1-1) est conforme à l'une des formules (Y-1) à (Y-6) qui suivent, dans lesquelles la ligne en pointillés représente la liaison sur le reste du composé :
| | |
|---|---|
| 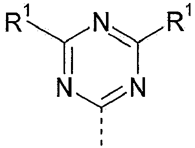 | 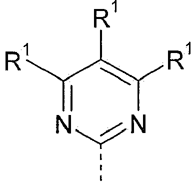 |
| formule (Y-1) | formule (Y-2) |
| 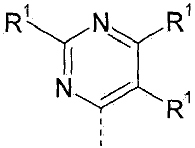 | 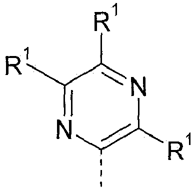 |
| formule (Y-3) | formule (Y-4) |
| 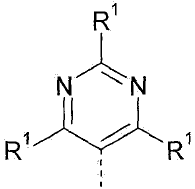 | 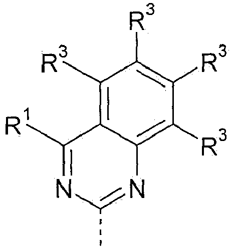 |
| formule (Y-5) | formule (Y-6) |
W est, de manière identique ou différente, égal à CR¹ ou N ;
V est, de manière identique ou différente, égal à CR¹ ou N ;
X est un pont divalent qui est sélectionné parmi N(R²), B(R²), O, C(R²)₂, Si(R²)₂, C=NR², C=C(R²)₂, S, S=O, SO₂, P(R²) et P(=O)R² ;
Ar¹, Ar² sont, de manière identique ou différente, un système de cycle aromatique ou hétéroaromatique, lequel peut être substitué par un radical ou par plusieurs radicaux R¹ ; où au moins l'un des groupes Ar¹ et Ar² représente un système de cycle aromatique qui comporte de 12 à 30 atomes de C aromatique, un groupe hétéroaryle qui comporte de 13 à 30 atomes de cycle aromatique ou un groupe aryle qui comporte de 10 à 20 atomes de C aromatique, dont chacun peut être substitué par un radical ou par plusieurs radicaux R¹, Ar¹ et Ar² peuvent ici également être connectés l'un à l'autre par un groupe E ;
Ar³, Ar⁴ sont pour chaque occurrence, de manière identique ou différente, un système de cycle aromatique qui comporte de 6 à 40 atomes de C aromatique ou un système de cycle hétéroaromatique qui comporte de 5 à 40 atomes de cycle aromatique, lequel peut être substitué par un radical ou par plusieurs radicaux R¹ ; étant entendu que Ar⁴ n'est pas un groupe anthracénylène ;
E est pour chaque occurrence, de manière identique ou différente, une liaison simple, N(R³), O, S, C(R³)₂, C(R³)₂-C(R³)₂, Si(R³)₂ ou B(R³) ;
R¹ est pour chaque occurrence, de manière identique ou différente, H, D, F, Br, Cl, I, C(=O)R³, CN, Si(R³)₃, N(R³)₂, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent chacun être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³, où deux radicaux R¹ adjacents qui sont localisés sur Ar¹ ou deux radicaux R¹ adjacents qui sont localisés sur Ar² peuvent être liés l'un à l'autre et peuvent former un cycle ;
R² est pour chaque occurrence, de manière identique ou différente, H, D, F, Br, Cl, I, C(=O)R³, CN, Si(R³)₃, N(R³)₂, P(=O)(R³)₂, S(=O)R³, S(=O)₂R³, un groupe alkyle ou alcoxy en chaîne droite qui comporte de 1 à 20 atome(s) de C ou un groupe alkyle ou alcoxy ramifié ou cyclique qui comporte de 3 à 20 atomes de C, ou un groupe alkényle ou alkynyle qui comporte de 2 à 20 atomes de C, où les groupes qui ont été mentionnés ci-avant peuvent chacun être substitués par un radical ou par plusieurs radicaux R³ et où un ou plusieurs groupe(s) CH₂ dans les groupes qui ont été mentionnés ci-avant peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R³, ou un groupe aryloxy ou hétéroaryloxy qui comporte de 5 à 30 atomes de cycle aromatique, lequel peut être substitué par un radical ou par plusieurs radicaux R³ ; où deux radicaux R² peuvent être liés l'un à l'autre et peuvent former un cycle ;
R³ est pour chaque occurrence, de manière identique ou différente, H, D, F, Br, Cl, I, C(=O)R⁴, CN, Si(R⁴)₃, N(R⁴)₂, P(=O)(R⁴)₂, S(=O)R⁴, S(=O)²R⁴, un groupe alkyle ou alcoxy en chaîne droite qui comporte de 1 à 20 atome(s) de C ou un groupe alkyle ou alcoxy ramifié ou cyclique qui comporte de 3 à 20 atomes de C ou un groupe alkényle ou alkynyle qui comporte de 2 à 20 atomes de C, où les groupes qui ont été mentionnés ci-avant peuvent chacun être substitués par un radical ou par plusieurs radicaux R⁴ et où un ou plusieurs groupe(s) CH₂ dans les groupes qui ont été mentionnés ci-avant peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂, ou un système de cycle aromatique ou hétéroaromatique qui comporte de 5 à 30 atomes de cycle aromatique, lequel peut dans chaque cas être substitué par un radical ou par plusieurs radicaux R⁴, ou un groupe aryloxy ou hétéroaryloxy qui comporte de 5 à 30 atomes de cycle aromatique, lequel peut être substitué, par un radical ou par plusieurs radicaux R⁴, où deux radicaux R³ ou plus peuvent être liés l'un à l'autre ou les uns aux autres et peuvent former un cycle ;
R⁴ est pour chaque occurrence, de manière identique ou différente, H, D, F ou un radical organique aliphatique, aromatique ou hétéroaromatique qui comporte de 1 à 20 atome(s) de C, où, en outre, un ou plusieurs atome(s) de H peut/peuvent être remplacé(s) par D ou F ; deux substituants R⁴ ou plus peuvent ici être liés l'un à l'autre ou les uns aux autres et peuvent former un cycle ;
n est égal à 0 ou 1 ; et
m est égal à 0 ou 1.

2. Composé selon la revendication 1, **caractérisé en ce que** l'indice m est égal à 1.

3. Composé selon la revendication 1 ou 2, **caractérisé en ce que** l'indice n est égal à 0.

4. Composé selon une ou plusieurs des revendications 1 à 3, **caractérisé en ce que** W est égal à CR¹.

5. Composé selon une ou plusieurs des revendications 1 à 4, **caractérisé en ce que** X est égal à C(R²)₂.

6. Composé selon une ou plusieurs des revendications 1 à 5, **caractérisé en ce que** précisément trois groupes Y dans le cycle sont égaux à N, et les groupes Y restants sont égaux à CR¹.

7. Composé selon une ou plusieurs des revendications 1 à 6, **caractérisé en ce que** Ar⁴ représente un groupe de la formule (Ar⁴-I) qui suit : 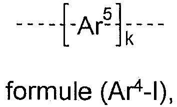 dans laquelle les lignes en pointillés représentent les liaisons sur le groupe indénocarbazole et sur le groupe -NAr¹Ar² ;
Ar⁵ est pour chaque occurrence, de manière identique ou différente, un groupe aryle ou hétéroaryle qui comporte de 6 à 18 atomes de cycle aromatique, lequel peut être substitué par un radical ou par plusieurs radicaux R¹, où R¹ est défini tel que selon la revendication 1 ; et
k est 1, 2, 3 ou 4, où l'indice k est sélectionné de telle sorte que le nombre d'atomes de cycle aromatique dans le groupe complet Ar⁴ n'excède pas le nombre 40.

8. Composé selon une ou plusieurs des revendications 1 à 7, **caractérisé en ce qu'**au moins l'un des groupes Ar¹ et Ar² est sélectionné, de manière identique ou différente, parmi le groupe qui est constitué par biphényle, terphényle, quaterphényle, fluorényle, spirobifluorényle, carbazolyle, dibenzofuranyle, dibenzothiophényle, naphtyle, anthracyle, phénantryle, chrysényle, triphénylényle, pyrényle, pérylényle, dont chacun peut être substitué par un radical ou par plusieurs radicaux R³.

9. Composé selon une ou plusieurs des revendications qui précèdent, **caractérisé en ce qu'**au moins l'un des groupes Ar¹ et Ar² est sélectionné, de manière identique ou différente, parmi les groupes des formules (11) à (77) qui suivent : 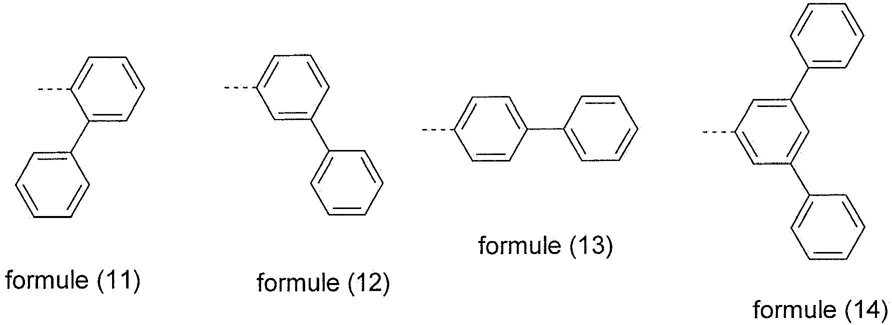 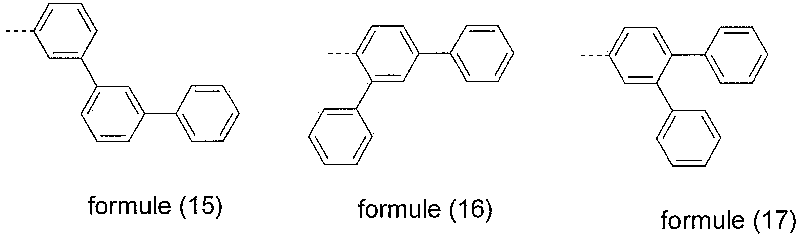 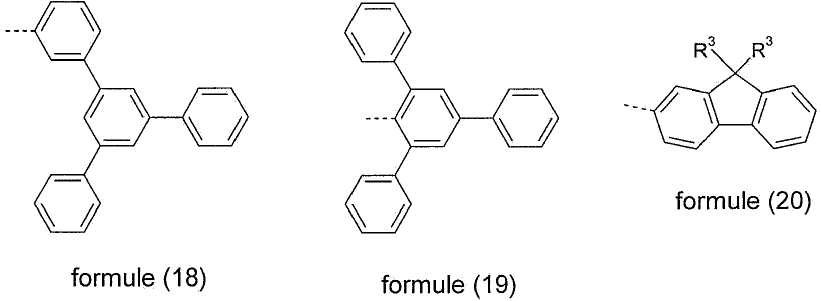 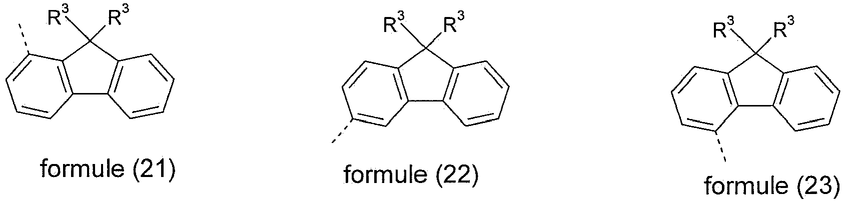 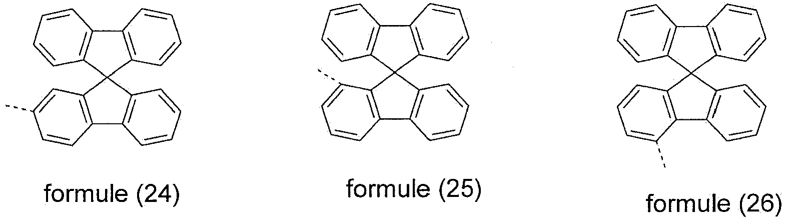 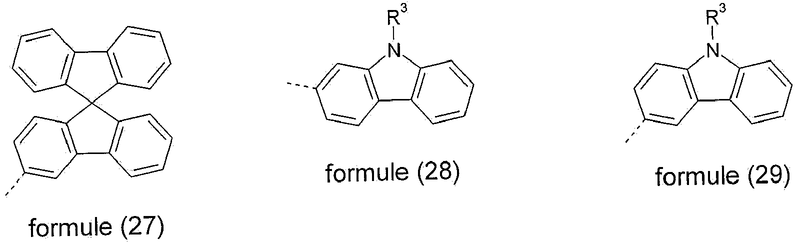 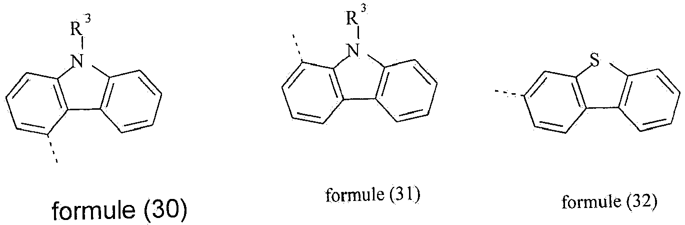 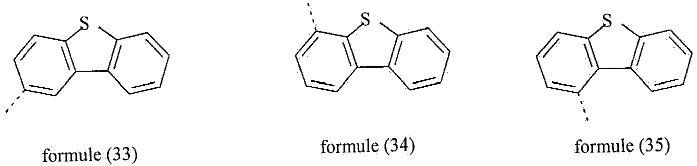 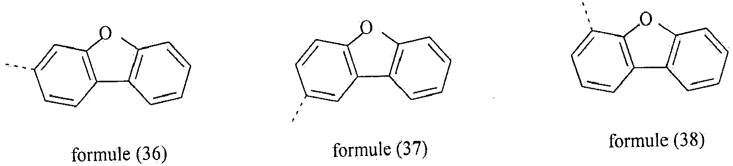 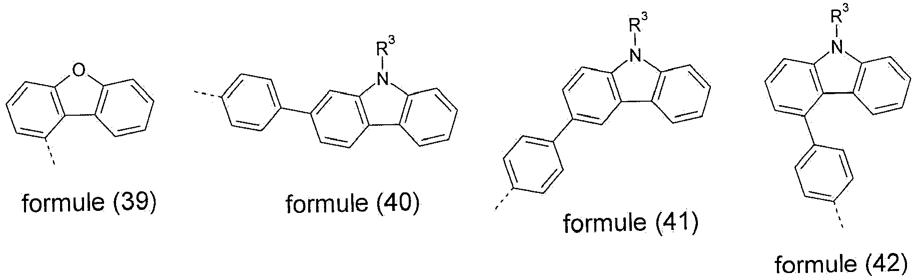 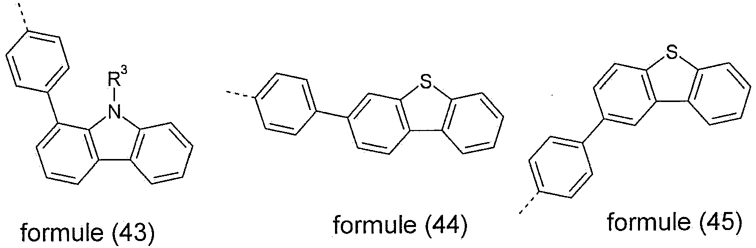 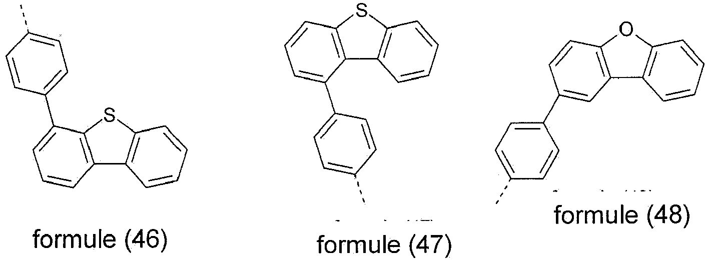 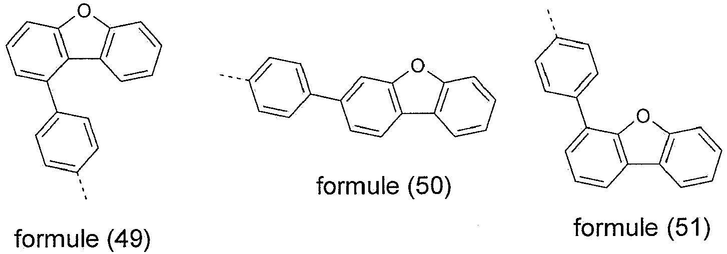 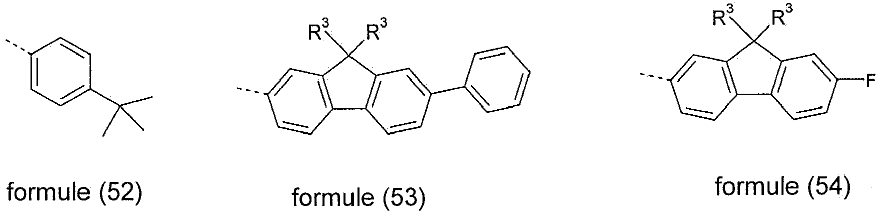 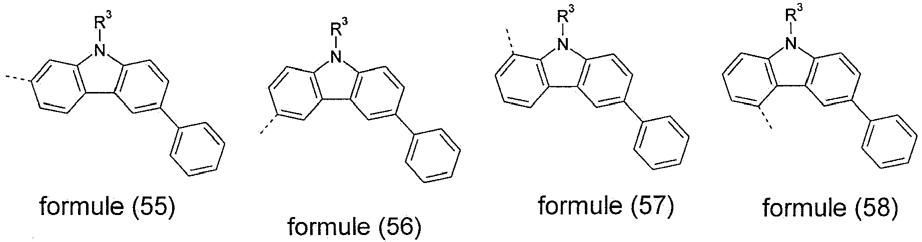 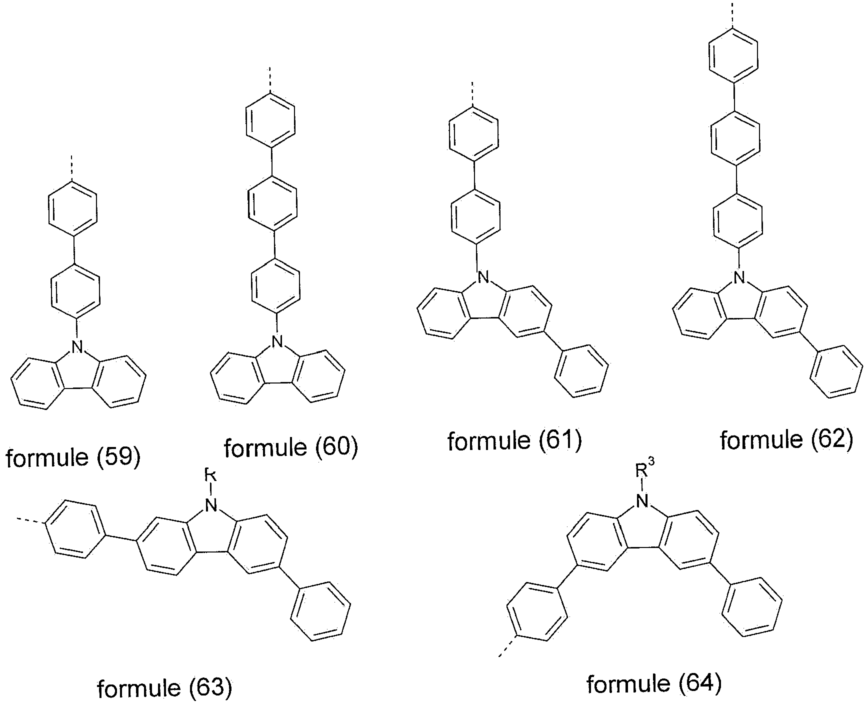 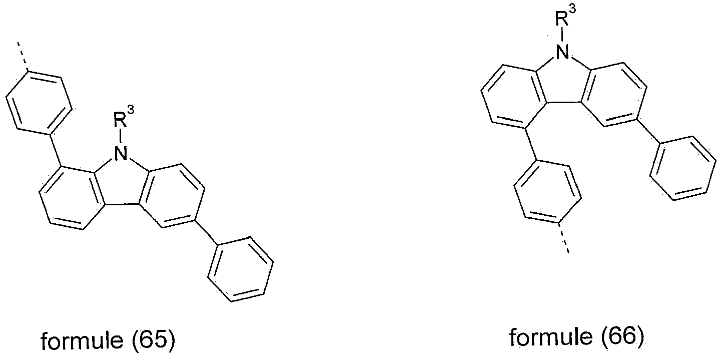 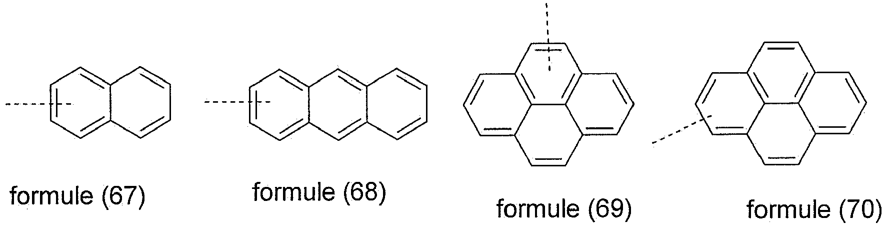 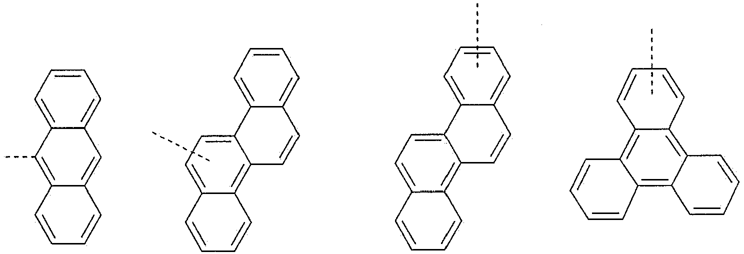 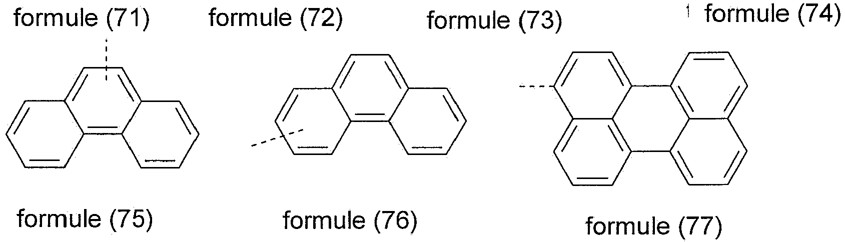 dans lesquelles les lignes en pointillés représentent les liaisons sur l'atome de N et sur le groupe Ar¹ ou Ar², et les groupes peuvent être substitués par un radical ou par plusieurs radicaux R³ tel qu'il a été défini selon la revendication 1.

10. Oligomère, polymère ou dendrimère contenant un ou plusieurs composé(s) selon une ou plusieurs des revendications 1 à 9, dans lequel la/les liaison(s) sur le polymère, sur l'oligomère ou sur le dendrimère peut/peuvent être localisée(s) au niveau de n'importe quelles positions souhaitées dans la formule (I) qui sont substituées par R¹ ou par R².

11. Formulation comprenant au moins un composé selon une ou plusieurs des revendications 1 à 9 ou au moins un polymère, un oligomère ou un dendrimère selon la revendication 10 et au moins un solvant.

12. Dispositif électronique sélectionné parmi les circuits intégrés organiques (OIC), les transistors à effet de champ organiques (OFET), les transistors à film mince organiques (OTFT), les transistors à émission de lumière organiques (OLET), les cellules solaires organiques (OSC), les détecteurs optiques organiques, les photorécepteurs organiques, les dispositifs à extinction de champ organiques (OFQD), les cellules électrochimiques à émission de lumière organiques (OLEC), les diodes laser organiques (O-laser) et les dispositifs électroluminescents organiques (OLED), **caractérisé en ce qu'**il comprend au moins un composé selon une ou plusieurs des revendications 1 à 9.

13. Dispositif électronique selon la revendication 12, sélectionné parmi les dispositifs électroluminescents organiques, **caractérisé en ce qu'**il comprend une anode, une cathode et au moins une couche d'émission, où au moins une couche du dispositif, qui est sélectionnée parmi les couches d'émission, les couches de transport d'électrons, les couches d'injection d'électrons et les couches de blocage de trous, comprend au moins un composé selon une ou plusieurs des revendications 1 à 9.

14. Dispositif électronique selon la revendication 13, **caractérisé en ce que** le composé selon une ou plusieurs des revendications 1 à 9 est présent dans une couche d'émission en combinaison avec un ou plusieurs composé(s) d'émetteur phosphorescent(s).

15. Procédé pour la préparation d'un composé selon une ou plusieurs des revendications 1 à 9, **caractérisé en ce qu'**un composé d'indénocarbazole est amené à réagir avec un système de cycle aromatique ou hétéroaromatique qui contient un groupe hétéroaryle déficient en électrons, où le système de cycle aromatique ou hétéroaromatique est couplé à l'atome d'azote de l'indénocarbazole.

16. Utilisation d'un composé selon une ou plusieurs des revendications 1 à 9 dans un dispositif électronique.
